# EUROPEAN PATENT SPECIFICATION

(11) **EP 1 601 766 B1**
(45) Date of publication and mention of the grant of the patent: **02.09.2009**
(21) Application number: 04712970.5
(22) Date of filing: 20.02.2004
(51) Int. Cl.: C12N 15/11, C07K 7/04, C07K 14/00, A61K 39/00, G01N 33/50

(54) **Peptides capable of inhibiting dimerisation of c-Jun**
Peptide fähig die Dimerisierung von c-Jun zu inhibieren
Peptides capables d'inhiber la dimérisation de c-Jun

(30) Priority: 21.02.2003 US 372003
(43) Date of publication of application: 07.12.2005
(73) Proprietor: Phylogica Limited, Northbridge, W.A. 6003 (AU)
(72) Inventor: WATT, Paul, Mount Claremont, Western Australia 6010 (AU); THOMAS, Wayne, Nedlands, Western Australia 6009 (AU); HOPKINS, Richard, North Perth, Western Australia 6006 (AU)
(74) Representative: Forrest, Graham Robert
(86) International application number: PCT/AU2004/000214
(87) International publication number: WO 2004/074479

(56) References cited:
- WO-A-20/06017913
- WO-A1-00/68373
- YAO S ET AL: "UNCOILING C-JUN COILED COILS: INHIBITORY EFFECTS OF TRUNCATED FOS PEPTIDES ON JUN DIMERIZATION AND DNA BINDING IN VITRO" BIOPOLYMERS, NEW YORK, NY, US, vol. 47, 1998, pages 277-283, XP002924577 ISSN: 0006-3525
- ZHOU X-F ET AL: "LIGAND-ACTIVATED RETINOIC ACID RECEPTOR INHIBITS AP-1 TRANSACTIVATION BY DISRUPTING C-JUN/C-FOS DIMERIZATION" MOLECULAR ENDOCRINOLOGY, BALTIMORE, MD, US, vol. 13, 1999, pages 276-285, XP008057705 ISSN: 0888-8809
- ESTUS S ET AL: "ALTERED GENE EXPRESSION IN NEURONS DURING PROGRAMMED CELL DEATH: IDENTIFICATION OF C-JUN AS NECESSARY FOR NEURONAL APOPTOSIS" JOURNAL OF CELL BIOLOGY, ROCKEFELLER UNIVERSITY PRESS, NEW YORK, US, US, vol. 127, no. 6, PART 1, 1 December 1994 (1994-12-01), pages 1717-1727, XP000674679 ISSN: 0021-9525
- FURMONAVICIENE P.J. ET AL.: 'The use of phage-peptide libraries to define the epitope specificity of a mouse monoclonal anti-Der p 1 antibody representative of a major component of the human immunoglobulin E anti-Der p1 response' CLIN. EXP. ALLERGY vol. 29, no. 11, 1999, pages 1563 - 1571, XP001016249
- DAVIES J.M. ET AL.: 'Use of phage display technology to investigate allerge-antibody interactions' J. ALLERGY CLIN. IMMUNOL. vol. 105, no. 8, 2000, pages 1085 - 1092, XP008057706
- ZHOU X.-F. ET AL.: 'Ligand-activated retinoic acid receptor inhibits AP-1 transactivation by disrupting c-Jun/c-Fos dimerization' MOL. ENDOCRIN. vol. 13, 1999, pages 267 - 285, XP008071353
- YAO S.Q. ET AL.: 'Inhibiting dimerization and DNA binding of c-Jun' PEPTIDES:FRONTIERS OF PEPTIDE SCIENCE, PROCEEDINGS OF AMERICAN PEPTIDE SYMPOSIUM, 15TH 14 July 1997 - 19 July 1997, NASHVILLE, USA, pages 751 - 752, XP008057704

## Description

### Field of the invention

Described herein are methods for the production and of nucleic acid fragment libraries that express highly diverse peptides, polypeptides or protein domains and, in particular, methods for producing nucleic acid fragment libraries wherein the nucleic acid fragments of the libraries are derived from one and preferably from two or more prokaryote genomes or compact eukaryote genomes, such as, for example, organisms having diverse characterized genomes. The nucleic acid fragments may be expressed as protein domains capable of assuming a conformation that binds to a target protein or nucleic acid during library screening. The present invention provides peptides, polypeptide or protein domains are capable of inhibiting dimerization of C-JUN, and uses thereof. Also provided are nucleic acids encoding such peptides, polypeptides or protein domains.

### Background of the invention

### 1. General information

The designation of nucleotide residues referred to herein are those recommended by the IUPAC-IUB Biochemical Nomenclature Commission, wherein A represents Adenine, C represents Cytosine, G represents Guanine, T represents thymine, Y represents a pyrimidine residue, R represents a purine residue, M represents Adenine or Cytosine, K represents Guanine or Thymine, S represents Guanine or Cytosine, W represents Adenine or Thymine, H represents a nucleotide other than Guanine, B represents a nucleotide other than Adenine, V represents a nucleotide other than Thymine, D represents a nucleotide other than Cytosine and N represents any nucleotide residue.

As used herein the term "derived from" shall be taken to indicate that a specified integer may be obtained from a particular source albeit not necessarily directly from that source.

Throughout this specification, unless the context requires otherwise, the word "comprise", or variations such as "comprises" or "comprising", will be understood to imply the inclusion of a stated step or element or integer or group of steps or elements or integers but not the exclusion of any other step or element or integer or group of elements or integers.

The present invention is performed without undue experimentation using, unless otherwise indicated, conventional techniques of molecular biology, microbiology, virology, recombinant DNA technology, peptide synthesis in solution, solid phase peptide synthesis, and immunology. Such procedures are described, for example, in the following texts:
1. Sambrook, Fritsch & Maniatis,, whole of Vols I, II, and III;
2. DNA Cloning: A Practical Approach, Vols. I and II (D. N. Glover, ed., 1985), IRL Press, Oxford, whole of text;
3. Oligonucleotide Synthesis: A Practical Approach (M. J. Gait, ed., 1984) IRL Press, Oxford, whole of text, and particularly the papers therein by Gait, pp1-22; Atkinson *et al.,* pp35-81; Sproat *et al.,* pp 83-115; and Wu *et al.,* pp 135-151;
4. Nucleic Acid Hybridization: A Practical Approach (B. D. Hames & S. J. Higgins, eds., 1985) IRL Press, Oxford, whole of text;
5. Animal Cell Culture: Practical Approach, Third Edition (John R.W. Masters, ed., 2000), ISBN 0199637970, whole of text;
6. Immobilized Cells and Enzymes: A Practical Approach (1986) IRL Press, Oxford, whole of text;
7. Perbal, B., A Practical Guide to Molecular Cloning (1984);
8. Methods In Enzymology (S. Colowick and N. Kaplan, eds., Academic Press, Inc.), whole of series;
9. J.F. Ramalho Ortigão, "The Chemistry of Peptide Synthesis" In: Knowledge database of Access to Virtual Laboratory website (Interactiva, Germany);
10. Sakakibara, D., Teichman, J., Lien, E. Land Fenichel, R.L. (1976). Biochem. Biophys. Res. Common. 73 336-342
11. Merrifield, R.B. (1963). J. Am. Chem, Soc. 85, 2149-2154.
12. Barany, G. and Merrifield, R.B. (1979) in The Peptides (Gross, E. and Meienhofer, J. eds.), vol. 2, pp. 1-284, Academic Press, New York.
13. Wünsch, E., ed. (1974) Synthese von Peptiden in Houben-Weyls Metoden der Organischen Chemie (Müler, E., ed.), vol. 15, 4th edn., Parts 1 and 2, Thieme, Stuttgart.
14. Bodanszky, M. (1984) Principles of Peptide Synthesis, Springer-Verlag, Heidelberg.
15. Bodanszky, M. & Bodanszky, A. (1984) The Practice of Peptide Synthesis, Springer-Verlag, Heidelberg.
16. Bodanszky, M. (1985) Int. J. Peptide Protein Res. 25, 449-474.
17. Handbook of Experimental Immunology, Vols. I-IV (D. M. Weir and C. C. Blackwell, eds., 1986, Blackwell Scientific Publications).
18. McPherson et al., In: PCR A Practical Approach., IRL Press, Oxford University Press, Oxford, United Kingdom, 1991.
19. Methods in Yeast Genetics: A Cold Spring Harbor Laboratory Course Manual (D. Burke et al., eds) Cold Spring Harbor Press, New York, 2000 (*see whole of text*).
20. Guide to Yeast Genetics and Molecular Biology. In: Methods in Enzymology Series, Vol. 194 (C. Guthrie and G.R. Fink eds) Academic Press, London, 1991 2000 (*see whole of text*)*.*

### 2. Description of the related art.

As a response to the increasing demand for new lead compounds and new target identification and validation reagents, the pharmaceutical industry has increased its screening of various sources for new lead compounds having a unique activity or specificity in therapeutic applications, such as, for example, in the treatment of neoplastic disorders, infection, modulating immunity, autoimmunity, fertility, etc.

It is known that proteins bind to other proteins, antigens, antibodies, nucleic acids, and carbohydrates. Such binding enables the protein to effect changes in a wide variety of biological processes in all living organisms. As a consequence, proteins represent an important source of natural modulators of phenotype. Accordingly, peptides that modulate the binding activity of a protein represent attractive lead compounds (drug candidates) in primary or secondary drug screening. For example, the formation of a target biological interaction that has a deleterious effect (eg. replication of a pathogen or of a cancer cell), can be assayed to identify lead compounds that antagonize the biological interaction.

Differential gene expression between normal and diseased (eg., neoplastic or apoptotic) cells, such as, for example, differential expression of cellular receptors, and/or differential signal transduction processes between normal and diseased cells, implicate those differential patterns of gene expression in disease. Accordingly, the genes or proteins that are differentially expressed in diseased and normal cells, or the differential cellular processes between normal and diseased cells, form attractive targets for therapy. Similarly, cyclin proteins such as Cdc2, Cdc25, and cyclin-dependent kinases (CDKs) are attractive targets for cellular proliferation. Peptides that agonize or antagonize the expression of such target genes or target processes are suitable lead compounds for therapeutic applications.

It is widely recognized that there is a need to develop methods for determining novel compounds, including nucleic acid-based products and peptide-based products, that modulate an activity or function of a particular target. In such approaches, an activity of a target protein or nucleic acid is screened in the absence and presence of a potential lead compound, which is a peptide, and modified activity of the target is determined.

Similarly, peptides can be used as dominant negative inhibitors or the validation of prospective drug targets using assays such as observing the phenotype resulting from over-expression of the peptides in ex-vivo assays or in transgenic mice.

In one known approach to identify novel lead compounds, random peptide (synthetic mimetic or mimotope) libraries are produced using short random oligonucleotides produced by synthetic combinatorial chemistry. The DNA sequences are cloned into an appropriate vehicle for expression and the encoded peptide is then screened using one of a variety of approaches. However, the ability to isolate active peptides from random fragment libraries can be highly variable with low affinity interactions occurring between the peptide-binding partners. Moreover, the expressed peptides often show little or none of the secondary or tertiary structure required for efficient binding activity, and/or are unstable. This is not surprising, considering that biological molecules appear to recognise shape and charge rather than primary sequence (Yang and Honig J. Mol. Biol 301 (3), 691-711 2000) and that such random peptide aptamers are generally too small to comprise a protein domain or to form the secondary structure of a protein domain. The relatively unstructured 'linear' nature of these peptide aptamers also leads to their more rapid degradation and clearance following administration to a subject *in vivo,* thereby reducing their appeal as therapeutic agents.

To enhance the probability of obtaining useful bioactive peptides or proteins from random peptide libraries, peptides have previously been constrained within scaffold structures, eg., thioredoxin (Trx) loop (Blum et al. Proc. Natl. Acad. Sci. USA, 97, 2241-2246, 2000) or catalytically inactive staphylococcal nuclease (Norman et al, Science, 285, 591-595, 1999), to enhance their stability. Constraint of peptides within such structures has been shown, in some cases, to enhance the affinity of the interaction between the expressed peptides and its target, presumably by limiting the degrees of conformational freedom of the peptide, and thereby minimizing the entropic cost of binding.

It is also known to tailor peptide expression libraries for identifying specific peptides involved in a particular process, eg., antigen-antibody-binding activity. For example US Patent No 6,319,690 (Dade Behring Marburg GmBH) teaches a PCR-based method of amplifying cDNA sequences encoding a population of antibodies, wherein oligonucleotide primers that are homologous to conserved regions of antibody-encoding cDNAs derived from a mixture of non-activated B- lymphocytes are used to amplify nucleic acids that encode antibody variable regions. The amplified sequences are expressed using a bacterial display system, for screening with selected antigens to determine those antibody fragments that bind the antigens. However, the expression libraries described in US Pat. No. 6,319,690 show limited diversity, because the amplified fragments were all antibody-encoding fragments derived from a single complex eukaryote. Additionally, the antibody-encoding libraries described in US Pat. No. 6,319,690 were screened for antigen-binding activity rather than for a novel bioactivity (ie. the expressed peptides were not mimotopes).

Several attempts have been made to develop libraries based on naturally occurring proteins (eg genomic expression libraries). Libraries of up to several thousand polypeptides or peptides have been prepared by gene expression systems and displayed on chemical supports or in biological systems suitable for testing biological activity. For example, genome fragments isolated from *Escherichia coli* MG1655 have been expressed using phage display technology, and the expressed peptides screened to identify peptides that bind to a polyclonal anti-Rec A protein antisera (Palzkill et al. Gene, 221 79-83, 1998). Such expression libraries are generally produced using nucleic acid from single genomes, and generally comprise nucleic acid fragments comprising whole genes and/or multiple genes or whole operons, including multiple linked protein domains of proteins. Additionally, as many bacteria comprise recA-encoding genes, the libraries described by Palzkill *et al.,* were screened for an activity that was known for the organism concerned, rather than for a novel bioactivity (ie. the expressed peptides were not necessarily mimotopes).

US Patent No. 5,763,239 (Diversa Corporation) describes a procedure for producing normalized genomic DNA libraries from uncharacterized environmental samples containing a mixture of uncharacterized genomes. The procedure described by Diversa Corp. comprises melting DNA isolated from an environmental sample, and allowing the DNA to reanneal under stringent conditions. Rare sequences, that are less likely to reanneal to their complementary strand in a short period of time, are isolated as single-stranded nucleic acid and used to generate a gene expression library. However, total normalization of each organism within such uncharacterized samples is difficult to achieve, thereby reducing the biodiversity of the library. Such libraries also tend to be biased toward the frequency with which a particular organism is found in the native environment. As such, the library does not represent the true population of the biodiversity found in a particular biological sample. In cases where the environmental sample includes a dominant organism, there is likely to be a significant species bias that adversely impacts on the sequence diversity of the library. Furthermore, as many of the organisms found in such samples are uncharacterized, very little information is known regarding the constitution of the genomes that comprise such libraries. Accordingly, it is not possible to estimate the true diversity of such libraries. Additionally, since the Diversa Corp. process relies upon PCR using random primers to amplify uncharacterized nucleic acids, there is no possibility of accounting for biasing factors, such as, for example, a disproportionate representation of repeated sequences across genomes of the organisms in the environmental sample.

Accordingly, there remains a need to produce improved methods for constructing highly diverse and well characterized expression libraries wherein the expressed peptides are capable of assuming a secondary structure or conformation sufficient to bind to a target protein or nucleic acid, such as, for example, by virtue of the inserted nucleic acid encoding a protein domain.

### Summary of the invention

The methods described herein are based upon the understanding of the present inventors that, in contrast to random synthetic peptide libraries produced by combinatorial approaches, or short random peptides produced by expression of PCR products, amino acids are not randomly distributed in nature (Pande et al., Proc Natl Acad. Sci. USA 91 12972-12975, 1994). Proteins that fold well in nature have non-random hydrophobicity distributions (Irback et al., Proc Natl Acad. Sci. USA 93, 9533 - 9538, 1996). In any native peptide, the distribution of amino acid residues according to their chemical properties (eg hydrophobicity, polarity, etc) is also non-random (Baud and Karlin, Proc Natl Acad Sci. USA 96, 12494-12499, 1999). Accordingly, the present inventors realized that random peptide libraries have a low frequency of naturally occurring or native peptide conformational structures or secondary structures, such as, for example, those structures formed by protein domains.

In work leading up to the present invention, the inventors sought to take advantage of diverse and well-characterized prokaryotic genomes and/or compact eukaryotic genomes in the construction of highly diverse expression libraries for isolating bioactive peptides or proteins. In particular, the use of combinations of nucleic acid fragments from one or two or more well characterized genomes has allowed the inventors to control the degree the diversity of peptides/proteins expressed in their expression libraries, to enhance the possibility of isolating novel peptides having the ability to bind to a desired protein or nucleic acid. It will be understood from the disclosure herein that the bioactive peptides or proteins expressed by individual library clones of such libraries are screened for an activity of the encoded peptide, particularly a binding activity, which said encoded protein has not been shown to possess in the context of the protein from which it was derived (ie in its native environment). In the screening process, any library clone encoding a peptide that has the same activity as it would have in its native environment is excluded during the screening process, since an objective of the present invention is to isolate novel bioactive peptides or proteins.

Peptides encoded by genomes which differ from the genome of the drug target organism (eg. humans) are a particularly rich source of high affinity target binding agents. This is because in the evolution of the target organism itself, such high affinity peptide domains have been selected against other than the interaction interfaces which may exist in that organism for functional dimerization with natural partners.

The invention provides an isolated peptide or protein domain comprising or consisting of an amino acid sequence set forth in SEQ ID NO: 133 or SEQ ID NO: 134.

The invention further provides an isolated nucleic acid encoding a peptide or protein domain according to claim 1. The isolated nucleic acid may comprise the sequence set forth in SEQ ID NO: 132.

The invention further provides a pharmaceutical formulation comprising the isolated peptide, protein domain or nucleic acid of the invention in combination with a physiologically acceptable vehicle or carrier.

The invention further provides an isolated peptide, protein domain or nucleic acid of the invention for use in a method of medical treatment, and in particular for the treatment of a neurodegenerative disease, for example Huntingdon's disease..

The invention further provides the use of an isolated peptide, protein domain or nucleic acid of the invention in the preparation of a medicament for the treatment of a neurodegenerative disease, for example Huntingdon' disease.

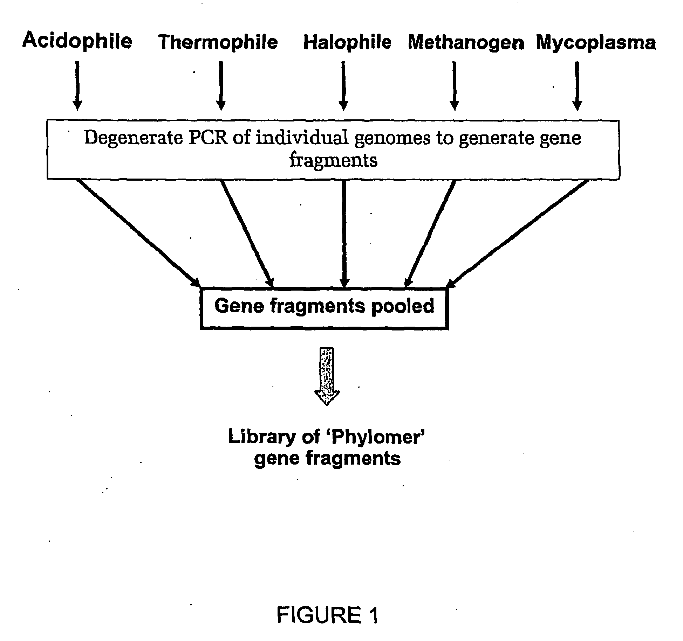

Figure 1 illustrates a method of generating an expression library, wherein nucleic acid fragments are isolated from multiple evolutionary diverse organisms and pooled in such a way as to ensure about equal representation of each of the genomes. Nested PCR using degenerate PCR primers amplifies sequences from the pooled genomes in a first round, and specific PCR amplifies the nucleic acid fragments so as to permit their direct cloning into an expression vector.

The poor representation of low copy number sequences may be reduced or minimized by normalizing the nucleic acid according to the complexity and size of the genome of the microorganism or compact eukaryote (ie., relative genome size of content of each contributing genome of the expression library). Thus, where genomes from more than one organism are used in the construction of the library, each of those contributing genomes may be used in an amount that is proportional to that complexity and size of the genome (or transcriptome), such as, for example, in comparison to the complexity and size of another genome in the mixture of genomes. This process results in about equal representation of the genome fragments in the biodiverse nucleic acid fragment library.

The nucleic acid fragments may be selected such that the encoded peptides have an average length that is about the length of a protein domain, eg., at least about 12-1 S amino acids in length and more preferably at least about 15 amino acids in length or at least about 20 amino, acids in length or at least about 30 amino acids in length.

Alternatively, or in addition, the nucleic acid fragments may encode peptides that, on average, comprise or consist of a protein domain. As used herein, the term "protein domain" shall be taken to mean a discrete portion of a protein that assumes a secondary structure or conformation sufficient to permit said portion to perform a specific function in the context of a target protein or target nucleic acid and, in particular, to bind with high affinity to the target protein or nucleic acid. Preferred protein domains are not required to be constrained within a scaffold structure to bind to the target nucleic acid or target protein, or for said binding to be enhanced.
The term "protein domain" or "domain" or similar shall be taken to include an independently folding peptide structure (ie. a "subdomain") unless the context requires otherwise. For example, protein subdomain consisting of a 19-residue fragment from the C-loop of the fourth epidermal growth factor-like domain of thrombomodulin has been described by Alder et al, J. Biol. Chem., 270: 23366-23372, 1995. Accordingly, the skilled artisan is aware of the meaning of the term "protein subdomain".

Accordingly, nucleic acid fragments used in the generation of the expression libraries may encode peptides that form stable secondary structures or conformations in the absence of a Trx loop or catalytically inactive staphylococcal nuclease peptide.

The nucleic acid fragments of the expression libraries of the invention may encode a single protein domain. Accordingly, the nucleic acid fragments of the expression libraries may encode a peptide having an upper length of about 50 amino acid residues.

The isolated peptide or protein domain of the invention can block an interaction between two c-Jun proteins, ie c-Jun self-dimerization. Even more preferably, the isolated peptide or protein domain can block c-Jun self dimerization in a cell. The isolated peptide comprises an amino acid sequence selected from the group consisting of SEQ ID NO: 133 and SEQ ID NO: 134.

The present invention clearly extends to any isolated nucleic acid encoding the peptide or protein domain of the invention. Exemplary nucleic acids provided herein comprise the nucleotide sequence of: SEQ ID NO: 132.

### Brief description of the drawings

Figure 1 is a schematic representation showing a simplified method of generating an expression library of the present invention, said library comprising nucleic acid fragments from multiple evolutionary diverse organisms. Initially nucleic acids are isolated from such organisms and pooled in such a way as to ensure equal representation of each of the genomes. Degenerate PCR is then used to amplify sequences from the pool of the genomes, before specific PCR is used to further amplify these nucleic acid fragments in such a way that they may be cloned into an expression vector.

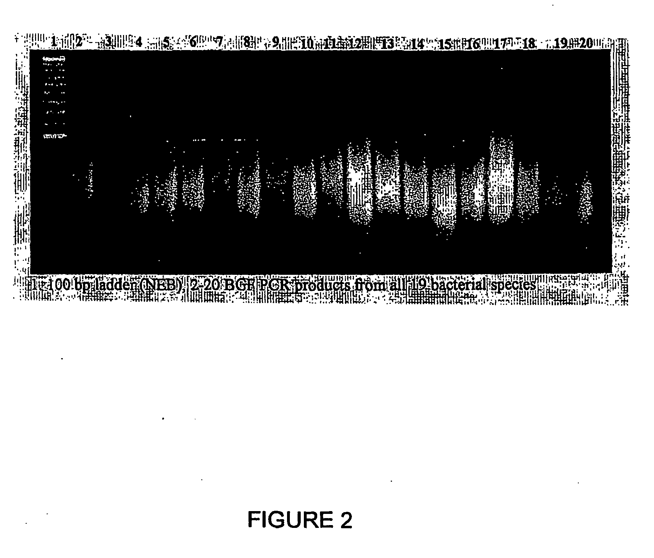

Figure 2 is a photographic representation showing amplification products of random PCR amplification of genomic DNA isolated from *Archaeoglobus fulgidis, Aquifex aeliticus, Aeropyrum pernix, Bacillus subtilis, Bordetella pertussis TOX6, Borrelia burgdorferi, Chlamydia trachomati, Escherichia coli K12, Haemophilus influenzae (rd), Helicobacter pylori, Methanobacterium thermoautotrophicum, Methanococcus jannaschii, Mycoplasma pneumoniae, Neisseria meningitidis, Pseudomonas aeruginosa, Pyrococcus horikoshii, Synechocystis PCC 6803, Thermoplasma volcanium,* and *Thermotoga maritima.* The molecular weight marker is shown on the far left.

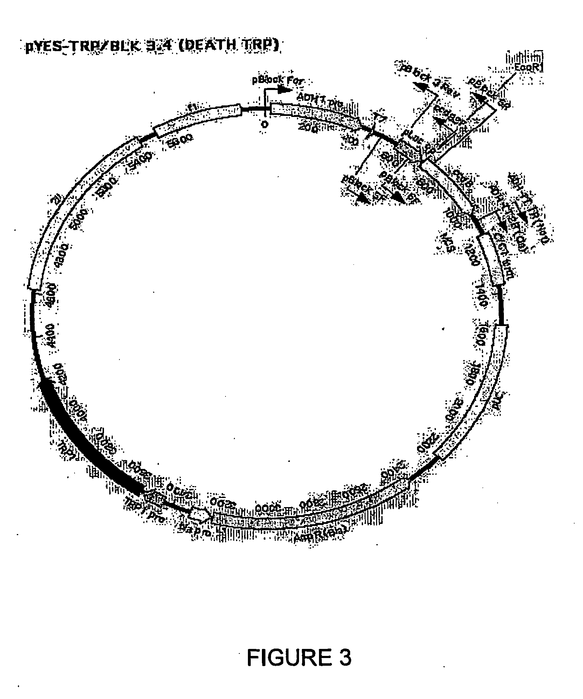

Figure 3 is a schematic representation of the pDEATH-Trp vector (SEQ ID NO: 36). The pDEATH-Trp vector comprises a minimal ADH promoter for constitutive expression of a nucleic acid inserted into the vector in yeast cells; a T7 promoter for expression of a nucleic acid fragment in bacterial cells; a nucleic acid encoding a SV-40 nuclear localization signal to force any expressed polypeptide into the nucleus of a yeast cell; a CYC1 terminator, for termination of transcription in yeast cells; a nucleic acid encoding a peptide conferring ampicillin resistance, for selection in bacterial cells; a nucleic acid encoding TRP1 which allows auxotrophic yeast to grow in media lacking tryptophan; a pUC origin of replication, to allow the plasmid to replicate in bacterial cells; and a 2µ origin of replication, to allow the plasmid to replicate in yeast cells.

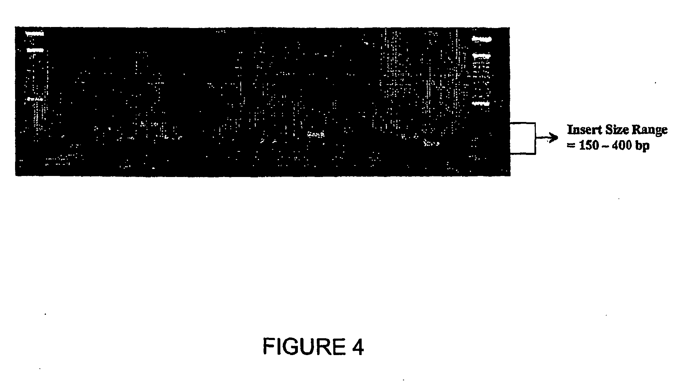

Figure 4 is a photographic representation showing nucleic acid fragments isolated from bacterial clones carrying the pDEATH-Trp vector. The isolated vector was digested with the restriction endonuclease EcoRI and the resulting fragments electrophoresed. The molecular weight marker is shown on the far left and far right, and the text indicates the size range of the nucleic acid fragments in base pairs.

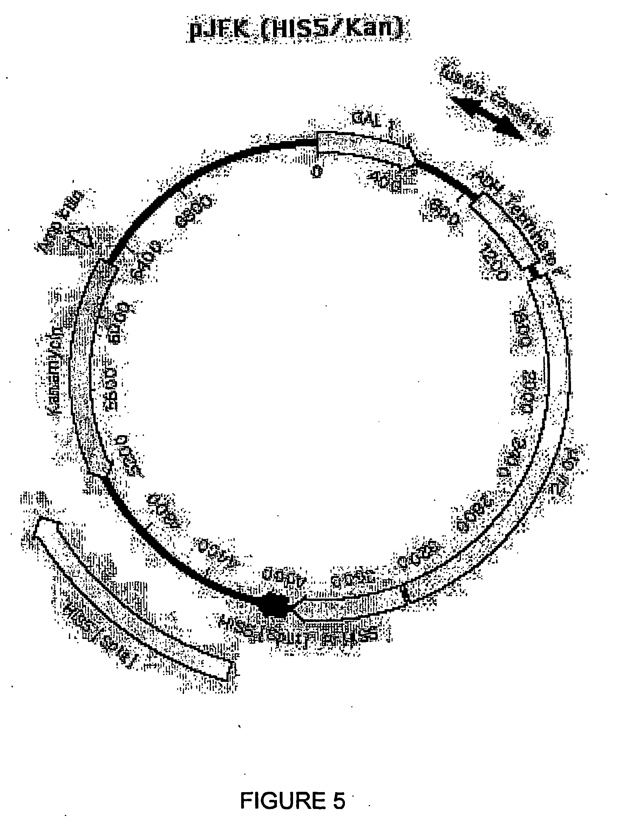

Figure 5 is a schematic representation of the pJFK vector (SEQ ID NO: 60). The pJFK vector comprises a GAL1 promoter for inducible expression of a nucleic acid fragment in yeast cells; a nuclear localization signal to force any expressed polypeptide into the nucleus of a yeast cell; a nucleic acid encoding an activation domain derived from the B42 protein, to be expressed as a fusion with a polypeptide of interest in a "n"-hybrid screen; an ADH terminator or termination of transcription in yeast cells; a 2µ origin of replication, to allow the plasmid to replicate in yeast cells; an HIS5 gene to allow auxotrophic yeast to grow in media lacking histidine; a nucleic acid encoding a peptide conferring ampicillin resistance, for selection in bacterial cells; and a nucleic acid encoding a peptide conferring kanamycin resistance.

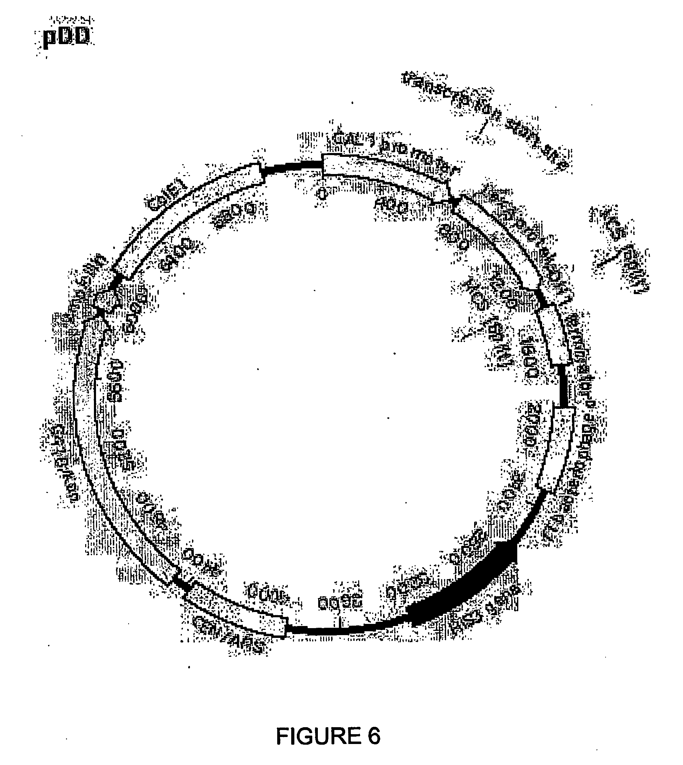

Figure 6 is a schematic representation of the pDD vector (SEQ ID NO: 61). The pDD vector comprises a GAL1 promoter for inducible expression of a nucleic acid fragment in yeast cells; a nucleic acid encoding a LEXA1 protein, to be expressed as a fusion with a polypeptide of interest in a "n"-hybrid screen; an ADH terminator or termination of transcription in yeast cells; a 2µ origin of replication, to allow the plasmid to replicate in yeast cells; an HIS5 gene to allow auxotrophic yeast to grow in media lacking histidine; a nucleic acid encoding a peptide conferring ampicillin resistance, for selection in bacterial cells; and a nucleic acid encoding a peptide conferring kanamycin resistance.

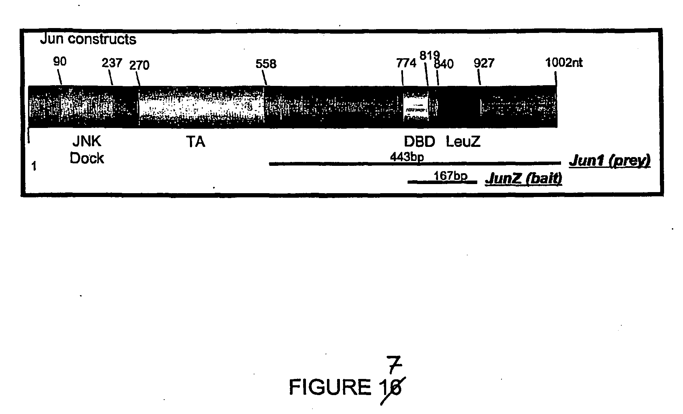

Figure 7 is a schematic representation of a JUN polypeptide. As shown the constructs JUN1 and JUNZ both encompass the DNA binding domain (DBD) and leucine zipper (LeuZ) domain of JUN. The leucine zipper domain is important for homo-dimerization of JUN.

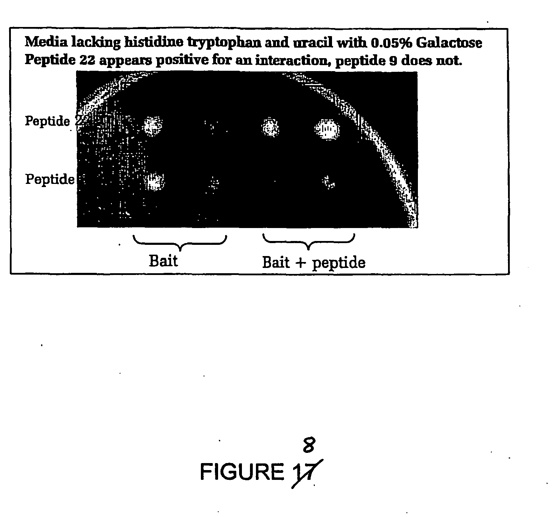

Figure 8 is a graphical representation of a photopgraph showing yeast colonies expressing JUN1 and a peptide thatinterats with JUN1 (Peptide 22) or JUN1 and a peptide that does not interact with JUN1 (Peptide 9). Also shown are cells expressing only the bait(ie JUN1). Note the increased growth in those cell expressing the interacting polypeptides.

### Detailed description of the preferred embodiments

A peptide of the present invention can be expressed such that it is conformationally constrained, or expressed in a "presentation structure". Such constraint, whilst not generally necessary for expressing protein domains or peptides having a conformation sufficient to bind to a target protein, is useful for displaying peptides that comprise more highly flexible sequences, or to enhance stability against proteolytic enzymes (Humphrey et al, Chem Rev 97, 2243-2266,1997).

A presentation structure will generally comprise a first component, ie. polypeptide, that is fused to the amino terminus of the polypeptide and a second component fused to the carboxyl- terminus of the peptide. Examples of such presentation structures include, but are not limited to, cysteine-linked (disulfide) structures, zinc-finger domains, cyclic peptides, and transglutaminase linked structures.

In a preferred embodiment, the presentation structure is a sequence that contains at least two cysteine residues, such that a disulphide bond is formed between the cysteine residues, resulting in a conformationally constrained peptide.

In another embodiment, a peptide encoded by an expression library of the present invention is expressed within a second polypeptide as a fusion protein. Polypeptides used for such purposes are capable of reducing the flexibility of another protein's amino and/or.carboxyl termini. Preferably, such proteins provide a rigid scaffold or platform for the protein. In addition, such proteins preferably are capable of providing protection from proteolytic degradation and the like, and/or are capable of enhancing solubility. Preferably, conformation-constraining proteins are small in size (generally, less than or equal to about 200 amino acids in length), rigid in structure, of known three-dimensional configuration, and are able to accommodate insertions of proteins without undue disruption of their structures. A key feature of such proteins is the availability, on their solvent exposed surfaces, of locations where peptide insertions can be made (eg., the Trx loop). It is also preferable that conformation-constraining protein producing genes be highly expressible in various prokaryotic and eukaryotic hosts, or in suitable cell-free systems, and that the proteins be soluble and resistant to protease degradation.

Examples of conformation-constraining proteins include the active site of thioredoxin or Trx loop and other thioredoxin-like proteins, nucleases (eg., RNase A), proteases (eg., trypsin), protease inhibitors (eg., bovine pancreatic trypsin inhibitor), antibodies or structurally rigid fragments thereof, conotoxins, and the pleckstrin homology domain. A conformation-constraining peptide can be of any appropriate length and can even be a single amino acid residue.

This technique has been successfully used for bacterial display of peptides in bacteria using a Trx scaffold (Blum et al Proc. Natl. Acad. Sci. USA 97, 2241-2246 2000) in addition to the use in yeast 2 hybrid screening using either a catalytically inactive form of staphylococcal nuclease, or Trx (Norman et al, Science,285, 591-595, 1999; and Colas et al, Nature 380, 548-550, 1996).

In one embodiment the peptide of the present invention is expressed as a fusion protein with a peptide sequence capable of enhancing, increasing or assisting penetration or uptake of the peptide by cells either *in vitro* or *in vivo.* For example, the peptide sequence capable of enhancing, increasing or assisting penetration or uptake is the *Drosophila* penetratin targeting sequence. This peptide sequence at least comprises the amino acid sequence:

CysArgGlnIleLysIleTrpPheGlnAsnArgArgMetLysTrpLysLys (SEQ ID NO. 29) further comprising (Xaa)n after the final Lys residue and followed by Cys wherein Xaa is any amino acid and n has a value greater than or equal to 1. Alternatively, a homologue, derivative or analogue of said sequence is used. The use of said sequence is particularly useful when peptides encoded by the nucleic acid fragment of the present invention are synthesised *in vitro* or secreted from a host cell, and must be taken up by a cell for screening said peptide encoded by the nucleic acid fragment of the present invention.

Those skilled in the art will also be aware of an analogous use of signals such as for example, the tat sequence of HIV to drive import of peptides into cells.

In an alternative embodiment, the peptide of the present invention is mixed with a peptide capable of enhancing, increasing or assisting penetration or uptake by cells *in vitro* or *in vivo.* A peptide sequence that is able to increase or assist penetration or uptake of cells is the synthetic peptide *Pep 1*, which at least comprises the amino acid sequence:
LysGluThrTrpTrpGluThrTrpTrpThrGluTrpserGlnLysLysLysLysArgLysVal (SEQ ID NO. 30).

The *Pep1* peptide does not need to be conjugated to the peptide encoded by the nucleic acid fragments of the present invention. Furthermore, *Pep1* dissociates from the peptide encoded by the expression library of the present invention. Thus *Pep1* will not interfere with the peptide forming a conformation sufficient for binding to a target protein or nucleic acid. *Pep1* is only useful when the peptides encoded by the expression library of the present invention are isolated prior to the addition to a cell or organism for screening. Thus *Pep1* is particularly useful when *in vitro* libraries are screened.

Other protein transduction domains are known in the art, and are clearly useful in the present invention. For example, amino acids 43-58 of *Drosophila* antennapedia, poly-arginine, PTD-5, Transportan and KALA (reviewed in Kabouridis, TRENDS in Biotechnology, 21: 498-503, 2003).

Nucleic acid fragment expression libraries have been screened for encoded peptides that inhibit or antagonize or block self dimerization of JUN protein. Such peptide antagonists ("peptide blockers") are particularly useful for validating a cellular target in the therapeutic treatment of a neurodegenerative disorder or for the therapeutic treatment of an individual suffering from a neurodegenerative disorder. As exemplified herein, reverse two hybrid screens that assay the interaction between JUN1 and JUNZ (fragments of c-JUN that include the leucine zipper domain), have successfully been used to identify several specific peptide blockers of c-JUN dimerization.

The peptides or nucleic acid fragments of the present invention may be expressed as fusion proteins to form single-chain Fv (scFv) or Fab antibody fragments as described in McCafferty et al, Nature 348 552-534 (1990) and Hoogenboom et al, Nucleic Acids Res 19, 4133-4137 (1991). The generation of a scFv library essentially involves generation of a gene construct comprising two or more nucleic acid fragments of the present invention separated by a nucleotide sequence encoding a scFv peptide linker, such as for example (Gly₄Ser) ₃. The resulting gene construct is then expressed in an appropriate system to produce a single chain fragment of an antibody. The displayed library is screened for antibody fragments having a conformation sufficient for binding a specific antigen using techniques known in the art, such as, for example, affinity purification.

Using techniques known in the art, scFv fragments are isolated that bind to specific antigens or molecules. Such techniques include, for example, affinity chromatography and 'n'-hybrid screening. Furthermore, through selection of increased nucleotide sequence diversity through, for example random mutagenesis, it is possible to select for antibodies with increased affinity for the specific antigen.

As will be apparent to the skilled artisan, the use of a peptide of the present invention to treat a disorder may require the peptide be formulated into a compound for administration.

Preferably, the compound is a pharmaceutical compound.

Formulation of a pharmaceutical compound will vary according to the route of administration selected (e.g., solution, emulsion, capsule). An appropriate composition comprising the identified modulator to be administered can be prepared in a physiologically acceptable vehicle or carrier. For solutions or emulsions, suitable carriers include, for example, aqueous or alcoholic/aqueous solutions, emulsions or suspensions, including saline and buffered media. Parenteral vehicles can include sodium chloride solution, Ringer's dextrose, dextrose and sodium chloride, lactated Ringer's or fixed oils, for instance. Intravenous vehicles can include various additives, preservatives, or fluid, nutrient or electrolyte replenishers and the like (See, generally, Remington's Pharmaceutical Sciences, 17th Edition, Mack Publishing Co., Pa., 1985). For inhalation, the agent can be solubilized and loaded into a suitable dispenser for administration (e.g., an atomizer, nebulizer or pressurized aerosol dispenser).

Furthermore, the protein or peptide can be administered via in vivo expression of the recombinant protein. In vivo expression can be accomplished via somatic cell expression according to suitable methods (see, e.g. U.S. Pat. No. 5,399,346). In this embodiment, nucleic acid encoding the protein can be incorporated into a retroviral, adenoviral or other suitable vector (preferably, a replication deficient infectious vector) for delivery, or can be introduced into a transfected or transformed host cell capable of expressing the protein for delivery. In the latter embodiment, the cells can be implanted (alone or in a barrier device), injected or otherwise introduced in an amount effective to express the protein in a therapeutically effective amount.

As will be apparent to a skilled artisan, a compound that is active *in vivo* is particular preferred. A compound that is active in a human subject is even more preferred. Accordingly, when manufacturing a compound that is useful for the treatment of a disease it is preferable to ensure that any components added to the peptide does not inhibit or modify the activity of said peptide.

As stated *supra* the peptides identified by the present inventors are capable of inhibiting the homo-dimerization of c-Jun. Accordingly, in one embodiment, the present invention provides a method for inhibiting self dimerization of c-Jun comprising administering a peptide or nucleic acid of the present invention in vitro to a cell comprising or expressing a c-Jun polypeptide. Preferably,. the peptide inhibitor has a conformation sufficient for binding a c-Jun polypeptide. The nucleic acid is placed in operable connection with a promoter thereby enabling expressionof said peptide.

A homodimer of c-Jun is required for effective activation of this transcription factor. Active c-Jun has been implicated in cell survival, cell differentiation and neuronal regeneration. Furthermore, c-Jun iinhibition has been shown to protect neurons from apoptosis both *in vitro* and *in vivo* (Estus et al., J. Cell Biol., 127: 1717-1727, 1994 and Behrens et al., Nat. Genet. 21: 326-329, 1999). In fact, studies have shown that inhibition of c-Jun function is neuroprotective in a model of neurodegenerative disease (Garcia et al., J. Neuroscience, 22: 2174-2184, 2002.

Accordingly, the peptides, proteins and nucleic acids of the invention may be used for treating a neurodegenerative disease such as an amino acid sequence selected from the group consisting of SEQ ID NO: 94, SEQ ID NO: 95, SEQ ID NO: 97, SEQ ID NO: 98, SEQ ID NO: 100, SEQ ID NO: 101, SEQ ID NO: 103, SEQ ID NO: 104, SEQ ID NO: 106, SEQ ID NO: 107, SEQ ID NO: 109, SEQ ID NO: 110, SEQ ID NO: 112, SEQ ID NO: 113, SEQ ID NO: 115, SEQ ID NO: 116, SEQ ID NO: 118, SEQ ID NO: 119, SEQ ID NO: 121, SEQ ID NO: 122, SEQ ID NO: 124, SEQ ID NO: 125, SEQ ID NO: 127, SEQ ID NO: 128, SEQ ID NO: 130, SEQ ID NO: 131, SEQ ID NO: 133, SEQ ID NO: 134, SEQ ID NO: 136, SEQ ID NO: 137, SEQ ID NO: 139, SEQ ID NO: 140, SEQ ID NO: 142, SEQ ID NO: 143, SEQ ID NO: 145, SEQ ID NO: 146, SEQ ID NO: 148, SEQ ID NO: 149, SEQ ID NO: 151, SEQ ID NO: 152, SEQ ID NO: 154, SEQ ID NO: 155, SEQ ID NO: 157, SEQ ID NO: 158, SEQ ID NO: 160, SEQ ID NO: 161, SEQ ID NO: 163, SEQ ID NO: 164 and SEQ ID NO: 165. Preferably, the Huntington's disease.

When used in therapeutic aspects of the invention, the nucleic acid of the invention may be placed in operable connetction with a promoter thereby enabling expressionof said peptide.in a cell of the subject. The subject in need of treatment suffers from a neurodegenerative disease, such as Huntington's disease.

The invention is further described with reference to the following non-limiting Examples.

### EXAMPLE 1

### The construction of a biodiverse nucleic acid fragment expression library in the vector pDEATH-Trp

**Nucleic acid was isolated from the following bacterial species:**

| | |
|---|---|
| *1* | *Archaeoglobus fulgidis* |
| *2* | *Aquifex aeliticus* |
| *3* | *Aeropyrum pernix* |
| *4* | *Bacillus subtilis* |
| *5* | *Bordetella pertussis TOX6* |
| *6* | *Borrelia burgdorferi* |
| *7* | *Chlamydia trachomatis* |
| *8* | *Escherichia coli Kl2* |
| *9* | *Haemophilus influenzae (rd)* |
| *10* | *Helicobacter pylori* |
| *11* | *Methanobacterium thermoautotrophicum* |
| *12* | *Methanococcus jannaschii* |
| *13* | *Mycoplasma pneumoniae* |
| *14* | *Neisseria meningitidis* |
| *15* | *Pseudomonas aeruginosa* |
| *16* | *Pyrococcus horikoshii* |
| 17 | *S nechosistis PCC 6803* |
| *18* | *Thermoplasma volcanium* |
| *19* | *Thermotoga maritima* |

Nucleic acid fragments were generated from the genomic DNA of each genome using 2 consecutive rounds of primer extension amplification using tagged random oligonucleotides with the sequence:
5'-GACTACAAGGACGACGACGACAAGGCTTATCAATCAATCAN₆-3' (SEQ ID NO: 33). The PCR amplification was completed using the Klenow fragment of *E*. *coli* DNA polymerase I in the following primer extension reaction:

| Reagent | Volume |
|---|---|
| DNA (100-200ng) | |
| Oligonucleotide comprising SEQ ID NO: 33 (25µM) | 4µl |
| H₂O | to 17.4µl. |

Samples were then boiled for 3-5 minutes to denature the nucleic acid isolated from the bacteria, before being snap cooled, to allow the tagged random oligonucleotides to anneal to said nucleic acid. These samples were then added to the following reagents:

| | |
|---|---|
| Klenow buffer | 3µl |
| dNTP (2mM) | 3µl |
| Klenow | 0.6µl |
| Polyethylene Glycol (8,500) | 6µl |

Primer extension reactions were then incubated at 15°C for 30 minutes, then at room temperature for 2 hours, before being heated to 37°C for 15 minutes.

Samples were boiled for 5 minutes to again denature the nucleic acid, before being snap cooled to allow renaturation of said nucleic acid. Another 0.5µl of the Klenow fragment of *E*. *coli* DNA polymerase I was added to each reaction and the samples incubated at 15°C for 30 minutes, then at room temperature for 2 hours, before being heated to 37°C for 15 minutes.

Following boiling the samples, following snap cooling another 2 rounds of primer extension were completed using the tagged random oligonucleotide:
5'-GACTACAAGGACGACGACGACAAGGCTTATCAATCAATCAN₉-3' (SEQ ID NO: 34)

To complete this the following reagents were added to the samples of the previous step:

| | |
|---|---|
| Oligonucleotide comprising SEQ ID NO 34 (25µM) | 4µl |
| Klenow Buffer | 1µl |
| dNTP(2mM) | 3µl |
| Klenow | 0.5µl |
| H₂O | to 40µl |

Samples were then incubated at 15°C for 30 minutes, then at room temperature for 2 hours, before being heated to 37°C for 15 minutes.

Samples were boiled for 5 minutes to again denature the nucleic acid, before being snap cooled to allow renaturation of said nucleic acid. Another 0.5µl of the Klenow fragment of *E*. *coli* DNA polymerase I was added to each reaction and the samples incubated at 15°C for 30 minutes, then at room temperature for 2 hours, before being heated to 37°C for 15 minutes.

Following completion of the primer extension amplification all sample volumes were increased to 500µl with TE buffer and added to an Amicon spin column. These columns were then centrifuged for 15 minutes at 3,800 rpm in a microcentrifuge. Columns were then inverted and 30µl of TE buffer was added before the columns were centrifuged for 2 minutes at 3,800rpm, with this fraction collected for later use. The Klenow amplified DNA was then used in subsequent DNA manipulations.

The now purified primer extension products were then used in a PCR reaction with an oligonucleotide comprising the following sequence:
5'-GAGA**GAATTCA**GGTCAGACTACAAGGACGACGACGACAAG-3' (SEQ ID NO: 35), wherein an *Eco*RI restriction endonuclease site is shown in bold text, and three stop codons are underlined. Note that each of the stop codons is in a different reading frame.

Thus, the following PCR reaction was used:

| | |
|---|---|
| Oligonucleotide comprising SEQ ID NO: 35 (10µM) | 12µl |
| PCR buffer | 5µl |
| dNTP (2mM) | 5µl |
| Taq polymerase (Boehringer) 5.5U/µl) | 0.4µl |
| H₂O | 26.6µl |
| Klenow amplified DNA | 2µl |

Reactions were then cycled in a thermocycler using the following program:
95°C for 2 min; 60°C for 30sec; 72°C for 1 min;
95°C for 20 sec; 60°C for 30sec; 72°C for 1 min (repeated 29 times); and
72°C for 5 min.

PCR products were then purified using Amicon spins columns which fractionate on the basis of size.

The PCR products were then analyzed by electrophoresis on standard TAE-agarose gels to determine the approximate size of the nucleic acid fragments generated as shown in Figure 2. The nucleic acid concentration of the samples was also determined.

PCR products from each of the 19 bacterial species were then pooled to generate a biodiverse nucleic acid library. To do so, DNA from each organism was added in an equimolar amount when compared to the amount of nucleic acid added to the pool from the organism with the smallest genome. Between 1 µg and 10µg of DNA from each organism was used, depending on the genome size of the organism from which the DNA was obtained.

In order to allow efficient cloning of the nucleic acid fragments into the pDEATH-Trp vector (SEQ ID NO: 36; Figure 3), both the fragments and the vector were digested with the EcoRI restriction endonuclease. Restriction digests were completed in the following reactions:

Digestion of PCR products used the following reaction conditions:

| PCR products (1µg) | |
|---|---|
| EcoR I Buffer (Promega) | 17µl |
| BSA (10x) | 17µl |
| EcoR I enzyme (20U/µL) (Promega) | 0.9µl |
| H₂0 | to 170 µl |

Restriction digests were allowed to proceed for 40 minutes at 37°C. Samples were then purified using QIAquick PCR purification columns as per manufacturer's instructions. Nucleic acid was eluted into 50µl of H₂O.

Digestion of pDEATH-Trp vector used the following reaction conditions:

| pDEATH-Trp (25µg) | |
|---|---|
| EcoR I Buffer (Promega) | 100µl |
| BSA(10X) | 100µl |
| EcoR I enzyme (20U/µL) | 4µl |
| H₂0 | to 1000µl |

Restriction digests were allowed to proceed for 5 minutes at 37°C. Samples were then purified using 3 QIAquick PCR purification columns as per manufacturer's instructions. Nucleic acid was eluted into 150µl of H₂O.

The fragments generated from the PCR products were then ligated into the pDEATH-Trp vector (SEQ ID NO 36) using the following reaction:

| | |
|---|---|
| pDEATH-Trp (2 µg) | |

| BGF-PCR Fragments (1µg) | |
|---|---|
| Ligation Buffer (10x) (NEB) | 20µl |
| T4 DNA Ligase (NEB) | 10µl |
| H₂0 | to 200µl |

Ligation reactions were allowed to proceed overnight at 16°C. The ligase was then heat inactivated by incubating the samples at 65°C for 30 minutes. Following completion of the ligation reaction sample volumes were increased to 500µl with TE buffer and added to an Amicon spin column. These columns were then centrifuged for 15 minutes at 3,800 rpm in a microcentrifuge. Columns were then inverted and 30µl of TE buffer was added before the columns were centrifuged for 2 minutes at 3,800rpm, with this fraction collected for later use.

The pDEATH-Trp vector containing the biodiverse nucleic acid fragment was then transformed into *E*. *coli* TOP 10 cells. Expression vectors were then isolated from bacteria using standard procedures. Restriction enzyme digestion of the isolated vectors using EcoRI was then used to characterise the size of the inserts contained in the library, as shown in Figure 4.

Vectors were then pooled and transformed into the yeast strain PRT 51. Yeast strain PRT-51 is characterized by the following genotype: *MATα, his3, trp1, ura3, 6 LexA-LEU2, lys2: 3 cIop-LYS2, CYH2^{R}, ade2:G418 pZero-ade2, met15: Zeo-pBLUE-met15, his5::hygro.*

The result of this transformation was a library of 61 million clones. The recombinant clones each express a peptide that is fused to another polynucleotide sequence encoding the FLAG epitope or other marker.

### EXAMPLE 2

### Characterization of a biodiverse nucleic acid fragment expression library in the pDEATH-Trp vector

Sequence analysis of nucleic acids cloned into pDEATH-Trp vector show that the fragments are derived from a variety of organisms, and encode a variety of proteins, as shown in Table 2.

**TABLE 2.**

| Characterization of nucleic acid fragment cloned into pDEATH-Trp | | | | |
|---|---|---|---|---|
| No. | Insert size (bp) | Organism | Genbank ID | Function |
| 1 | 114 | *P. aeruginosa* | AAG05339.1 | Hypothetical Protein |
| 2 | 143 | *Synechocystis PCC6803* | BAA10184.1 | Fructose |
| 3 | 166 | *E. coli* | AAC73742.1 | Lipoprotein |
| 4 | 180 | *B. subtilis* | CAB12555.1 | methyl-accepting chemotaxis protein |
| 5 | 150 | *N. meningitis* | AAF41991.1 | N utilization substance protein A |
| 6 | 240 | *E. coli* | AAC75637.1 | Hypothetical protein |
| 7 | 357 | *H. pylori* | AAD08555.1 | transcription termination factor NusA |
| 8 | 83 | *Z. maritima* | AAD36283.1 | Hypothetical protein |

### EXAMPLE 3

### Identification of a peptide capable of inhibiting the self-dimerization of c-Jun.

A biodiverse nucleic acid fragment library was produced in the vector pMF4-5 (Phylogica Ltd, Australia) (SEQ ID NO: 165) essentially as described in Example 1. Amplified fragmanets were digested with *EcoR*I and *Acc*651. The resulting fragments were then purified using a QIAQuick PCR purification column (Qiagen) essentially according to manufacturer's instructions. The expression vector pMF4-5 was also digested with *EcoR*I and *Acc*651, treated with shrimp alkaline phosphatase and then purified using a QIAQuick PCR purification column (Qiagen) essentially according to manufacturer's instructions. Ligations were then performed at a molar ratio of 10:1 insert:vector, and transformed into TOP10 electrocompetent cells (Invitrogen).

These vectors were then isolated from bacteria using standard methods and transformed into the PRT51 yeast strain (with the genotype MATα, his3, trp1, ura3, 6 LexA-LEU2, lys2::3 cIop-LYS2, CYH2R , ade2::G418-pZero-ade2, met15::Zeo-pBLUE-met15, his5::hygroR). Transformants were then aliquoted and snap frozen in 15% glycerol..

The bait and prey used in the present screen were JUN1 and JUNZ (these regions of c-Jun are shown in Figure 7). Briefly, nucleic acid encoding the JUN1 protein was cloned into the prey vector pJFK (SEQ ID NO: 60; Figure 5) in operable connection with a nuclear localisation signal, and a B42 activation domain. The nucleic acid encoding the JUNZ protein was cloned into the bait vector pDD (SEQ ID NO: 61; Figure 6) in operable connection with the LexA DNA binding domain. The pDD vector also contains a nucleic acid encoding the HIS3 gene (Figure 6). These vectors were then transformed into the yeast strain PRT480 (with the genotype MATα, his3, trp1, ura3, 4 LexA-LEU2, lys2::3 cIop-LYS2, CANR, CYH2R , ade2::2 LexA-CYH2-ZEO, his5::1 LexA-URA3-G418).

The yeast that carry the bait and prey proteins and the potential blocking peptides were then mass mated, and from approximately 300,000 clones, 95 positives were identified (ie, approximately 1/3000).

Two methods of analysis were used to identify interaction-blocking activity:
The first of these comprised plating approximately 500 cells per half plate onto HTU media containing plates and counting the number of colonies growing after 3 days. In these conditions, an interaction of JUN1 and JUNZ enables the cells to grow. Accordingly, a reduction in the number of colonies indicates that the library being screened comprises peptide inhibitors of the JUN1/JUNZ interaction.

The second screening method involved isolation and streaking of 10 individual colonies to new HTU media containing plates and analysing for growth of new single colonies. After 3 days, those that express a peptide inhibitor generally have very little or no new growth, while those that do not express a peptide inhibitor have re-grown a streak of single colonies. As a positive control a known inhibitor of JUN1/JUNZ interaction, FosZ was used. As a negative control empty pYTB3 vector (SEQ ID NO: 92) with no peptide insert was used. A score of 1-10 given depending on growth of 10 individual clones of each peptide copared to the two control samples.

The score from method 1 and method 2 was then combined to determine if a specific colony expressed a peptide inhibotor of JUN1/JUNZ interaction. In the present case a cell expressing a peptide inhibitor was one that showed >50% reduction of growth compared to negative control in both tests.

All scoring was performed by two independent individuals and scores of both individuals were combined.

Following screening it was found that 60 of the clones were capable of inhibiting the interaction of JUN1 and JUNZ.

Of the 60 clones identified, 27 were sequenced and analysed to determine their most likely source using BLAST-P. Results of this analysis are set forth in Table 3.

**Table 3: Characterisation of peptides capable of blocking the interaction of JUNZ and JUN1.**

| Peptide # | Length (aa) | Native ORF (Yes/No) | Species |
|---|---|---|---|
| 4 | 75 | No | *Bacillus subtilis* |
| 5 | 12 | No | *Aquifex aeolicus* |
| 8 | 39 | Yes | *Helicobacter pylorii* |
| 12 | 27 | Yes | *Escherichia coli* |
| 15 | 86 | Yes | *Escherichia coli* |
| 20 | 20 | No | *Helicobacter pylorii* |
| 21 | 25 | No | *Borrelia burgdorferi* |
| 22 | 40 | Yes | *Bordatella pertussis* |
| 24 | 26 | No | *Haemophilus influenzae* |
| 30 | 53 | No | *Pseudomonas aeruginosa* |
| 32 | 13 | No | *Plasmodium falciparum* |
| 33 | 11 | No | *Haemophilus influenzae* |
| 34 | 29 | No | *Aquifex aeolicus* |
| 35 | 62 | Yes | *Pyrococcus horikoshii* |
| 36 | 16 | Yes | *Bacillus subtilis* |
| 39 | 12 | No | *Bordatella pertussis* |
| 43 | 12 | No | *Neisseria meningitidis* |
| 54 | 32 | Yes | *Escherichia coli* |
| 58 | 45 | No | *Bacillus subtilis* |
| 60 | 20 | No | *Bacillus subtilis* |
| 66 | 39 | Yes | *Bacillus subtilis* |
| 72 | 38 | No | *Haemophilus influenzae* |
| 73 | 33 | No | *Pyrococcus horikoshit* |
| 76 | 24 | No | *Thermoplasma volcanium* |
| 77 | 18 | No | *Thermoplasma volcanium* |
| 79 | 12 | No | *Haemophilus influenzae* |
| 80 | 26 | Yes | *Bacillus subtilis* |

Note that 30% of the identified peptides are expressed in their native reading frame (ie they are identical to a region of a protein found in nature). This represents a significantly greater (p<0.009) number than would be expected by chance (as only 1 in 6 fragments would be expected to be in their native reading frame).

The sequence of the peptides identified in this screen are set forth in Table 4.

| Clone number | Source of nucleic acid | Native ORF (Y/N) | Nucleotide sequence | Sequence of peptide encoded by 1st ORF with flanking phage sequence | Sequence of peptide encoded by 1st ORF without flanking phage sequence |
|---|---|---|---|---|---|
| 4 | *B subtilis* | N | SEQ ID NO: 93 | SEQ ID NO: 94 | SEQ ID NO: 95 |
| 5 | *A aeolicus* | N | SEQ ID NO: 96 | SEQ ID NO: 97 | SEQ ID NO: 98 |
| 8 | *H Pylori* | Y | SEQ ID NO: 99 | SEQ ID NO: 100 | SEQ ID NO: 101 |
| 12 | *E. coli* | N | SEQ ID NO: 102 | SEQ ID NO:103 | SEQ ID NO:104 |
| 15 | *E. coli* | Y | SEQ ID NO: 105 | SEQ ID NO: 106 | SEQ ID NO: 107 |
| 20 | *H. pylori* | N | SEQ ID NO: 108 | SEQ ID NO: 109 | SEQ ID NO: 110 |
| 21 | *B burgdorferei* | N | SEQ ID NO: 111 | SEQ ID NO: 112 | SEQ ID NO: 113 |
| 22 | *B. pertussis* | Y | SEQ ID NO: 114 | SEQ ID NO:115 | SEQ ID NO: 116 |
| 24 | *H. influenzae* | N | SEQ ID NO: 117 | SEQ ID NO: 118 | SEQ ID NO: 119 |
| 30 | *P. aeruginosa* | Y | SEQ ID NO: 120 | SEQ ID NO: 121 | SEQ ID NO: 122 |
| 32 | *P. falciparum* | N | SEQ ID NO: 123 | SEQ ID NO: 124 | SEQ ID NO: 125 |
| 33 | *H. influenzae* | N | SEQ ID NO: 126 | SEQ ID NO: 127 | SEQ ID NO: 128 |
| 34 | *A. aeolicus* | Y | SEQ ID NO: 129 | SEQ ID NO: 130 | SEQ ID NO: 131 |
| 35 | *P. horikoshii* | Y | SEQ ID NO: 132 | SEQ IDNO: 133 | SEQ ID NO: 134 |
| 36 | *B. subtilis* | N | SEQ ID NO: 135 | SEQ ID NO: 136 | SEQ ID NO: 137 |
| 39 | *B. pertussis* | N | SEQ ID NO: 138 | SEQ ID NO: 139 | SEQ ID NO: 140 |
| 43 | *P. horikoshii* | N | SEQ ID NO: 141 | SEQ ID NO: 142 | SEQ ID NO: 143 |
| 54 | *Synechocystis PCC 6803* | Y | SEQ ID NO: 144 | SEQ ID NO: 145 | SEQ ID NO: 146 |
| 58 | *B.pertussis* | Y | SEQ ID NO: 147 | SEQ ID NO: 148 | SEQ ID NO: 149 |
| 60 | *N. meningitidis* | N | SEQ ID NO: 150 | SEQ ID NO: 151 | SEQ ID NO: 152 |
| 66 | *E. coli* | Y | SEQ ID NO: 153 | SEQ ID NO: 154 | SEQ ID NO: 155 |
| 72, 73, 76 and 77 | *B*. *subtilis* | N | SEQ ID NO: 156 | SEQ ID NO:157 | SEQ ID NO: 158 |
| 79 | *H. influenzae* | N | SEQ ID NO: 159 | SEQ ID NO: 160 | SEQ ID NO: 161 |
| 80 | *B. subtilis* | N | SEQ ID NO: 162 | SEQ ID NO: 163 | SEQ ID NO: 164 |

The ability of the peptides to interact with JUN1 was then confirmed with a forward two-hybrid assay. Each of the identified pepotides capable of inhibiting the interaction of JUN1 and JUNZ was cloned into the bait vector pDD (SEQ ID NO: 61; Figure 6). Additionally nucleci acid encoding a peptide known not to inhibit the interaction between JUN1 and JUNZ was also cloned into pDD. The pDD vector and the JUN1 prey vector was transformed into the yeast strain PRT480 and the interaction of the encoded peptide and JUN1 assessed by determining the amount of growthin the absence of uracil. An example of such a screen is shown in Figure 8.

### EXAMPLE 4

### Identifying those peptides capable of inhibiting neurodegeneration in a cellular model of Huntington's disease

Huntington disease is a chronic neuropathological disease characterized by preferential degeneration of striatal neurons. While the disease is known to be caused by a pathological expansion of a polyglutamine repeat in the huntingtin protein, the means by which neuronal degeneration occurs is unknown, making it difficult to identify potential therapeutics of this disease.

Chronic 3-nitropropionic acid (3-NP) administration in several model organisms has provided a similar pattern of neurodegeneration as seen in Huntington's disease subjects. Accordingly, 3-NP administration is a useful model for screening compounds for their utility in the treatment of a neurodegenerative disease, and, in particular, Huntington's disease.

The effect of the peptides identified in Example 3 is studied in a cellular model of Huntington's disease using 3-NP. Nucleic acid capable of encoding a peptide that comprises a sequence selected from the group consisting of SEQ ID NO: 94, SEQ ID NO: 95, SEQ ID NO: 97, SEQ ID NO: 98, SEQ ID NO: 100, SEQ ID NO: 101, SEQ ID NO: 103, SEQ ID NO: 104, SEQ ID NO: 106, SEQ ID NO: 107, SEQ ID NO: 109, SEQ ID NO:110,SEQ ID NO:112,SEQ ID NO:113,SEQ ID NO:115,SEQ ID NO: 116, SEQ ID NO: 118, SEQ ID NO: 119, SEQ ID NO: 121, SEQ ID NO: 122, SEQ ID NO: 124, SEQ ID NO: 125, SEQ ID NO: 127, SEQ ID NO: 128, SEQ ID NO: 130, SEQ ID NO: 131, SEQ ID NO: 133, SEQ ID NO: 134, SEQ ID NO: 136, SEQ ID NO: 137, SEQ ID NO: 139, SEQ ID NO: 140, SEQ ID NO: 142, SEQ ID NO: 143, SEQ ID NO: 145, SEQ ID NO: 146, SEQ ID NO: 148, SEQ ID NO: 149, SEQ ID NO: 151, SEQ ID NO: 152, SEQ ID NO: 154, SEQ ID NO: 155, SEQ ID NO: 157, SEQ ID NO: 158, SEQ ID NO: 160, SEQ ID NO: 161, SEQ ID NO: 163, SEQ ID NO: 164 and SEQ ID NO: 165 is cloned into the pcDNA3.1 mammalian expression vector (Invitogen).

The model used to study the effect of the peptides is an *in vitro* striatal neuron culture. To produce such a culture striata of fetal rat (embryonic day 17) from pregnant Sprague Dawley rats are dissected, and tissues dissociated by repeated trituration with a pipette in PBS and 0.6% glucose. After decantation for 5 min, cells are collected by centrifugation at 1000 × g for 5 min. Cell pellets are resuspended in Neurobasal media supplemented with B27, glutamine, penicillin-streptomycin (Invitrogen, Gaithersburg, MD), and β-mercaptoethanol (Sigma). Cells are seeded at 960 cells/mm² into poly-D-lysine (Sigma)-coated 24-well plates. The cultures are maintained at 37°C in a humidified incubator with 5% CO₂ and 95% air.

Cells are then transiently transfected with the expression construct produced previously with LipofectAMINE 2000 (Invitrogen) as recommended by the manufacturer's protocol. Cells (1.8 × 10⁵) are transfected with 1 µg of enhanced green fluorescent protein (pEGFP-N3; Clontech, Cambridge, UK) alone or in the of an expression vector produced previously. After 6 hr, the cultures are rinsed with fresh medium. Cells are then incubated for an appropriate period. On the seventh day *in vitro,* the medium is removed and replaced by fresh medium containing 3-NP (Fluka) at 1 mM.

Following the treatment with 3-NP, the cells are fixed using 4% paraformaldehyde in 0.1 M Na₂HPO₄NaH₂PO₄ buffer, pH 7.5.

The degree of cell death is then determined using TUNEL staining. The detection of DNA strand breaks is performed using terminal deoxynucleotidyl transferase-mediated biotinylated UTP nick end-labeling (TUNEL) according to the procedure of the manufacturers (Roche Molecular Biochemicals, Bagnolet, France) with minor modifications. Briefly, sections are mounted on slides and rehydrated. They are then treated with 0.1% sodium citrate and 0.1% Triton X-100 for 30min at room temperature and rinsed three times in PBS. They are then incubated in proteinase K (1 mg/ml in PBS, pH 7.4) for 5 min, reimmersed in 4% paraformaldehyde for 15 min, and rinsed three times in PBS before TUNEL reactions. Sections are then covered with 50 µl of TUNEL mixture for 30 min at 37°C in a humidified chamber. After three washes in PBS, the slides are mounted with Vectashield (Vector Laboratories).

Cells are also monitored for changes in neurite outgrowths and changes in the suze of the cell body, both of which are measures of neuronal dysfunction.

Those peptides that are inhibit c-Jun hoodimerization and reduce or inhibit cell death and/or neuronal dysfunction are selected as these peptides are useful for further study for their utility in the treatment of neurodegenerative disease.

## Claims

1. An isolated peptide or protein domain comprising or consisting of an amino acid sequence set forth in SEQ ID NO: 133 or SEQ ID NO: 134.

2. An isolated nucleic acid encoding a peptide or protein domain according to claim 1.

3. An isolated nucleic acid according to claim 2, comprising a sequence set forth in SEQ ID NO: 132.

4. A pharmaceutical formulation comprising the isolated peptide or protein domain according to claim 1 in combination with a physiologically acceptable vehicle or carrier.

5. A pharmaceutical formulation comprising the isolated nucleic acid according to claim 2 or claim 3 in combination with a physiologically acceptable vehicle or carrier.

6. An isolated peptide or protein domain according to claim 1 for use in a method of medical treatment.

7. An isolated nucleic acid according to claim 2 or claim 3 for use in a method of medical treatment.

8. Use of an isolated peptide or protein domain according to claim 1 in the preparation of a medicament for the treatment of a neurodegenerative disease.

9. Use of an isolated nucleic acid according to claim 2 or claim 3 in the preparation of a medicament for the treatment of a neurodegenerative disease.

10. Use according to claim 8 or claim 9 wherein the neurodegenerative disease is Huntington's disease.

11. An isolated peptide or protein domain according to claim 1 for use in a method of treatment of a neurodegenerative disease.

12. An isolated nucleic acid according to claim 2 or claim 3 for use in a method of treatment of a neurodegenerative disease.

13. An isolated peptide or protein domain for use according to claim 11, or isolated nucleic acid for use according to claim 12, wherein the neurodegenerative disease is Huntington's disease.

14. A method for inhibiting dimerization of c-Jun comprising administering an isolated peptide or protein domain according to claim 1, or a nucleic acid according to claim 2 or claim 3, in vitro, to a cell comprising or expressing a c-Jun polypeptide.

## Patentansprüche

1. Isolierte Peptid- oder Proteindomäne, umfassend oder bestehend aus eine(r) Aminosäuresequenz, die in Seq.-ID Nr. 133 oder Seq.-ID Nr. 134 dargelegt ist.

2. Isolierte Nucleinsäure, die für eine Peptid- oder Proteindomäne nach Anspruch 1 kodiert.

3. Isolierte Nucleinsäure nach Anspruch 2, die eine Sequenz umfasst, die in Seq.-ID Nr. 132 dargelegt ist.

4. Pharmazeutische Formulierung, umfassend eine isolierte Peptid- oder Proteindomäne nach Anspruch 1 in Kombination mit einem physiologisch annehmbaren Vehikel oder Träger.

5. Pharmazeutische Formulierung, umfassend eine isolierte Nucleinsäure nach Anspruch 2 oder Anspruch 3 in Kombination mit einem physiologisch annehmbaren Vehikel oder Träger.

6. Isolierte Peptid- oder Proteindomäne nach Anspruch 1 zur Verwendung in einem medizinischen Behandlungsverfahren.

7. Isolierte Nucleinsäure nach Anspruch 2 oder Anspruch 3 zur Verwendung in einem medizinischen Behandlungsverfahren.

8. Verwendung einer isolierten Peptid- oder Proteindomäne nach Anspruch 1 zur Herstellung eines Medikaments zur Behandlung einer neurodegenerativen Erkrankung.

9. Verwendung einer isolierten Nucleinsäure nach Anspruch 2 oder Anspruch 3 zur Herstellung eines Medikaments zur Behandlung einer neurodegenerativen Erkrankung.

10. Verwendung nach Anspruch 8 oder Anspruch 9, worin es sich bei der neurodegenerativen Erkrankung um die Huntington-Krankheit handelt.

11. Isolierte Peptid- oder Proteindomäne nach Anspruch 1 zur Verwendung in einem Behandlungsverfahren für eine neurodegenerativen Erkrankung.

12. Isolierte Nucleinsäure nach Anspruch 2 oder Anspruch 3 zur Verwendung in einem Behandlungsverfahren für eine neurodegenerative Erkrankung.

13. Isolierte Peptid- oder Proteindomäne zur Verwendung nach Anspruch 11 oder isolierte Nucleinsäure zur Verwendung nach Anspruch 12, worin es sich bei der neurodegenerativen Erkrankung um die Huntington-Krankheit handelt.

14. Verfahren zur Inhibierung der Dimerisierung von c-Jun, umfassend das Verabreichen einer isolierten Peptid- oder Proteindomäne nach Anspruch 1 oder einer Nucleinsäure nach Anspruch 2 oder Anspruch 3 in vitro an eine Zelle, die ein c-Jun-Polypeptid umfasst oder exprimiert.

## Revendications

1. Domaine de peptide isolé ou de protéine comprenant ou consistant en une séquence d'acides aminés exposée dans SEQ ID NO: 133 ou SEQ ID NO: 134.

2. Acide nucléique isolé codant pour un domaine de peptide ou de protéine selon la revendication 1.

3. Acide nucléique isolé selon la revendication 2, comprenant une séquence exposée dans SEQ ID NO: 132.

4. Formulation pharmaceutique comprenant le domaine de peptide isolé ou de protéine selon la revendication 1 en combinaison avec un véhicule ou support physiologiquement acceptable.

5. Formulation pharmaceutique comprenant l'acide nucléique isolé selon la revendication 2 ou la revendication 3 en combinaison avec un véhicule ou support physiologiquement acceptable.

6. Domaine de peptide isolé ou de protéine selon la revendication 1, pour utilisation dans une méthode de traitement médical.

7. Acide nucléique isolé selon la revendication 2 ou la revendication 3 pour utilisation dans une méthode de traitement médical.

8. Utilisation d'un domaine de peptide isolé ou de protéine selon la revendication 1 dans la préparation d'un médicament pour le traitement d'une maladie neurodégénérative.

9. Utilisation d'un acide nucléique isolé selon la revendication 2 ou la revendication 3 dans la préparation d'un médicament pour le traitement d'une maladie neurodégénérative.

10. Utilisation selon la revendication 8 ou la revendication 9, où la maladie neurodégénérative est la maladie de Huntington.

11. Domaine de peptide isolé ou de protéine selon la revendication 1 pour utilisation dans une méthode de traitement d'une maladie neurodégérative.

12. Acide nucléique isolé selon la revendication 2 ou la revendication 3 pour utilisation dans une méthode de traitement d'une maladie neurodégérative.

13. Domaine de peptide isolé ou de protéine pour utilisation selon la revendication 11, ou acide nucléique isolé pour utilisation selon la revendication 12, où la maladie neurodégénérative est la maladie de Huntington.

14. Méthode pour inhiber la dimérisation de c-Jun comprenant l'administration d'un domaine de peptide isolé ou de protéine selon la revendication 1, ou d'un acide nucléique selon la revendication 2 ou la revendication 3, in vitro, à une cellule comprenant ou exprimant un polypeptide de c-Jun.


### SEQUENCE LISTING

<110> Phylogica Limited
<120> Methods of constructing and screening diverse expression libraries <130> 502133
<160> 197
<170> PatentIn version 3.1
<210> 1
   <211> 6
   <212> DNA
   <213> Kozak consensus sequence
<220>
   <221> misc_feature
   <222> (2)..(3)
   <223> N at position 2 and 3 is any nucleotide
<400> 1
   rnnatg 6
<210> 2
   <211> 8
   <212> DNA
   <213> Kozak consensus sequence
<400> 2
   ccrccatg 8
<210> 3
   <211> 11
   <212> DNA
   <213> Kozak consensus sequence
<400> 3
   gccagccatg g 11
<210> 4
   <211> 8
   <212> DNA
   <213> Kozak consensus sequence
<400> 4
   ctaccatg 8
<210> 5
   <211> 10
   <212> DNA
   <213> Shine Dalgarno sequence
<400> 5
   gaagaagata 10
<210> 6
   <211> 7
   <212> DNA
   <213> translational slippage sequence
<400> 6
   aaaaaac 7
<210> 7
   <211> 7
   <212> DNA
   <213> translational slippage sequence
<400> 7
   aaattta 7
<210> 8
   <211> 7
   <212> DNA
   <213> translational slippage sequence
<400> 8
   aaatttt 7
<210> 9
   <211> 7
   <212> DNA
   <213> translational slippage sequence
<400> 9
   gggaaac 7
<210> 10
   <211> 7
   <212> DNA
   <213> translational slippage sequence
<400> 10
   gggcccc 7
<210> 11
   <211> 7
   <212> DNA
   <213> translational slippage sequence
<400> 11
   gggttta 7
<210> 12
   <211> 7
   <212> DNA
   <213> translational slippage sequence
<400> 12
   gggtttt 7
<210> 13
   <211> 7
   <212> DNA
   <213> translational slippage sequence
<400> 13
   tttaaac 7
<210> 14
   <211> 7
   <212> DNA
   <213> translational slippage sequence
<400> 14
   tttaaat 7
<210> 15
   <211> 6
   <212> DNA
   <213> translational slippage sequence
<400> 15
   ttttta 6
<210> 16
   <211> 7
   <212> DNA
   <213> translational slippage sequence
<400> 16
   ggattta 7
<210> 17
   <211> 7
   <212> DNA
   <213> translational slippage sequence
<400> 17
   cttaggc 7
<210> 18
   <211> 7
   <212> DNA
   <213> translational slippage sequence
<400> 18
   gcgagtt 7
<210> 19
   <211> 7
   <212> DNA
   <213> translational slippage sequence
<400> 19
   tcctgat 7
<210> 20
   <211> 7
   <212> DNA
   <213> translational slippage sequence
<400> 20
   aaaaaag 7
<210> 21
   <211> 7
   <212> DNA
   <213> translational slippage sequence
<400> 21
   aaaaaaa 7
<210> 22
   <211> 7
   <212> DNA
   <213> translational slippage sequence
<400> 22
   aaaaaac 7
<210> 23
   <211> 7
   <212> DNA
   <213> translational slippage sequence
<400> 23
   gggaaag 7
<210> 24
   <211> 7
   <212> DNA
   <213> translational slippage sequence
<400> 24
   aaaaggg 7
<210> 25
   <211> 7
   <212> DNA
   <213> translational slippage sequence
<400> 25
   gggaaaa 7
<210> 26
   <211> 7
   <212> DNA
   <213> translational slippage sequence
<400> 26
   tttaaag 7
<210> 27
   <211> 7
   <212> DNA
   <213> translational slippage sequence
<400> 27
   aaagggg 7
<210> 28
   <211> 3
   <212> DNA
   <213> translational slippage sequence
<400> 28
   ctt 3
<210> 29
   <211> 17
   <212> PRT
   <213> Drosophila penetratin targetting sequence
<400> 29
   Cys Arg Gln Ile Lys Ile Trp Phe Gln Asn Arg Arg Met Lys Trp Lys
   1 5 10 15
   Lys
<210> 30
   <211> 21
   <212> PRT
   <213> syntehtic Pep1 peptide
<400> 30
   Lys Glu Thr Trp Trp Glu Thr Trp Trp Thr Glu Trp Ser Gln Lys Lys
   1 5 10 15
   Lys Lys Arg Lys Val
   20
<210> 31
   <211> 5
   <212> DNA
   <213> initiator sequence recognized by P2A protein
<400> 31
   tcgga 5
<210> 32
   <211> 5
   <212> PRT
   <213> membrane anchor sequence
<400> 32
   Pro Asp Gly Phe Arg
   1 5
<210> 33
   <211> 46
   <212> DNA
   <213> synthetic oligonucleotide primer
<220>
   <221> misc_feature
   <222> (41)..(46)
   <223> N at any one of positions 41 through 46 is any nucleotide residue
<400> 33
   gactacaagg acgacgacga caaggcttat caatcaatca nnnnnn 46
<210> 34
   <211> 49
   <212> DNA
   <213> synthetic oligonucleotide primer
<220>
   <221> misc_feature
   <223> N at any one of positions 41 through 49 is any nucleotide residue
<220>
   <221> misc_feature
   <222> (41)..(49)
   <223> N at any one of positions 41 through 49 is any nucleotide residue
<400> 34
   gactacaagg acgacgacga caaggcttat caatcaatca nnnnnnnnn 49
<210> 35
   <211> 40
   <212> DNA
   <213> synthetic oligonucleotide primer
<400> 35
   gagagaattc aggtcagact acaaggacga cgacgacaag 40
<210> 36
   <211> 5562
   <212> DNA
   <213> pDEATH-TRYP vector
<400> 36
   ctagcgattt tggtcatgag atcagatcaa cttcttttct ttttttttct tttctctctc     60
   ccccgttgtt gtctcaccat atccgcaatg acaaaaaaat gatggaagac actaaaggaa    120
   aaaattaacg acaaagacag caccaacaga tgtcgttgtt ccagagctga tgaggggtat    180
   ctcgaagcac acgaaacttt ttccttcctt cattcacgca cactactctc taatgagcaa    240
   cggtatacgg ccttccttcc agttacttga atttgaaata aaaaaaagtt tgctgtcttg    300
   ctatcaagta taaatagacc tgcaattatt aatcttttgt ttcctcgtca ttgttctcgt    360
   tccctttctt ccttgtttct ttttctgcac aatatttcaa gctataccaa gcatacaatc    420
   aactccaagc ttccccggat cggactacta gcagctgtaa tacgactcac tatagggaat 480
   attaagctca ccatgggtaa gcctatccct aaccctctcc tcggtctcga ttctacacaa 540
   gctatgggtg ctcctccaaa aaagaagaga aaggtagctg aattcgagct cagatctcag 600
   ctgggcccgg taccaattga tgcatcgata ccggtactag tcggaccgca tatgcccggg 660
   cgtaccgcgg ccgctcgagg catgcatcta gagggccgca tcatgtaatt agttatgtca 720
   cgcttacatt cacgccctcc ccccacatcc gctctaaccg aaaaggaagg agttagacaa 780
   cctgaagtct aggtccctat ttattttttt atagttatgt tagtattaag aacgttattt 840
   atatttcaaa tttttctttt ttttctgtac agacgcgtgt acgcatgtaa cattatactg 900
   aaaaccttgc ttgagaaggt tttgggacgc tcgaaggctt taatttgcgg ccctgcatta 960
   atgaatcggc caacgcgcgg ggagaggcgg tttgcgtatt gggcgctctt ccgcttcctc 1020
   gctcactgac tcgctgcgct cggtcgttcg gctgcggcga gcggtatcag ctcactcaaa 1080
   ggcggtaata cggttatcca cagaatcagg ggataacgca ggaaagaaca tgtgagcaaa 1140
   aggccagcaa aagcccagga accgtaaaaa ggccgcgttg ctggcgtttt tccataggct 1200
   ccgcccccct gacgagcatc acaaaaatcg acgctcaagt cagaggtggc gaaacccgac 1260
   aggactataa agataccagg cgtttccccc tggaagctcc ctcgtgcgct ctcctgttcc 1320
   gaccctgccg cttaccggat acctgtccgc ctttctccct tcgggaagcg tggcgctttc 1380
   tcatagctca cgctgtaggt atctcagttc ggtgtaggtc gttcgctcca agctgggctg 1440
   tgtgcacgaa ccccccgttc agcccgaccg ctgcgcctta tccggtaact atcgtcttga 1500
   gtccaacccg gtaagacacg acttatcgcc actggcagca gccactggta acaggattag 1560
   cagagcgagg tatgtaggcg gtgctacaga gttcttgaag tggtggccta actacggcta 1620
   cactagaagg acagtatttg gtatctgcgc tctgctgaag ccagttacct tcggaaaaag 1680
   agttggtagc tcttgatccg gcaaacaaac caccgctggt agcggtggtt tttttgtttg 1740
   caagcagcag attacgcgca gaaaaaaagg atctcaagaa gatcctttga tcttttctac 1800
   ggggtctgac gctcagtgga acgaaaactc acgttaaggg attttggtca tgagattatc 1860
   aaaaaggatc ttcacctaga tccttttaaa ttaaaaatga agttttaaat caatctaaag 1920
   tatatatgag taaacttggt ctgacagtta ccaatgctta atcagtgagg cacctatctc 1980
   agcgatctgt ctatttcgtt catccatagt tgcctgactc cccgtcgtgt agataactac 2040
   gatacgggag cgcttaccat ctggccccag tgctgcaatg ataccgcgag acccacgctc 2100
   accggctcca gatttatcag caataaacca gccagccgga agggccgagc gcagaagtgg 2160
   tcctgcaact ttatccgcct ccatccagtc tattaattgt tgccgggaag ctagagtaag 2220
   tagttcgcca gttaatagtt tgcgcaacgt tgttgccatt gctacaggca tcgtggtgtc 2280
   acgctcgtcg tttggtatgg cttcattcag ctccggttcc caacgatcaa ggcgagttac 2340
   atgatccccc atgttgtgca aaaaagcggt tagctccttc ggtcctccga tcgttgtcag 2400
   aagtaagttg gccgcagtgt tatcactcat ggttatggca gcactgcata attctcttac 2460
   tgtcatgcca tccgtaagat gcttttctgt gactggtgag tactcaacca agtcattctg 2520
   agaatagtgt atgcggcgac cgagttgctc ttgcccggcg tcaacacggg ataataccgc 2580
   gccacatagc agaactttaa aagtgctcat cattggaaaa cgttcttcgg ggcgaaaact 2640
   ctcaaggatc ttaccgctgt tgagatccag ttcgatgtaa cccactcgtg cacccaactg 2700
   atcttcagca tcttttactt tcaccagcgt ttctgggtga gcaaaaacag gaaggcaaaa 2760
   tgccgcaaaa aagggaataa gggcgacacg gaaatgttga atactcatac tcttcctttt 2820
   tcaatattat tgaagcattt atcagggtta ttgtctcatg agcggataca tatttgaatg 2880
   tatttagaaa aataaacaaa taggggttcc gcgcacattt ccccgaaaag tgccacctga 2940
   cgtctaagaa accattatta tcatgacatt aacctataaa aataggcgta tcacgaggcc 3000
   ctttcgtctt caagaaattc ggtcgaaaaa agaaaaggag agggccaaga gggagggcat 3060
   tggtgactat tgagcacgtg agtatacgtg attaagcaca caaaggcagc ttggagtatg 3120
   tctgttatta atttcacagg tagttctggt ccattggtga aagtttgcgg cttgcagagc 3180
   acagaggccg cagaatgtgc tctagattcc gatgctgact tgctgggtat tatatgtgtg 3240
   cccaatagaa agagaacaat tgacccggtt attgcaagga aaatttcaag tcttgtaaaa 3300
   gcatataaaa atagttcagg cactccgaaa tacttggttg gcgtgtttcg taatcaacct 3360
   aaggaggatg ttttggctct ggtcaatgat tacggcattg atatcgtcca actgcacgga 3420
   gatgagtcgt ggcaagaata ccaagagttc ctcggtttgc cagttattaa aagactcgta 3480
   tttccaaaag actgcaacat actactcagt gcagcttcac agaaacctca ttcgtttatt 3540
   cccttgtttg attcagaagc aggtgggaca ggtgaacttt tggattggaa ctcgatttct 3600
   gactgggttg gaaggcaaga gagccccgag agcttacatt ttatgttagc tggtggactg 3660
   acgccagaaa atgttggtga tgcgcttaga ttaaatggcg ttattggtgt tgatgtaagc 3720
   ggaggtgtgg agacaaatgg tgtaaaagac tctaacaaaa tagcaaattt cgtcaaaaat 3780
   gctaagaaat aggttattac tgagtagtat ttatttaagt attgtttgtg cacttgcctg 3840
   cagcttctca atgatattcg aatacgcttt gaggagatac agcctaatat ccgacaaact 3900
   gttttacaga tttacgatcg tacttgttac ccatcattga attttgaaca tccgaacctg 3960
   ggagttttcc ctgaaacaga tagtatattt gaacctgtat aataatatat agtctagcgc 4020
   tttacggaag acaatgtatg tatttcggtt cctggagaaa ctattgcatc tattgcatag 4080
   gtaatcttgc acgtcgcatc cccggttcat tttctgcgtt tccatcttgc acttcaatag 4140
   catatctttg ttaacgaagc atctgtgctt cattttgtag aacaaaaatg caacgcgaga 4200
   gcgctaattt ttcaaacaaa gaatctgagc tgcattttta cagaacagaa atgcaacgcg 4260
   aaagcgctat tttaccaacg aagaatctgt gcttcatttt tgtaaaacaa aaatgcaacg   4320
   cgagagcgct aatttttcaa acaaagaatc tgagctgcat ttttacagaa cagaaatgca    4380
   acgcgagagc gctattttac caacaaagaa tctatacttc ttttttgttc tacaaaaatg   4440
   catcccgaga gcgctatttt tctaacaaag catcttagat tacttttttt ctcctttgtg  4500
   cgctctataa tgcagtctct tgataacttt ttgcactgta ggtccgttaa ggttagaaga   4560
   aggctacttt ggtgtctatt ttctcttcca taaaaaaagc ctgactccac ttcccgcgtt   4620
   tactgattac tagcgaagct gcgggtgcat tttttcaaga taaaggcatc cccgattata   4680
   ttctataccg atgtggattg cgcatacttt gtgaacagaa agtgatagcg ttgatgattc   4740
   ttcattggtc agaaaattat gaacggtttc ttctattttg tctctatata ctacgtatag   4800
   gaaatgttta cattttcgta ttgttttcga ttcactctat gaatagttct tactacaatt   4860
   tttttgtcta aagagtaata ctagagataa acataaaaaa tgtagaggtc gagtttagat   4920
   gcaagttcaa ggagcgaaag gtggatgggt aggttatata gggatatagc acagagatat   4980
   atagcaaaga gatacttttg agcaatgttt gtggaagcgg tattcgcaat gggaagctcc   5040
   accccggttg ataatcagaa aagccccaaa aacaggaaga ttgtataagc aaatatttaa   5100
   attgtaaacg ttaatatttt gttaaaattc gcgttaaatt tttgttaaat cagctcattt   5160
   tttaacgaat agcccgaaat cggcaaaatc ccttataaat caaaagaata gaccgagata   5220
   gggttgagtg ttgttccagt ttccaacaag agtccactat taaagaacgt ggactccaac   5280
   gtcaaagggc gaaaaagggt ctatcagggc gatggcccac tacgtgaacc atcaccctaa   5340
   tcaagttttt tggggtcgag gtgccgtaaa gcagtaaatc ggaagggtaa acggatgccc   5400
   ccatttagag cttgacgggg aaagccggcg aacgtggcga gaaaggaagg gaagaaagcg   5460
   aaaggagcgg gggctagggc ggtgggaagt gtaggggtca cgctgggcgt aaccaccaca   5520
   cccgccgcgc ttaatggggc gctacagggc gcgtggggat ga                      5562
<210> 37
   <211> 40
   <212> DNA
   <213> synthetic oligonucleotide primer
<400> 37
   agaggaattc aggtcagact acaaggacga cgacgacaag 40
<210> 38
   <211> 27
   <212> DNA
   <213> synthetic oligonucleotide primer
<400> 38
   cagaagctta aggacgacga cgacaag 27
<210> 39
   <211> 27
   <212> DNA
   <213> synthetic oligonucleotide primer
<400> 39
   caggaattca aggacgacga cgacaag 27
<210> 40
   <211> 28
   <212> DNA
   <213> synthetic oligonucleotide primer
<400> 40
   caggaattcc aaggacgacg acgacaag 28
<210> 41
   <211> 29
   <212> DNA
   <213> synthetic oligonucleotide primer
<400> 41
   caggaattca caaggacgac gacgacaag 29
<210> 42
   <211> 16
   <212> DNA
   <213> synthetic adaptor oligonucleotide
<400> 42
   aattcgaacc ccttcg 16
<210> 43
   <211> 12
   <212> DNA
   <213> synthetic adaptor oligonucleotide
<400> 43
   cgaaggggtt cg 12
<210> 44
   <211> 17
   <212> DNA
   <213> synthetic adaptor oligonucleotide
<400> 44
   aattcgaacc ccttcgc 17
<210> 45
   <211> 13
   <212> DNA
   <213> synthetic adaptor oligonucleotide
<400> 45
   gcgaaggggt tcg 13
<210> 46
   <211> 18
<212> DNA
   <213> synthetic adaptor oligonucleotide
<400> 46
   aattcgaacc ccttcgcg 18
<210> 47
   <211> 13
   <212> DNA
   <213> synthetic adaptor oligonucleotide
<400> 47
   gcgaaggggt tcg 13
<210> 48
   <211> 16
   <212> DNA
   <213> synthetic adaptor oligonucleotide
<400> 48
   agctcgaagg ggttcg 16
<210> 49
   <211> 12
   <212> DNA
   <213> synthetic adaptor oligonucleotide
<400> 49
   cgaacccctt cg 12
<210> 50
   <211> 8
   <212> PRT
   <213> FLAG epitope
<400> 50
   Asp Tyr Lys Asp Asp Asp Asp Lys
   1               5
<210> 51
   <211> 31
   <212> DNA
   <213> synthetic FLAG-encoding oligonucleotide
<400> 51
   aattccgact acaaggacga cgatgacaag a 31
<210> 52
   <211> 31
   <212> DNA
   -<213> synthetic FLAG-encoding oligonucleotide
<400> 52
   agcttcttgt catcgtcgtc cttgtagtcg g 31
<210> 53
   <211> 89
   <212> DNA
   <213> synthetic tagged random oligonucleotide
<220>
   <221> misc_feature
   <222> (81)..(89)
   <223>
<220>
   <221> misc_feature
   <222> (81)..(89)
   <223> N at any one of positions 81 through 89 is any nucleotide residue
<400> 53
   tttcccgaat tgtgagcgga taacaataga aataattttg tttaacttta agaaggagat 60
   atatccatgg actacaaaga nnnnnnnnn 89
<210> 54
   <211> 61
   <212> DNA
   <213> synthetic oligonucleotide
<400> 54
   ggggccaagc agtaataata cgagtcacta tagggagacc acaacggttt cccgaattgt 60
   g 61
<210> 55
   <211> 23
   <212> DNA
   <213> synthetic adaptor oligonucleotide
<400> 55
   tttaagcagc tcgatagcag cac 23
<210> 56
   <211> 23
   <212> DNA
   <213> synthetic adaptor oligonucleotide
<400> 56
   gtgctgctat cgagctgctt aaa 23
<210> 57
   <211> 51
   <212> DNA
   <213> synthetic lipoprotein terminator oligonucleotide
<400> 57
   agacccgttt agaggcccca aggggttatg gaattcacct ttaagcagct c 51
<210> 58
   <211> 26
   <212> DNA
   <213> synthetic M13 gene III oligonucleotide
<400> 58
   cgtgaaaaaa ttattattcg caattc 26
<210> 59
   <211> 30
   <212> DNA
   <213> synthetic M13 gene III oligonucleotide
<400> 59
   ttaagactcc ttattacgca gtatgttagc 30
<210> 60
   <211> 7551
   <212> DNA
   <213> pJFK vector.
<400> 60
   ccccattatc ttagcctaaa aaaaccttct ctttggaact ttcagtaata cgcttaactg 60
   ctcattgcta tattgaagta cggattagaa gccgccgagc gggtgacagc cctccgaagg 120
   aagactctcc tccgtgcgtc ctcgtcttca ccggtcgcgt tcctgaaacg cagatgtgcc 180
   tcgcgccgca ctgctccgaa caataaagat tctacaatac tagcttttat ggttatgaag 240
   aggaaaaatt ggcagtaacc tggccccaca aaccttcaaa tgaacgaatc aaattaacaa 300
   ccataggatg ataatgcgat tagtttttta gccttatttc tggggtaatt aatcagcgaa 360
   gcgatgattt ttgatctatt aacagatata taaatgcaaa aactgcataa ccactttaac 420
   taatactttc aacattttcg gtttgtatta cttcttattc aaatgtaata aaagtatcaa 480
   caaaaaattg ttaatatacc tctatacttt aacgtcaagg aggaattaag cttatgggtg 540
   ctcctccaaa aaagaagaga aaggtagctg gtatcaataa agatatcgag gagtgcaatg 600
   ccatcattga gcagtttatc gactacctgc gcaccggaca ggagatgccg atggaaatgg 660
   cggatcaggc gattaacgtg gtgccgggca tgacgccgaa aaccattctt cacgccgggc 720
   cgccgatcca gcctgactgg ctgaaatcga atggttttca tgaaattgaa gcggatgtta 780
   acgataccag cctcttgctg agtggagatg cctcctaccc ttatgatgtg ccagattatg 840
   cctctcccga attcggccga ctcgagaagc tttggacttc ttcgccagag gtttggtcaa 900
   gtctccaatc aaggttgtcg gcttgtctac cttgccagaa atttacgaaa agatggaaaa 960
   gggtcaaatc gttggtagat acgttgttga cacttctaaa taagcgaatt tcttatgatt 1020
   tatgattttt attattaaat aagttataaa aaaaataagt gtatacaaat tttaaagtga 1080
   ctcttaggtt ttaaaacgaa aattcttgtt cttgagtaac tctttcctgt aggtcaggtt 1140
   gctttctcag gtatagcatg aggtcgctct tattgaccac acctctaccg gcatgccgag 1200
   caaatgcctg caaatcgctc cccatttcac ccaattgtag atatgctaac tccagcaatg 1260
   agttgatgaa tctcggtgtg tattttatgt cctcagagga caacacctgt tgtaatcgtt 1320
   cttccacacg gatcctctag agtcgactag cggccgcttc gacctgcagc aattctgaac 1380
   cagtcctaaa acgagtaaat aggaccggca attcttcaag caataaacag gaataccaat 1440
   tattaaaaga taacttagtc agatcgtaca ataaagcttt gaagaaaaat gcgccttatt 1500
   caatctttgc tataaaaaat ggcccaaaat ctcacattgg aagacatttg atgacctcat 1560
   ttctttcaat gaagggccta acggagttga ctaatgttgt gggaaattgg agcgataagc 1620
   gtgcttctgc cgtggccagg acaacgtata ctcatcagat aacagcaata cctgatcact 1680
   acttcgcact agtttctcgg tactatgcat atgatccaat atcaaaggaa atgatagcat 1740
   tgaaggatga gactaatcca attgaggagt ggcagcatat agaacagcta aagggtagtg 1800
   ctgaaggaag catacgatac cccgcatgga atgggataat atcacaggag gtactagact 1860
   acctttcatc ctacataaat agacgcatat aagtacgcat ttaagcataa acacgcacta 1920
   tgccgttctt ctcatgtata tatatataca ggcaacacgc agatataggt gcgacgtgaa 1980
   cagtgagctg tatgtgcgca gctcgcgttg cattttcgga agcgctcgtt ttcggaaacg 2040
   ctttgaagtt cctattccga agttcctatt ctctagaaag tataggaact tcagagcgct 2100
   tttgaaaacc aaaagcgctc tgaagacgca ctttcaaaaa accaaaaacg caccggactg 2160
   taacgagcta ctaaaatatt gcgaataccg cttccacaaa cattgctcaa aagtatctct 2220
   ttgctatata tctctgtgct atatccctat ataacctacc catccacctt tcgctccttg 2280
   aacttgcatc taaactcgac ctctacattt tttatgttta tctctagtat tactctttag 2340
   acaaaaaaat tgtagtaaga actattcata gagtgaatcg aaaacaatac gaaaatgtaa 2400
   acatttccta tacgtagtat atagagacaa aatagaagaa accgttcata attttctgac 2460
   caatgaagaa tcatcaacgc tatcactttc tgttcacaaa gtatgcgcaa tccacatcgg 2520
   tatagaatat aatcggggat gcctttatct tgaaaaaatg cacccgcagc ttcgctagta 2580
   atcagtaaac gcgggaagtg gagtcaggct ttttttatgg aagagaaaat agacaccaaa 2640
   gtagccttct tctaacctta acggacctac agtgcaaaaa gttatcaaga gactgcatta 2700
   tagagcgcac aaaggagaaa aaaagtaatc taagatgctt tgttagaaaa atagcgctct 2760
   cgggatgcat ttttgtagaa caaaaaagaa gtatagattc tttgttggta aaatagcgct 2820
   ctcgcgttgc atttctgttc tgtaaaaatg cagctcagat tctttgtttg aaaaattagc 2880
   gctctcgcgt tgcatttttg ttttacaaaa atgaagcaca gattcttcgt tggtaaaata 2940
   gcgctttcgc gttgcatttc tgttctgtaa aaatgcagct cagattcttt gtttgaaaaa 3000
   ttagcgctct cgcgttgcat ttttgttcta caaaatgaag cacagatgct tcgttaacaa 3060
   agatatgcta ttgaagtgca agatggaaac gcagaaaatg aaccggggat gcgacgtgca 3120
   agattaccta tgcaatagat gcaatagttt ctccaggaac cgaaatacat acattgtctt 3180
   ccgtaaagcg ctagactata tattattata caggttcaaa tatactatct gtttcaggga 3240
   aaactcccag gttcggatgt tcaaaattca atgatgggta acaagtacga tcgtaaatct 3300
   gtaaaacagt ttgtcggata ttaggctgta tctcctcaaa gcgtattcga tctgtctttc 3360
   gccgaaacct gtttgatgac tacttcatca attttttttt tttctgccgc attccaaagg 3420
   tcataacttt gcaaaaataa agggtaaatg gttaaaaatt gttatcataa ataaggtgac 3480
   cggttatatt gagacctttc ctggacagta actaatacag aagccattgg taatgcaata 3540
   atttatttga tcatgtgact acgatccggg tgagactatt caaaaaagga gtcaagcatt 3600
   gaaataatta atgactaatc cgaagttaat tgttaggagt caattgtttt ttccaatgaa 3660
   tggaatctga gatgactaaa ctaccaattt tcaatagttc atggtatagt gacgtagtta 3720
   gtgctttttt ttcttggatc tgttgactca cttcaattga tgtttcttac cctgacatga 3780
   catacttgat attttatctc tcacgttata taacttgaaa aggatgcaca cagttctgtt 3840
   caatataccc tccaatatgt aaaaacagtt tttccattga ttactcttaa tttgtttcct 3900
   gctaaaccag cagtacgtgt gtgccgtata tattaaaatt acactatggt ttttgatttg 3960
   aaaagaattg ttagaccaaa aatttataac ttggaacctt atcgctgtgc aagagatgat 4020
   ttcaccgagg gtatattgct agacgccaat gaaaatgccc atggacctac tccagttgaa 4080
   ttgagcaaga ccaatttaca tcgttacccg gatcctcacc aattggagtt caagaccgca 4140
   atgacgaaat acaggaacaa aacaagcagt tatgccaatg acccagaggt aaaaccttta 4200
   actgctgaca atctgtgcct aggtgtggga tctgatgaga gtattgatgc tattattaga 4260
   gcatgctgtg ttcccgggaa agaaaagatt ctggttcttc caccaacata ttctatgtac 4320
   tctgtttgtg caaacattaa tgatatagaa gtcgtccaat gtcctttaac tgtttccgac 4380
   ggttcttttc aaatggatac cgaagctgta ttaaccattt tgaaaaacga ctcgctaatt 4440
   aagttgatgt tcgttacttc accaggtaat ccaaccggag ccaaaattaa gaccagttta 4500
   atcgaaaagg tcttacagaa ttgggacaat gggttagtcg ttgttgatga agcttacgta 4560
   gatttttgtg gtggctctac agctccacta gtcaccaagt atcctaactt ggttactttg 4620
   caaactctat ccaagtcatt cggtttagcc gggattaggt tgggtatgac atatgcaaca 4680
   gcagagttgg ccagaatttt aaatgcaatg aaggcgcctt ataatatttc ctccctagcc 4740
   tctgaatatg cactaaaagc tgttcaagac agtaatctaa agaagatgga agccacttcg 4800
   aaaataatca atgaagagaa aatgcgcctc ttaaaggaat taactgcttt ggattacgtt 4860
   gatgaccaat atgttggtgg attagatgct aattttcttt taatacggat caacgggggt 4920
   gacaatgtct tggcaaagaa gttatattac caattggcta ctcaatctgg ggttgtcgtc 4980
   agatttagag gtaacgaatt aggctgttcc ggatgtttga gaattaccgt tggaacccat 5040
   gaggagaaca cacatttgat aaagtacttc aaggagacgt tatataagct ggccaatgaa 5100
   taaatagacg tcaacaaaat tcagaagaac tcgtcaagaa ggcgatagaa ggcgatgcgc 5160
   tgcgaatcgg gagcggcgat accgtaaagc acgaggaagc ggtcagccca ttcgccgcca 5220
   agctcttcag caatatcacg ggtagccaac gctatgtcct gatagcggtc cgccacaccc 5280
   agccggccac agtcgatgaa tccagaaaag cggccatttt ccaccatgat attcggcaag 5340
   caggcatcgc catgggtcac gacgagatcc tcgccgtcgg gcatgctcgc cttgagcctg 5400
   gcgaacagtt cggctggcgc gagcccctga tgctcttcgt ccagatcatc ctgatcgaca 5460
   agaccggctt ccatccgagt acgtgctcgc tcgatgcgat gtttcgcttg gtggtcgaat 5520
   gggcaggtag ccggatcaag cgtatgcagc cgccgcattg catcagccat gatggatact 5580
   ttctcggcag gagcaaggtg agatgacagg agatcctgcc ccggcacttc gcccaatagc 5640
   agccagtccc ttcccgcttc agtgacaacg tcgagcacag ctgcgcaagg aacgcccgtc 5700
   gtggccagcc acgatagccg cgctgcctcg tcttgcagtt cattcagggc accggacagg 5760
   tcggtcttga caaaaagaac cgggcgcccc tgcgctgaca gccggaacac ggcggcatca 5820
   gagcagccga ttgtctgttg tgcccagtca tagccgaata gcctctccac ccaagcggcc 5880
   ggagaacctg cgtgcaatcc atcttgttca atcatgcgaa acgatcctca tcctgtctct 5940
   tgatcagatc ttgatcccct gcgccatcag atccttggcg gcgagaaagc catccagttt 6000
   actttgcagg gcttcccaac cttaccagag ggcgccccag ctggcaattc cggttcgctt 6060
   gctgtccata aaaccgccca gtctagctat cgccatgtaa gcccactgca agctacctgc 6120
   tttctctttg cgcttgcgtt ttcccttgtc cagatagccc agtagctgac attcatccgg 6180
   ggtcagcacc gtttctgcgg actggctttc tacgtgaaaa ggatctaggt gaagatcctt 6240
   tttgataatc tcatgaccaa aatcccttaa cgtgagtttt cgtgactccc cgtcaggcaa 6300
   ctatggatga acgaaataga cagatcgctg agataggtgc ctcactgatt aagcattggt 6360
   aactgtcaga ccaagtttac tcatatatac tttagattga tttaaaactt catttttaat 6420
   ttaaaaggat ctaggtgaag atcctttttg ataatctcat gaccaaaatc ccttaacgtg 6480
   agttttcgtt ccactgagcg tcagaccccg tagaaaagat caaaggatct tcttgagatc 6540
   ctttttttct gcgcgtaatc tgctgcttgc aaacaaaaaa accaccgcta ccagcggtgg 6600
   tttgtttgcc ggatcaagag ctaccaactc tttttccgaa ggtaactggc ttcagcagag 6660
   cgcagatacc aaatactgtc cttctagtgt agccgtagtt aggccaccac ttcaagaact 6720
   ctgtagcacc gcctacatac ctcgctctgc taatcctgtt accagtggct gctgccagtg 6780
   gcgataagtc gtgtcttacc gggttggact caagacgata gttaccggat aaggcgcagc 6840
   ggtcgggctg aacggggggt tcgtgcacac agcccagctt ggagcgaacg acctacaccg 6900
   aactgagata cctacagcgt gagctatgag aaagcgccac gcttcccgaa gggagaaagg 6960
   cggacaggta tccggtaagc ggcagggtcg gaacaggaga gcgcacgagg gagcttccag 7020
   ggggaaacgc ctggtatctt tatagtcctg tcgggtttcg ccacctctga cttgagcgtc 7080
   gatttttgtg atgctcgtca ggggggcgga gcctatggaa aaacgccagc aacgcggcct 7140
   ttttacggtt cctggccttt tgctggcctt ttgctcacat gttctttcct gcgttatccc 7200
   ctgattctgt ggataaccgt attaccgcct ttgagtgagc tgataccgct cgccgcagcc 7260
   gaacgaccga gcgcagcgag tcagtgagcg aggaagcgga agagcgccca atacgcaaac 7320
   cgcctctccc cgcgcgttgg ccgattcatt aatgcagctg gcacgacagg tttcccgact 7380
   ggaaagcggg cagtgagcgc aacgcaatta atgtgagtta gctcactcat taggcacccc 7440
   aggctttaca ctttatgctt ccggctcgta tgttgtgtgg aattgtgagc ggataacaat 7500
   ttcacacagg aaacagctat gacatgatta cgaattaatt cgagctcggt a 7551
<210> 61
   <211> 7308
   <212> DNA
   <213> pDD vector
<400> 61
   cttgaatttt caaaaattct tacttttttt ttggatggac gcaaagaagt ttaataatca    60
   tattacatgg cattaccacc atatacatat ccatatacat atccatatct aatcttactt   120
   atatgttgtg gaaatgtaaa gagccccatt atcttagcct aaaaaaacct tctctttgga   180
   actttcagta atacgcttaa ctgctcattg ctatattgaa gtacggatta gaagccgccg   240
   agcgggtgac agccctccga aggaagactc tcctccgtgc gtcctcgtct tcaccggtcg   300
   cgttcctgaa acgcagatgt gcctcgcgcc gcactgctcc gaacaataaa gattctacaa   360
   tactagcttt tatggttatg aagaggaaaa attggcagta acctggcccc acaaaccttc   420
   aaatgaacga atcaaattaa caaccatagg atgataatgc gattagtttt ttagccttat   480
   ttctggggta attaatcagc gaagcgatga tttttgatct attaacagat atataaatgc   540
   aaaaactgca taaccacttt aactaatact ttcaacattt tcggtttgta ttacttctta   600
   ttcaaatgta ataaaagtat caacaaaaaa ttgttaatat acctctatac tttaacgtca   660
   aggagaaaaa accccggatc aagggtgcga tatgaaagcg ttaacggcca ggcaacaaga   720
   ggtgtttgat ctcatccgtg atcacatcag ccagacaggt atgccgccga cgcgtgcgga   780
   aatcgcgcag cgtttggggt tccgttcccc aaacgcggct gaagaacatc tgaaggcgct   840
   ggcacgcaaa ggcgttattg aaattgtttc cggcgcatca cgcgggattc gtctgttgca   900
   ggaagaggaa gaagggttgc cgctggtagg tcgtgtggct gccggtgaac cacttctggc   960
   gcaacagcat attgaaggtc attatcaggt cgatccttcc ttattcaagc cgaatgctga  1020
   tttcctgctg cgcgtcagcg ggatgtcgat gaaagatatc ggcattatgg atggtgactt  1080
   gctggcagtg cataaaactc aggatgtacg taacggtcag gtcgttgtcg cacgtattga  1140
   tgacgaagtt accgttaagc gcctgaaaaa acagggcaat aaagtcgaac tgttgccaga  1200
   aaatagcgag tttaaaccaa ttgtcgtaga tcttcgtcag cagagcttca ccattgaagg  1260
   gctggcggtt ggggttattc gcaacggcga ctggctggaa ttcccgggga tccgtcgacc 1320
   atggcggccg ctcgagtcga cctgcagcca agctaattcc gggcgaattt cttatgattt 1380
   atgattttta ttattaaata agttataaaa aaaataagtg tatacaaatt ttaaagtgac 1440
   tcttaggttt taaaacgaaa attcttgttc ttgagtaact ctttcctgta ggtcaggttg 1500
   ctttctcagg tatagcatga ggtcgctctt attgaccaca cctctaccgg catgccgagc 1560
   aaatgcctgc aaatcgctcc ccatttcacc caattgtaga tatgctaact ccagcaatga 1620
   gttgatgaat ctcggtgtgt attttatgtc ctcagaggac aacacctgtt gtaatccgtc 1680
   cgagctccaa ttcgccctat agtgagtcgt attacaattc actggccgtc gttttacaac 1740
   gtcgtgactg ggaaaaccct ggcgttaccc aacttaatcg ccttgcagca catccccctt 1800
   tcgccagctg gcgtaatagc gaagaggccc gcaccgatcg cccttcccaa cagttgcgca 1860
   gcctgaatgg cgaatggcgc gacgcgccct gtagcggcgc attaagcgcg gcgggtgtgg 1920
   tggttacgcg cagcgtgacc gctacacttg ccagcgccct agcgcccgct cctttcgctt 1980
   tcttcccttc ctttctcgcc acgttcgccg gctttccccg tcaagctcta aatcgggggc 2040
   tccctttagg gttccgattt agtgctttac ggcacctcga ccccaaaaaa cttgattagg 2100
   gtgatggttc acgtagtggg ccatcgccct gatagacggt ttttcgccct ttgacgttgg 2160
   agtccacgtt ctttaatagt ggactcttgt tccaaactgg aacaacactc aaccctatct 2220
   cggtctattc ttttgattta taagggattt tgccgatttc ggcctattgg ttaaaaaatg 2280
   agctgattta acaaaaattt aacgcgaatt ttaacaaaat attaacgttt acaatttcct 2340
   gatgcggtat tttctcctta cgcatctgtg cggtatttca caccgcatat gatccgtcga 2400
   gttcaagaga aaaaaaaaga aaaagcaaaa agaaaaaagg aaagcgcgcc tcgttcagaa 2460
   tgacacgtat agaatgatgc attaccttgt catcttcagt atcatactgt tcgtatacat 2520
   acttactgac attcataggt atacatatat acacatgtat atatatcgta tgctgcagct 2580
   ttaaataatc ggtgtcacta cataagaaca cctttggtgg agggaacatc gttggtacca 2640
   ttgggcgagg tggcttctct tatggcaacc gcaagagcct tgaacgcact ctcactacgg 2700
   tgatgatcat tcttgcctcg cagacaatca acgtggaggg taattctgct agcctctgca 2760
   aagctttcaa gaaaatgcgg gatcatctcg caagagagat ctcctacttt ctccctttgc 2820
   aaaccaagtt cgacaactgc gtacggcctg ttcgaaagat ctaccaccgc tctggaaagt 2880
   gcctcatcca aaggcgcaaa tcctgatcca aaccttttta ctccacgcgc cagtagggcc 2940
   tctttaaaag cttgaccgag agcaatcccg cagtcttcag tggtgtgatg gtcgtctatg 3000
   tgtaagtcac caatgcactc aacgattagc gaccagccgg aatgcttggc cagagcatgt 3060
   atcatatggt ccagaaaccc tatacctgtg tggacgttaa tcacttgcga ttgtgtggcc 3120
   tgttctgcta ctgcttctgc ctctttttct gggaagatcg agtgctctat cgctagggga 3180
   ccacccttta aagagatcgc aatctgaatc ttggtttcat ttgtaatacg ctttactagg 3240
   gctttctgct ctgtcatctt tgccttcgtt tatcttgcct gctcattttt tagtatattc 3300
   ttcgaagaaa tcacattact ttatataatg tataattcat tatgtgataa tgccaatcgc 3360
   taagaaaaaa aaagagtcat ccgctaggtg gaaaaaaaaa aatgaaaatc attaccgagg 3420
   cataaaaaaa tatagagtgt actagaggag gccaagagta atagaaaaag aaaattgcgg 3480
   gaaaggactg tgttatgact tccctgacta atgccgtgtt caaacgatac ctggcagtga 3540
   ctcctagcgc tcaccaagct cttaaaacgg aattatggtg cactctcagt acaatctgct 3600
   ctgatgccgc atagttaagc cagccccgac acccgccaac acccgctgac gcgccctgac 3660
   gggcttgtct gctcccggca tccgcttaca gacaagctgt gaccgtctcc gggagctgca 3720
   tgtgtcagag gttttcaccg tcatcaccga aacgcgcgag acgaaagggc ctcgtgatac 3780
   gcctattttt ataggttaat gtcatgataa taatggtttc ttaggacgga tcgcttgcct 3840
   gtaacttaca cgcgcctcgt atcttttaat gatggaataa tttgggaatt tactctgtgt 3900
   ttatttattt ttatgttttg tatttggatt ttagaaagta aataaagaag gtagaagagt 3960
   tacggaatga agaaaaaaaa ataaacaaag gtttaaaaaa tttcaacaaa aagcgtactt 4020
   tacatatata tttattagac aagaaaagca gattaaatag atatacattc gattaacgat 4080
   aagtaaaatg taaaatcaca ggattttcgt gtgtggtctt ctacacagac aagatgaaac 4140
   aattcggcat taatacctga gagcaggaag agcaagataa aaggtagtat ttgttggcga 4200
   tccccctaga gtcttttaca tcttcggaaa acaaaaacta ttttttcttt aatttctttt 4260
   tttactttct atttttaatt tatatattta tattaaaaaa tttaaattat aattattttt 4320
   atagcacgtg atgaaaagga cccaggtggc acttttcggg gaaatgtgcg cggaacccct 4380
   atttgtttat ttttctaaat acattcaaat atgtatccgc tcatgagaca ataaccctga 4440
   taaatgcttc aataaattgg tcacccggcc agcgacatgg aggcccagaa taccctcctt 4500
   gacagtcttg acgtgcgcag ctcaggggca tgatgtgact gtcgcccgta catttagccc 4560
   atacatcccc atgtataatc atttgcatcc atacattttg atggccgcac ggcgcgaagc 4620
   aaaaattacg gctcctcgct gcagacctgc gagcagggaa acgctcccct cacagacgcg 4680
   ttgaattgtc cccacgccgc gcccctgtag agaaatataa aaggttagga tttgccactg 4740
   aggttcttct ttcatatact tccttttaaa atcttgctag gatacagttc tcacatcaca 4800
   tccgaacata aacaaccatg ggtaaggaaa agactcacgt ttcgaggccg cgattaaatt 4860
   ccaacatgga tgctgattta tatgggtata aatgggctcg cgataatgtc gggcaatcag 4920
   gtgcgacaat ctatcgattg tatgggaagc ccgatgcgcc agagttgttt ctgaaacatg 4980
   gcaaaggtag cgttgccaat gatgttacag atgagatggt cagactaaac tggctgacgg 5040
   aatttatgcc tcttccgacc atcaagcatt ttatccgtac tcctgatgat gcatggttac 5100
   tcaccactgc gatccccggc aaaacagcat tccaggtatt agaagaatat cctgattcag 5160
   gtgaaaatat tgttgatgcg ctggcagtgt tcctgcgccg gttgcattcg attcctgttt 5220
   gtaattgtcc ttttaacagc gatcgcgtat ttcgtctcgc tcaggcgcaa tcacgaatga 5280
   ataacggttt ggttgatgcg agtgattttg atgacgagcg taatggctgg cctgttgaac 5340
   aagtctggaa agaaatgcat aagcttttgc cattctcacc ggattcagtc gtcactcatg 5400
   gtgatttctc acttgataac cttatttttg acgaggggaa attaataggt tgtattgatg 5460
   ttggacgagt cggaatcgca gaccgatacc aggatcttgc catcctatgg aactgcctcg 5520
   gtgagttttc tccttcatta cagaaacggc tttttcaaaa atatggtatt gataatcctg 5580
   atatgaataa attgcagttt catttgatgc tcgatgagtt tttctaatca gtcctcggag 5640
   atccgtcccc cttttccttt gtcgatatca tgtaattagt tatgtcacgc ttacattcac 5700
   gccctccccc cacatccgct ctaaccgaaa aggaaggagt tagacaacct gaagtctagg 5760
   tccctattta tttttttata gttatgttag tattaagaac gttatttata tttcaaattt 5820
   ttcttttttt tctgtacaga cgcgtgtacg catgtaacat tatactgaaa accttgcttg 5880
   agaaggtttt gggacgctcg aaggctttaa tttgcaagct ggggtctcgc ggtcggtatc 5940
   attgcagcac tggggccaga tggtaagccc tcccgtatcg tagttatcta cacgacgggc 6000
   agtcaggcaa ctatggatga acgaaataga cagatcgctg agataggtgc ctcactgatt 6060
   aagcattggt aactgtcaga ccaagtttac tcatatatac tttagattga tttaaaactt 6120
   catttttaat ttaaaaggat ctaggtgaag atcctttttg ataatctcat gaccaaaatc 6180
   ccttaacgtg agttttcgtt ccactgagcg tcagaccccg tagaaaagat caaaggatct 6240
   tcttgagatc ctttttttct gcgcgtaatc tgctgcttgc aaacaaaaaa accaccgcta 6300
   ccagcggtgg tttgtttgcc ggatcaagag ctaccaactc tttttccgaa ggtaactggc 6360
   ttcagcagag cgcagatacc aaatactgtc cttctagtgt agccgtagtt aggccaccac 6420
   ttcaagaact ctgtagcacc gcctacatac ctcgctctgc taatcctgtt accagtggct 6480
   gctgccagtg gcgataagtc gtgtcttacc gggttggact caagacgata gttaccggat 6540
   aaggcgcagc ggtcgggctg aacggggggt tcgtgcacac agcccagctt ggagcgaacg 6600
   acctacaccg aactgagata cctacagcgt gagcattgag aaagcgccac gcttcccgaa 6660
   gggagaaagg cggacaggta tccggtaagc ggcagggtcg gaacaggaga gcgcacgagg 6720
   gagcttccag ggggaaacgc ctggtatctt tatagtcctg tcgggtttcg ccacctctga 6780
   cttgagcgtc gatttttgtg atgctcgtca ggggggcgga gcctatggaa aaacgccagc 6840
   aacgcggcct ttttacggtt cctggccttt tgctggcctt ttgctcacat gttctttcct 6900
   gcgttatccc ctgattctgt ggataaccgt attaccgcct ttgagtgagc tgataccgct 6960
   cgccgcagcc gaacgaccga gcgcagcgag tcagtgagcg aggaagcgga agagcgccca 7020
   atacgcaaac cgcctctccc cgcgcgttgg ccgattcatt aatgcagctg gcacgacagg   7080
   tttcccgact ggaaagcggg cagtgagcgc aacgcaatta atgtgagtta gctcactcat   7140
   taggcacccc aggctttaca ctttatgctt ccggctcgta tgttgtgtgg aattgtgagc   7200
   ggataacaat ttcacacagg aaacagctat gaccatgatt accccaagct cgaaattaac   7260
   cctcactaaa gggaacaaaa gctggtaccg ggccccccct cgaaattc                 7308
<210> 62
   <211> 289
   <212> DNA
   <213> clone BGF05 with first open reading frame
<220>
   <221> CDS
   <222> (1)..(165)
   <223>
<400> 62
   atg ggt aag cct atc cct aac cct ctc ctc ggt ctc gat tct aca caa       48
   Met Gly Lys Pro Ile Pro Asn Pro Leu Leu Gly Leu Asp Ser Thr Gln
   1               5                   10                  15
   gct atg ggt gct cct cca aaa aag aag aga aag gta gct gaa ttc gca       96
   Ala Met Gly Ala Pro Pro Lys Lys Lys Arg Lys Val Ala Glu Phe Ala
               20                  25                  30
   agg acg acg acg aca agg ctt atc aat caa tca gtg gtt aga aaa ata      144
   Arg Thr Thr Thr Thr Arg Leu Ile Asn Gln Ser Val Val Arg Lys Ile
           35                  40                  45
   tgg cta tat cga gat gca agg taacgaaacg ttcaaattgg cagttcgtga         195
   Trp Leu Tyr Arg Asp Ala Arg
       50                  55
   actttcaaat gtggtggaag aaacactttt agccaataat ttagataaaa aagatttaga    255
   ctggcttgtc gtcgtcgtcc ttgtagtctg acct                                289
<210> 63
   <211> 55
   <212> PRT
   <213> clone BGF05 with first open reading frame
<400> 63
   Met Gly Lys Pro Ile Pro Asn Pro Leu Leu Gly Leu Asp Ser Thr Gln
   1               5                   10                  15
   Ala Met Gly Ala Pro Pro Lys Lys Lys Arg Lys Val Ala Glu Phe Ala
               20                  25                  30
   Arg Thr Thr Thr Thr Arg Leu Ile Asn Gln Ser Val Val Arg Lys Ile
           35                  40                  45
   Trp Leu Tyr Arg Asp Ala Arg
       50                  55
<210> 64
   <211> 196
   <212> DNA
   <213> clone BGF05 H. influenzae insertion
<400> 64
   gcaaggacga cgacgacaag gcttatcaat caatcagtgg ttagaaaaat atggctatat 60
   cgagatgcaa ggtaacgaaa cgttcaaatt ggcagttcgt gaactttcaa atgtggtgga 120
   agaaacactt ttagccaata atttagataa aaaagattta gactggcttg tcgtcgtcgt 180
   ccttgtagtc tgacct 196
<210> 65
   <211> 24
   <212> PRT
   <213> SCL-E47 antagonist encoded by BGF05 A. aeolicus insertion
<400> 65
   Ala Arg Thr Thr Thr Thr Arg Leu Ile Asn Gln Ser Val Val Arg Lys
   1               5                   10                  15
   Ile Trp Leu Tyr Arg Asp Ala Arg
               20
<210> 66
   <211> 419
   <212> DNA
   <213> clone BGF06 with first open reading frame
<220>
   <221> CDS
   <222> (1)..(165)
   <223>
<220>
   <221> misc feature
   <222> (307)..(307)
   <223> N at position 307 is any nucleotide residue
<220>
   <221> misc feature
   <222> (357)..(357)
   <223> N at position 357 is any nucleotide residue
<220>
   <221> misc feature
   <222> (355)..(355)
   <223> N at position 355 is any nucleotide residue
<400> 66
   atg ggt aag cct atc cct aac cct ctc ctc ggt ctc gat tct aca caa        48
   Met Gly Lys Pro Ile Pro Asn Pro Leu Leu Gly Leu Asp Ser Thr Gln
   1               5                   10                  15
   gct atg ggt gct cct cca aaa aag aag aga aag gta gct gaa ttc agg        96
   Ala Met Gly Ala Pro Pro Lys Lys Lys Arg Lys Val Ala Glu Phe Arg
               20                  25                  30
   tca gac tac aag gac gac gac gac aag gct tat cac ata att gct gtt       144
   Ser Asp Tyr Lys Asp Asp Asp Asp Lys Ala Tyr His Ile Ile Ala Val
           35                  40                  45
   tgt ttt tta atc aag gta tca tgacatgtcc caacctcgcc cactgctctc          195
   Cys Phe Leu Ile Lys Val Ser
       50                  55
   tcctcccgaa actgaagaac agttgttagc gcaagcacag caactttctg gttatacatt    255
   gggagaactg gcggcacttg tcgggctggt tacgccagag aatttaaaac gngataaagg    315
   ctggattggc gtgttactgg agatctggct aggtgccagn gnagggagta aacctgagca    375
   agattttgct gttgaataca cctgattgat tgataacctt gtcg                      419
<210> 67
   <211> 55
   <212> PRT
   <213> clone BGF06 with first open reading frame
<400> 67
   Met Gly Lys Pro Ile Pro Asn Pro Leu Leu Gly Leu Asp Ser Thr Gln
   1               5                   10                  15
   Ala Met Gly Ala Pro Pro Lys Lys Lys Arg Lys Val Ala Glu Phe Arg
               20                  25                  30
   Ser Asp Tyr Lys Asp Asp Asp Asp Lys Ala Tyr His Ile Ile Ala Val
           35                  40                  45
   Cys Phe Leu Ile Lys Val Ser
       50                  55
<210> 68
   <211> 326
   <212> DNA
   <213> clone BGF06 A. aeolicus insertion
<220>
   <221> misc_feature
   <222> (214)..(214)
   <223> N at position 214 is any nucleotide residue
<220>
   <221> misc feature
   <222> (262)..(262)
   <223> N at position 262 is any nucleotide residue
<220>
   <221> misc_feature
   <222> (264)..(264)
   <223> N at position 264 is any nucleotide residue
<400> 68
   aggtcagact acaaggacga cgacgacaag gcttatcaca taattgctgt ttgtttttta     60
   atcaaggtat catgacatgt cccaacctcg cccactgctc tctcctcccg aaactgaaga    120
   acagttgtta gcgcaagcac agcaactttc tggttataca ttgggagaac tggcggcact    180
   tgtcgggctg gttacgccag agaatttaaa acgngataaa ggctggattg gcgtgttact    240
   ggagatctgg ctaggtgcca gngnagggag taaacctgag caagattttg ctgttgaata    300
   cacctgattg attgataacc ttgtcg                                         326
<210> 69
   <211> 24
   <212> PRT
   <213> SCL-E47 antagonist encoded by clone BGF06 H. influenzae insertion
<400> 69
   Arg Ser Asp Tyr Lys Asp Asp Asp Asp Lys Ala Tyr His Ile Ile Ala
   1               5                   10                  15
   Val Cys Phe Leu Ile Lys Val Ser
               20
<210> 70
   <211> 220
   <212> DNA
   <213> clone BGF13 with first open reading frame
<220>
   <221> CDS
   <222> (1)..(123)
   <223>
<400> 70
   atg ggt aag cct atc cct aac cct ctc ctc ggt ctc gat tct aca caa      48
   Met Gly Lys Pro Ile Pro Asn Pro Leu Leu Gly Leu Asp Ser Thr Gln
   1               5                   10                  15
   gct atg ggt gct cct cca aaa aag aag aga aag gta gct gaa ttc gga      96
   Ala Met Gly Ala Pro Pro Lys Lys Lys Arg Lys Val Ala Glu Phe Gly
               20                  25                  30
   ttt tgc tct aat caa atg aga gaa atg taaaatgaag ccacgaacat           143
   Phe Cys Ser Asn Gln Met Arg Glu Met
           35                  40
   attaataata gcctaccact gcaaccatta gttcctgatc aggagaacaa aaataagaga   203
   aatgaagaga aatccgt                                                  220
<210> 71
   <211> 41
   <212> PRT
   <213> clone BGF13 with first open reading frame
<400> 71
   Met Gly Lys Pro Ile Pro Asn Pro Leu Leu Gly Leu Asp Ser Thr Gln
   1               5                   10                  15
   Ala Met Gly Ala Pro Pro Lys Lys Lys Arg Lys Val Ala Glu Phe Gly
               20                  25                  30
   Phe Cys Ser Asn Gln Met Arg Glu Met
           35                  40
<210> 72
   <211> 127
   <212> DNA
   <213> clone BGF13 H. influenzae insertion
<400> 72
   ggattttgct ctaatcaaat gagagaaatg taaaatgaag ccacgaacat attaataata      60
   gcctaccact gcaaccatta gttcctgatc aggagaacaa aaataagaga aatgaagaga     120
   aatccgt                                                               127
<210> 73
   <211> 10
   <212> PRT
   <213> SCL-E47 antagonist encoded by clone BGF13 T. maritima insertion
<400> 73
   Gly Phe Cys Ser Asn Gln Met Arg Glu Met
   1               5                   10
<210> 74
   <211> 273
   <212> DNA
   <213> clone BGF24 with first open reading frame
<220>
   <221> CDS
   <222> (1)..(171)
   <223>
<400> 74
   atg ggt aag cct atc cct aac cct ctc ctc ggt ctc gat tct aca caa       48
   Met Gly Lys Pro Ile Pro Asn Pro Leu Leu Gly Leu Asp Ser Thr Gln
   1               5                   10                  15
   gct atg ggt gct cct cca aaa aag aag aga aag gta gct gaa ttc agg       96
   Ala Met Gly Ala Pro Pro Lys Lys Lys Arg Lys Val Ala Glu Phe Arg
               20                  25                  30
   tca gac tac aag gac gac gac gac aag ctt atc aaa cat cac tgg gtg      144
   Ser Asp Tyr Lys Asp Asp Asp Asp Lys Leu Ile Lys His His Trp Val
           35                  40                  45
   ctt tca atc atc acc acc gcg tct cgc taacctgttt caaataccac            191
   Leu Ser Ile Ile Thr Thr Ala Ser Arg
       50                  55
   catgaactcc tctccaccgt atcttgacta caagggatga ttgattgata agccttgtcg     251
   tcgtcgtcct tgtagtctga ct                                              273
<210> 75
   <211> 57
   <212> PRT
   <213> clone BGF24 with first open reading frame
<400> 75
   Met Gly Lys Pro Ile Pro Asn Pro Leu Leu Gly Leu Asp Ser Thr Gln
   1               5                   10                  15
   Ala Met Gly Ala Pro Pro Lys Lys Lys Arg Lys Val Ala Glu Phe Arg
               20                  25                  30
   Ser Asp Tyr Lys Asp Asp Asp Asp Lys Leu Ile Lys His His Trp Val
           35                  40                  45
   Leu Ser Ile Ile Thr Thr Ala Ser Arg
       50                  55
<210> 76
   <211> 180
   <212> DNA
   <213> clone BGF24 T. maritima insertion
<400> 76
   aggtcagact acaaggacga cgacgacaag cttatcaaac atcactgggt gctttcaatc     60
   atcaccaccg cgtctcgcta acctgtttca aataccacca tgaactcctc tccaccgtat    120
   cttgactaca agggatgatt gattgataag ccttgtcgtc gtcgtccttg tagtctgact    180
<210> 77
   <211> 26
   <212> PRT
   <213> SCL-E47 antagonist encoded by clone BGF24 T. maritima insertion
<400> 77
   Arg Ser Asp Tyr Lys Asp Asp Asp Asp Lys Leu Ile Lys His His Trp
   1               5                   10                  15
   Val Leu Ser Ile Ile Thr Thr Ala Ser Arg
               20                  25
<210> 78
   <211> 288
   <212> DNA
   <213> clone BGF35 with first open reading frame
<220>
   <221> CDS
   <222> (1)..(282)
   <223>
<400> 78
   atg ggt aag cct atc cct aac cct ctc ctc ggt ctc gat tct aca caa       48
   Met Gly Lys Pro Ile Pro Asn Pro Leu Leu Gly Leu Asp Ser Thr Gln
   1               5                   10                  15
   gct atg ggt gct cct cca aaa aag aag aga aag gta gct gaa ttc caa       96
   Ala Met Gly Ala Pro Pro Lys Lys Lys Arg Lys Val Ala Glu Phe Gln
               20                  25                  30
   gga cga cga cga caa ggc tta tca atc aat cag tgg tta gaa aaa tat      144
   Gly Arg Arg Arg Gln Gly Leu Ser Ile Asn Gln Trp Leu Glu Lys Tyr
           35                  40                  45
   ggc tat atc gag atg caa ggt aac gaa acg ttc aaa ttg gca gtt cgt      192
   Gly Tyr Ile Glu Met Gln Gly Asn Glu Thr Phe Lys Leu Ala Val Arg
       50                  55                  60
   gaa ctt tca aat gtg gtg gaa gaa aca ctt tta gcc aat aat tta gat      240
   Glu Leu Ser Asn Val Val Glu Glu Thr Leu Leu Ala Asn Asn Leu Asp
   65                  70                  75                  80
   aaa aaa gat tta gac tgg ctt gtc gtc gtc gtc ctt gta gtc tgacct       288
   Lys Lys Asp Leu Asp Trp Leu Val Val Val Val Leu Val Val
                   85                  90
<210> 79
   <211> 94
   <212> PRT
   <213> clone BGF35 with first open reading frame
<400> 79
   Met Gly Lys Pro Ile Pro Asn Pro Leu Leu Gly Leu Asp Ser Thr Gln
   1               5                   10                  15
   Ala Met Gly Ala Pro Pro Lys Lys Lys Arg Lys Val Ala Glu Phe Gln
               20                  25                  30
   Gly Arg Arg Arg Gln Gly Leu Ser Ile Asn Gln Trp Leu Glu Lys Tyr
           35                  40                  45
   Gly Tyr Ile Glu Met Gln Gly Asn Glu Thr Phe Lys Leu Ala Val Arg
       50                  55                  60
   Glu Leu Ser Asn Val Val Glu Glu Thr Leu Leu Ala Asn Asn Leu Asp
   65                  70                  75                  80
   Lys Lys Asp Leu Asp Trp Leu Val Val Val Val Leu Val Val
                   85                  90
<210> 80
   <211> 195
   <212> DNA
   <213> clone BGF35 H. influenzae insertion
<400> 80
   caaggacgac gacgacaagg cttatcaatc aatcagtggt tagaaaaata tggctatatc     60
   gagatgcaag gtaacgaaac gttcaaattg gcagttcgtg aactttcaaa tgtggtggaa    120
   gaaacacttt tagccaataa tttagataaa aaagatttag actggcttgt cgtcgtcgtc    180
   cttgtagtct gacct                                                     195
<210> 81
   <211> 63
   <212> PRT
   <213> SCL-E47 antagonist encoded by clone BGF35 H. influenzae insertion
<400> 81
   Gln Gly Arg Arg Arg Gln Gly Leu Ser Ile Asn Gln Trp Leu Glu Lys
   1               5                   10                  15
   Tyr Gly Tyr Ile Glu Met Gln Gly Asn Glu Thr Phe Lys Leu Ala Val
               20                  25                  30
   Arg Glu Leu Ser Asn Val Val Glu Glu Thr Leu Leu Ala Asn Asn Leu
           35                  40                  45
   Asp Lys Lys Asp Leu Asp Trp Leu Val Val Val Val Leu Val Val
       50                  55                  60
<210> 82
   <211> 10
   <212> PRT
   <213> 2c7pan8 mimotope
<400> 82
   Gly Asp Pro Asn Ser Ser Thr Ser Pro Arg
   1               5                   10
<210> 83
   <211> 6
   <212> PRT
   <213> 2c7pan8 mimotope without phage sequence
<400> 83
   Ser Ser Thr Ser Pro Arg
   1 5
<210> 84
   <211> 10
   <212> PRT
   <213> 2c7pan9 mimotope
<400> 84
   Gly Asp Pro Asn Ser Ala Ser Gly Thr Ala
   1 5 10
<210> 85
   <211> 6
   <212> PRT
   <213> 2c7pan9 mimotope without phage sequence
<400> 85
   Ser Ala Ser Gly Thr Ala
   1 5
<210> 86
   <211> 14
   <212> PRT
   <213> 2c7pan14 mimotope
<400> 86
   Gly Asp Pro Asn Ser Arg Gly Lys Ser Arg Glu Tyr Leu Ser
   1 5 10
<210> 87
   <211> 10
   <212> PRT
   <213> 2c7pan14 mimotope without phage sequence
<400> 87
   Ser Arg Gly Lys Ser Arg Glu Tyr Leu Ser
   1 5 10
<210> 88
   <211> 7
   <212> PRT
   <213> 2c7pan26 mimotope
<400> 88
   Gly Asp Pro Arg Thr His Arg
   1               5
<210> 89
   <211> 4
   <212> PRT
   <213> 2c7pan26 mimotope without phage sequence
<400> 89
   Arg Thr His Arg
   1
<210> 90
   <211> 22
   <212> PRT
   <213> 2c7pan42 mimotope
<400> 90
   Gly Asp Pro Asn Ser Ser Ser Val Asp Lys Leu Gly Tyr Trp Arg Val
   1               5                   10                  15
   Thr Glu Ser Ser Asn Glu
               20
<210> 91
   <211> 18
   <212> PRT
   <213> 2c7pan42 mimotope without phage sequence
<400> 91 Asn Glu
<210> 92
   <211> 5562
   <212> DNA
   <213> pYTB vector
<400> 92
   ctagcgattt tggtcatgag atcagatcaa cttcttttct ttttttttct tttctctctc     60
   ccccgttgtt gtctcaccat atccgcaatg acaaaaaaat gatggaagac actaaaggaa    120
   aaaattaacg acaaagacag caccaacaga tgtcgttgtt ccagagctga tgaggggtat    180
   ctcgaagcac acgaaacttt ttccttcctt cattcacgca cactactctc taatgagcaa    240
   cggtatacgg ccttccttcc agttacttga atttgaaata aaaaaaagtt tgctgtcttg    300
   ctatcaagta taaatagacc tgcaattatt aatcttttgt ttcctcgtca ttgttctcgt    360
   tccctttctt ccttgtttct ttttctgcac aatatttcaa gctataccaa gcatacaatc    420
   aactccaagc ttccccggat cggactacta gcagctgtaa tacgactcac tatagggaat    480
   attaagctca ccatgggtaa gcctatccct aaccctctcc tcggtctcga ttctacacaa    540
   gctatgggtg ctcctccaaa aaagaagaga aaggtagctg aattcgagct cagatctcag    600
   ctgggcccgg taccaattga tgcatcgata ccggtactag tcggaccgca tatgcccggg    660
   cgtaccgcgg ccgctcgagg catgcatcta gagggccgca tcatgtaatt agttatgtca    720
   cgcttacatt cacgccctcc ccccacatcc gctctaaccg aaaaggaagg agttagacaa    780
   cctgaagtct aggtccctat ttattttttt atagttatgt tagtattaag aacgttattt    840
   atatttcaaa tttttctttt ttttctgtac agacgcgtgt acgcatgtaa cattatactg    900
   aaaaccttgc ttgagaaggt tttgggacgc tcgaaggctt taatttgcgg ccctgcatta    960
   atgaatcggc caacgcgcgg ggagaggcgg tttgcgtatt gggcgctctt ccgcttcctc 1020
   gctcactgac tcgctgcgct cggtcgttcg gctgcggcga gcggtatcag ctcactcaaa 1080
   ggcggtaata cggttatcca cagaatcagg ggataacgca ggaaagaaca tgtgagcaaa 1140
   aggccagcaa aagcccagga accgtaaaaa ggccgcgttg ctggcgtttt tccataggct 1200
   ccgcccccct gacgagcatc acaaaaatcg acgctcaagt cagaggtggc gaaacccgac 1260
   aggactataa agataccagg cgtttccccc tggaagctcc ctcgtgcgct ctcctgttcc 1320
   gaccctgccg cttaccggat acctgtccgc ctttctccct tcgggaagcg tggcgctttc 1380
   tcatagctca cgctgtaggt atctcagttc ggtgtaggtc gttcgctcca agctgggctg 1440
   tgtgcacgaa ccccccgttc agcccgaccg ctgcgcctta tccggtaact atcgtcttga 1500
   gtccaacccg gtaagacacg acttatcgcc actggcagca gccactggta acaggattag 1560
   cagagcgagg tatgtaggcg gtgctacaga gttcttgaag tggtggccta actacggcta 1620
   cactagaagg acagtatttg gtatctgcgc tctgctgaag ccagttacct tcggaaaaag 1680
   agttggtagc tcttgatccg gcaaacaaac caccgctggt agcggtggtt tttttgtttg 1740
   caagcagcag attacgcgca gaaaaaaagg atctcaagaa gatcctttga tcttttctac 1800
   ggggtctgac gctcagtgga acgaaaactc acgttaaggg attttggtca tgagattatc 1860
   aaaaaggatc ttcacctaga tccttttaaa ttaaaaatga agttttaaat caatctaaag 1920
   tatatatgag taaacttggt ctgacagtta ccaatgctta atcagtgagg cacctatctc 1980
   agcgatctgt ctatttcgtt catccatagt tgcctgactc cccgtcgtgt agataactac 2040
   gatacgggag cgcttaccat ctggccccag tgctgcaatg ataccgcgag acccacgctc 2100
   accggctcca gatttatcag caataaacca gccagccgga agggccgagc gcagaagtgg 2160
   tcctgcaact ttatccgcct ccatccagtc tattaattgt tgccgggaag ctagagtaag 2220
   tagttcgcca gttaatagtt tgcgcaacgt tgttgccatt gctacaggca tcgtggtgtc 2280
   acgctcgtcg tttggtatgg cttcattcag ctccggttcc caacgatcaa ggcgagttac 2340
   atgatccccc atgttgtgca aaaaagcggt tagctccttc ggtcctccga tcgttgtcag 2400
   aagtaagttg gccgcagtgt tatcactcat ggttatggca gcactgcata attctcttac 2460
   tgtcatgcca tccgtaagat gcttttctgt gactggtgag tactcaacca agtcattctg 2520
   agaatagtgt atgcggcgac cgagttgctc ttgcccggcg tcaacacggg ataataccgc 2580
   gccacatagc agaactttaa aagtgctcat cattggaaaa cgttcttcgg ggcgaaaact 2640
   ctcaaggatc ttaccgctgt tgagatccag ttcgatgtaa cccactcgtg cacccaactg 2700
   atcttcagca tcttttactt tcaccagcgt ttctgggtga gcaaaaacag gaaggcaaaa 2760
   tgccgcaaaa aagggaataa gggcgacacg gaaatgttga atactcatac tcttcctttt 2820
   tcaatattat tgaagcattt atcagggtta ttgtctcatg agcggataca tatttgaatg 2880
   tatttagaaa aataaacaaa taggggttcc gcgcacattt ccccgaaaag tgccacctga 2940
   cgtctaagaa accattatta tcatgacatt aacctataaa aataggcgta tcacgaggcc 3000
   ctttcgtctt caagaaattc ggtcgaaaaa agaaaaggag agggccaaga gggagggcat 3060
   tggtgactat tgagcacgtg agtatacgtg attaagcaca caaaggcagc ttggagtatg 3120
   tctgttatta atttcacagg tagttctggt ccattggtga aagtttgcgg cttgcagagc 3180
   acagaggccg cagaatgtgc tctagattcc gatgctgact tgctgggtat tatatgtgtg 3240
   cccaatagaa agagaacaat tgacccggtt attgcaagga aaatttcaag tcttgtaaaa 3300
   gcatataaaa atagttcagg cactccgaaa tacttggttg gcgtgtttcg taatcaacct 3360
   aaggaggatg ttttggctct ggtcaatgat tacggcattg atatcgtcca actgcacgga 3420
   gatgagtcgt ggcaagaata ccaagagttc ctcggtttgc cagttattaa aagactcgta 3480
   tttccaaaag actgcaacat actactcagt gcagcttcac agaaacctca ttcgtttatt 3540
   cccttgtttg attcagaagc aggtgggaca ggtgaacttt tggattggaa ctcgatttct 3600
   gactgggttg gaaggcaaga gagccccgag agcttacatt ttatgttagc tggtggactg 3660
   acgccagaaa atgttggtga tgcgcttaga ttaaatggcg ttattggtgt tgatgtaagc 3720
   ggaggtgtgg agacaaatgg tgtaaaagac tctaacaaaa tagcaaattt cgtcaaaaat 3780
   gctaagaaat aggttattac tgagtagtat ttatttaagt attgtttgtg cacttgcctg 3840
   cagcttctca atgatattcg aatacgcttt gaggagatac agcctaatat ccgacaaact 3900
   gttttacaga tttacgatcg tacttgttac ccatcattga attttgaaca tccgaacctg 3960
   ggagttttcc ctgaaacaga tagtatattt gaacctgtat aataatatat agtctagcgc 4020
   tttacggaag acaatgtatg tatttcggtt cctggagaaa ctattgcatc tattgcatag 4080
   gtaatcttgc acgtcgcatc cccggttcat tttctgcgtt tccatcttgc acttcaatag 4140
   catatctttg ttaacgaagc atctgtgctt cattttgtag aacaaaaatg caacgcgaga 4200
   gcgctaattt ttcaaacaaa gaatctgagc tgcattttta cagaacagaa atgcaacgcg 4260
   aaagcgctat tttaccaacg aagaatctgt gcttcatttt tgtaaaacaa aaatgcaacg 4320
   cgagagcgct aatttttcaa acaaagaatc tgagctgcat ttttacagaa cagaaatgca 4380
   acgcgagagc gctattttac caacaaagaa tctatacttc ttttttgttc tacaaaaatg 4440
   catcccgaga gcgctatttt tctaacaaag catcttagat tacttttttt ctcctttgtg 4500
   cgctctataa tgcagtctct tgataacttt ttgcactgta ggtccgttaa ggttagaaga 4560
   aggctacttt ggtgtctatt ttctcttcca taaaaaaagc ctgactccac ttcccgcgtt 4620
   tactgattac tagcgaagct gcgggtgcat tttttcaaga taaaggcatc cccgattata 4680
   ttctataccg atgtggattg cgcatacttt gtgaacagaa agtgatagcg ttgatgattc 4740
   ttcattggtc agaaaattat gaacggtttc ttctattttg tctctatata ctacgtatag 4800
   gaaatgttta cattttcgta ttgttttcga ttcactctat gaatagttct tactacaatt 4860
   tttttgtcta aagagtaata ctagagataa acataaaaaa tgtagaggtc gagtttagat 4920
   gcaagttcaa ggagcgaaag gtggatgggt aggttatata gggatatagc acagagatat 4980
   atagcaaaga gatacttttg agcaatgttt gtggaagcgg tattcgcaat gggaagctcc 5040
   accccggttg ataatcagaa aagccccaaa aacaggaaga ttgtataagc aaatatttaa 5100
   attgtaaacg ttaatatttt gttaaaattc gcgttaaatt tttgttaaat cagctcattt 5160
   tttaacgaat agcccgaaat cggcaaaatc ccttataaat caaaagaata gaccgagata 5220
   gggttgagtg ttgttccagt ttccaacaag agtccactat taaagaacgt ggactccaac 5280
   gtcaaagggc gaaaaagggt ctatcagggc gatggcccac tacgtgaacc atcaccctaa 5340
   tcaagttttt tggggtcgag gtgccgtaaa gcagtaaatc ggaagggtaa acggatgccc 5400
   ccatttagag cttgacgggg aaagccggcg aacgtggcga gaaaggaagg gaagaaagcg 5460
   aaaggagcgg gggctagggc ggtgggaagt gtaggggtca cgctgggcgt aaccaccaca 5520
   cccgccgcgc ttaatggggc gctacagggc gcgtggggat ga 5562
<210> 93
   <211> 248
   <212> DNA
   <213> clone 4 encoding JUN dimerization inhibitory peptide
<400> 93
   aggtcagact acaaggacga cgacgacaag gcttatcaat ccatgttctg tgaaagccgc 60
   ttcctggaca atgcatctgc ccctgccatg aggaatgcaa agaggcgttc cgaagagcgg 120
   gtcctgtgta acctgacagt tcatagaaaa cacattttgc acaagatcac aagtgatgac 180
   ctcttccgga cggccttctg cagaaatccg tttatctttt atggccacaa gatgatgcgc 240
   atgattga 248
<210> 94
   <211> 83
   <212> PRT
   <213> clone 4 JUN dimerization inhibitory peptide with phage sequence
<400> 94
   Arg Ser Asp Tyr Lys Asp Asp Asp Asp Lys Ala Tyr Gln Ser Met Phe
   1 5 10 15
   Cys Glu Ser Arg Phe Leu Asp Asn Ala Ser Ala Pro Ala Met Arg Asn
   20 25 30
   Ala Lys Arg Arg Ser Glu Glu Arg Val Leu Cys Asn Leu Thr Val His
   35 40 45
   Arg Lys His Ile Leu His Lys Ile Thr Ser Asp Asp Leu Phe Arg Thr
   50 55 60
   Ala Phe Cys Arg Asn Pro Phe Ile Phe Tyr Gly His Lys Met Met Arg
   65 70 75 80
   Met Ile Asp
<210> 95
   <211> 73
   <212> PRT
   <213> clone 4 JUN dimerization inhibitory peptide without phage sequence
<400> 95
   Ala Tyr Gln Ser Met Phe Cys Glu Ser Arg Phe Leu Asp Asn Ala Ser
   1 5 10 15
   Ala Pro Ala Met Arg Asn Ala Lys Arg Arg Ser Glu Glu Arg Val Leu
   20 25 30
   Cys Asn Leu Thr Val His Arg Lys His Ile Leu His Lys Ile Thr Ser
   35 40 45
   Asp Asp Leu Phe Arg Thr Ala Phe Cys Arg Asn Pro Phe Ile Phe Tyr
   50 55 60
   Gly His Lys Met Met Arg Met Ile Asp
   65 70
<210> 96
   <211> 38
   <212> DNA
   <213> clone 5 encoding JUN dimerization inhibitory peptide with phage sequence
<400> 96
   aggtcagact acaaggacga cgacgacaag gcttatca 38
<210> 97
   <211> 13
   <212> PRT
   <213> clone 5 JUN dimerization inhibitory peptide with phage sequence
<400> 97
   Arg Ser Asp Tyr Lys Asp Asp Asp Asp Lys Ala Tyr Gln
   1               5                   10
<210> 98
   <211> 3
   <212> PRT
   <213> clone 5 JUN dimerization inhibitory peptide without phage sequence
<400> 98
   Ala Tyr Gln
   1
<210> 99
   <211> 120
   <212> DNA
   <213> clone 8 encoding JUN dimerization inhibitory peptide with phage sequence
<400> 99
   aggtcagact acaaggacga cgacgacaag gcttatcaat caatcatagc taatgaagag 60
   gagagggaga aaaattttgc atccagcaaa aaggacggat cctataccga tctcttgtga 120
<210> 100
   <211> 39
   <212> PRT
   <213> clone 8 JUN dimerization inhibitory peptide with phage sequence
<400> 100
   Arg Ser Asp Tyr Lys Asp Asp Asp Asp Lys Ala Tyr Gln Ser Ile Ile
   1               5                   10                  15
   Ala Asn Glu Glu Glu Arg Glu Lys Asn Phe Ala Ser Ser Lys Lys Asp
               20                  25                  30
   Gly Ser Tyr Thr Asp Leu Leu
           35
<210> 101
   <211> 29
   <212> PRT
   <213> clone 8 JUN dimerization inhibitory peptide without phage sequence
<400> 101
   Ala Tyr Gln Ser Ile Ile Ala Asn Glu Glu Glu Arg Glu Lys Asn Phe
   1               5                   10                  15
   Ala Ser Ser Lys Lys Asp Gly Ser Tyr Thr Asp Leu Leu
               20                  25
<210> 102
   <211> 123
   <212> DNA
   <213> clone 12 encoding JUN dimerization inhibitory peptide with phage sequence
<220>
   <221> misc_feature
   <222> (49)..(50)
   <223> N at nucleotide position 49 or 50 is any nucleotide residue
<400> 102
   aggtcagact acaaggacga cgacgacaag gcttatcaag agtccaccnn agcgctggtg      60
   gaaggtggcg cggatctgat cctgattgaa accgttcttg tcgtcgtcgt ccttgtagtc     120
   tga                                                                   123
<210> 103
   <211> 40
   <212> PRT
   <213> clone 12 JUN dimerization inhibitory peptide with phage sequence
<220>
   <221> MISC_FEATURE
   <222> (17)..(17)
   <223> Xaa at position 17 is any amino acid residue
<400> 103
   Arg Ser Asp Tyr Lys Asp Asp Asp Asp Lys Ala Tyr Gln Glu Ser Thr
   1               5                   10                  15
   Xaa Ala Leu Val Glu Gly Gly Ala Asp Leu Ile Leu Ile Glu Thr Val
               20                  25                  30
   Leu Val Val Val Val Leu Val Val
           35                  40
<210> 104
   <211> 30
   <212> PRT
   <213> clone 12 JUN dimerization inhibitory peptide without phage sequence
<220>
   <221> MISC_FEATURE
   <222> (7)..(7)
   <223> Xaa at position 7 is any amino acid
<400> 104
   Ala Tyr Gln Glu Ser Thr Xaa Ala Leu Val Glu Gly Gly Ala Asp Leu
   1               5                   10                  15
   Ile Leu Ile Glu Thr Val Leu Val Val Val Val Leu Val Val
               20                  25                  30
<210> 105
   <211> 297
   <212> DNA
   <213> clone 15 encoding JUN dimerization inhibitory peptide with phage sequence
<220>
   <221> misc_feature
   <222> (247)..(247)
   <223> N at position 247 is any nucleotide residue
<400> 105
   aggtcagact acaaggacga cgacgacaag acttatcaat caatcaatgg cccagaaaat      60
   aaagtgaaaa tgtatttttt gaatgattta aatttctcta gacgcgatgc tggatttaaa     120
   gcaagaaaag atgcacggga cattgcttca gattatgaaa acatttctgt tgttaacatt     180
   cctctatggg gtggagtagt ccagagaatt attagttctg ttaagcttag tacatttctc     240
   tgcggtnttg aaaataaaga tgttttaatt ttcaatttcc cgatggccaa accattt        297
<210> 106
   <211> 99
   <212> PRT
   <213> clone 15 JUN dimerization inhibitory peptide with phage sequence
<220>
   <221> MISC_FEATURE
   <222> (83)..(83)
   <223> Xaa at position 83 is any amino acid
<400> 106
   Arg Ser Asp Tyr Lys Asp Asp Asp Asp Lys Thr Tyr Gln Ser Ile Asn
   1               5                   10                  15
   Gly Pro Glu Asn Lys Val Lys Met Tyr Phe Leu Asn Asp Leu Asn Phe
               20                  25                  30
   Ser Arg Arg Asp Ala Gly Phe Lys Ala Arg Lys Asp Ala Arg Asp Ile
           35                  40                  45
   Ala Ser Asp Tyr Glu Asn Ile Ser Val Val Asn Ile Pro Leu Trp Gly
       50                  55                  60
   Gly Val Val Gln Arg Ile Ile Ser Ser Val Lys Leu Ser Thr Phe Leu
   65                  70                  75                  80
   Cys Gly Xaa Glu Asn Lys Asp Val Leu Ile Phe Asn Phe Pro Met Ala
                   85                  90                  95
   Lys Pro Phe
<210> 107
   <211> 89
   <212> PRT
   <213> clone 15 JUN dimerization inhibitory peptide without phage sequence
<220>
   <221> MISC_FEATURE
   <222> (73)..(73)
   <223> Xaa at position 73 is any amino acid
<400> 107
   Thr Tyr Gln Ser Ile Asn Gly Pro Glu Asn Lys Val Lys Met Tyr Phe
   1 5 10 15
   Leu Asn Asp Leu Asn Phe Ser Arg Arg Asp Ala Gly Phe Lys Ala Arg
   20 25 30
   Lys Asp Ala Arg Asp Ile Ala Ser Asp Tyr Glu Asn Ile Ser Val Val
   35 40 45
   Asn Ile Pro Leu Trp Gly Gly Val Val Gln Arg Ile Ile Ser Ser Val
   50 55 60
   Lys Leu Ser Thr Phe Leu Cys Gly Xaa Glu Asn Lys Asp Val Leu Ile
   65 70 75 80
   Phe Asn Phe Pro Met Ala Lys Pro Phe
   85
<210> 108
   <211> 63
   <212> DNA
   <213> clone 20 encoding JUN dimerization inhibitory peptide with phage sequence
<400> 108
   aggtcagact acaaggacga cgacgacaag atttattcat caattctatg ggggacaaaa 60
   tgg 63
<210> 109
   <211> 29
   <212> PRT
   <213> clone 20 JUN dimerization inhibitory peptide with phage sequence
<400> 109
   Arg Ser Asp Tyr Lys Asp Asp Asp Asp Lys Ile Tyr Ser Ser Ile Leu
   1 5 10 15
   Trp Gly Thr Lys Trp Cys Val Leu Leu Val Ile Thr Pro
   20 25
<210> 110
   <211> 19
   <212> PRT
   <213> clone 20 JUN dimerization inhibitory peptide without phage sequence
<400> 110
   Ile Tyr Ser Ser Ile Leu Trp Gly Thr Lys Trp Cys Val Leu Leu Val
   1 5 10 15
   Ile Thr Pro
<210> 111
   <211> 105
   <212> DNA
   <213> clone 21 encoding JUN dimerization inhibitory peptide with phage sequence
<220>
   <221> misc_feature
   <222> (53)..(53)
   <223> N at position 52 is any nucleotide residue
<400> 111
   aggtcagact acaaggacga cgacgacaag atcattattt atattttcct tancatctct 60
   aatagcatca aaaacatctt cgacaatatg ggtaaaatca gataa 105
<210> 112
   <211> 34
   <212> PRT
   <213> clone 21 JUN dimerization inhibitory peptide with phage sequence
<220>
   <221> MISC_FEATURE
   <222> (18)..(18)
   <223> Xaa at position 18 is any amino acid
<400> 112
   Arg Ser Asp Tyr Lys Asp Asp Asp Asp Lys Ile Ile Ile Tyr Ile Phe
   1 5 10 15
   Leu Xaa Ile Ser Asn Ser Ile Lys Asn Ile Phe Asp Asn Met Gly Lys
   20 25 30
   Ile Arg
<210> 113
   <211> 24
   <212> PRT
   <213> clone 21 JUN dimerization inhibitory peptide without phage sequence
<220>
   <221> MISC_FEATURE
   <222> (8)..(8)
   <223> Xaa at position 8 is any amino acid residue
<400> 113
   Ile Ile Ile Tyr Ile Phe Leu Xaa Ile Ser Asn Ser Ile Lys Asn Ile
   1 5 10 15
   Phe Asp Asn Met Gly Lys Ile Arg
   20
<210> 114
   <211> 156
   <212> DNA
   <213> clone 22 encoding JUN dimerization inhibitory peptide with phage sequence
<220>
   <221> misc_feature
   <222> (49)..(51)
   <223> N at any one of positions 49 to 51 is any nucleotide
<400> 114
   aggtcagact acaaggacga cgacgacaag aaggactcca tacggcggnn ncccgagaat 60
   atttcctcgc aggaagtcga ggccgtcctc atgtcgcatc ccgaagtcgt caatgccgcg 120
   gtctaccccg tacgcggcga tctgcctggt gattga 156
<210> 115
   <211> 51
   <212> PRT
   <213> clone 24 JUN dimerization inhibitory peptide with phage sequence
<220>
   <221> MISC_FEATURE
   <222> (17)..(17)
   <223> Xaa at position 17 is any amino acid
<400> 115
   Arg Ser Asp Tyr Lys Asp Asp Asp Asp Lys Lys Asp Ser Ile Arg Arg
   1               5                   10                  15
   Xaa Pro Glu Asn Ile Ser Ser Gln Glu Val Glu Ala Val Leu Met Ser
               20                  25                  30
   His Pro Glu Val Val Asn Ala Ala Val Tyr Pro Val Arg Gly Asp Leu
           35                  40                  45
   Pro Gly Asp
       50
<210> 116
   <211> 41
   <212> PRT
   <213> clone 22 JUN dimerization inhibitory peptide without phage sequence
<220>
   <221> MISC_FEATURE
   <222> (7)..(7)
   <223> Xaa at position 7 is any amino acid
<400> 116
   Lys Asp Ser Ile Arg Arg Xaa Pro Glu Asn Ile Ser Ser Gln Glu Val
   1               5                   10                  15
   Glu Ala Val Leu Met Ser His Pro Glu Val Val Asn Ala Ala Val Tyr
               20                  25                  30
   Pro Val Arg Gly Asp Leu Pro Gly Asp
           35                  40
<210> 117
   <211> 111
   <212> DNA
   <213> clone 24 encoding JUN dimerization inhibitory peptide with phage sequence
<400> 117
   aggtcagact acaaggacga cgacgacaag ctatatcaat cactactcac tgctaccaaa 60
   gaattgcttt ttgtcgcgcc tgtagcaaaa gcattcacat cgtgtgattg a 111
<210> 118
   <211> 36
   <212> PRT
   <213> clone 24 JUN dimerization inhibitory peptide with phage sequence
<400> 118
   Arg Ser Asp Tyr Lys Asp Asp Asp Asp Lys Leu Tyr Gln Ser Leu Leu
   1 5 10 15
   Thr Ala Thr Lys Glu Leu Leu Phe Val Ala Pro Val Ala Lys Ala Phe
   20 25 30
   Thr Ser Cys Asp
   35
<210> 119
   <211> 26
   <212> PRT
   <213> clone 24 JUN dimerization inhibitory peptide without phage sequence
<400> 119
   Leu Tyr Gln Ser Leu Leu Thr Ala Thr Lys Glu Leu Leu Phe Val Ala
   1 5 10 15
   Pro Val Ala Lys Ala Phe Thr Ser Cys Asp
   20 25
<210> 120
   <211> 186
   <212> DNA
   <213> clone 30 encoding JUN dimerization inhibitory peptide with phage sequence
<220>
   <221> misc_feature
   <222> (50)..(50)
   <223> N at position 50 is any nucleotide residue
<400> 120
   aggtcagact acaaggacga cgacgacaag gtctacgcct acttcggtan caccggcgat 60
   gttgtcgaag taggcgtaga ccttgtaggt atcgccggcg ttgcccacgc tcaggccgct 120
   gacccgcagg gccagcagca acagggccag caggccggcc aggaggaaca ggccgacacc 180
   gattga 186
<210> 121
   <211> 61
   <212> PRT
   <213> clone 30 JUN dimerization inhibitory peptide with phage sequence
<220>
   <221> MISC_FEATURE
   <222> (17)..(17)
   <223> Xaa at position 17 is any amino acid
<400> 121
   Arg Ser Asp Tyr Lys Asp Asp Asp Asp Lys Val Tyr Ala Tyr Phe Gly
   1               5                   10                  15
   Xaa Thr Gly Asp Val Val Glu Val Gly Val Asp Leu Val Gly Ile Ala
               20                  25                  30
   Gly Val Ala His Ala Gln Ala Ala Asp Pro Gln Gly Gln Gln Gln Gln
           35                  40                  45
   Gly Gln Gln Ala Gly Gln Glu Glu Gln Ala Asp Thr Asp
       50                  55                  60
<210> 122
   <211> 51
   <212> PRT
   <213> clone 30 JUN dimerization inhibitory peptide without phage sequence
<220>
   <221> MISC_FEATURE
   <222> (7)..(7)
   <223> Xaa at position 7 is any amino acid
<400> 122
   Val Tyr Ala Tyr Phe Gly Xaa Thr Gly Asp Val Val Glu Val Gly Val
   1               5                   10                  15
   Asp Leu Val Gly Ile Ala Gly Val Ala His Ala Gln Ala Ala Asp Pro
               20                  25                  30
   Gln Gly Gln Gln Gln Gln Gly Gln Gln Ala Gly Gln Glu Glu Gln Ala
           35                  40                  45
   Asp Thr Asp
       50
<210> 123
   <211> 66
   <212> DNA
   <213> clone 32 encoding JUN dimerization inhibitory peptide with phage sequence
<400> 123
   aggtcagact acaaggacga cgacgacaat accccccact cctccgatgg ccacaataat 60
   ccctaa 66
<210> 124
   <211> 21
   <212> PRT
   <213> clone 32 JUN dimerization inhibitory peptide with phage sequence
<400> 124
   Arg Ser Asp Tyr Lys Asp Asp Asp Asp Asn Thr Pro His Ser Ser Asp
   1               5                   10                  15
   Gly His Asn Asn Pro
               20
<210> 125
   <211> 11
   <212> PRT
   <213> clone 32 JUN dimerization inhibitory peptide without phage sequence
<400> 125
   Thr Pro His Ser Ser Asp Gly His Asn Asn Pro
   1               5                   10
<210> 126
   <211> 60
   <212> DNA
   <213> clone 33 encoding JUN dimerization inhibitory peptide with phage sequence
<400> 126
   aggtcagact acaaggacga cgacgacaag gcttatcaat caatcaaatg gccaatgtaa 60
<210> 127
   <211> 19
   <212> PRT
   <213> clone 33 JUN dimerization inhibitory peptide with phage sequence
<400> 127
   Arg Ser Asp Tyr Lys Asp Asp Asp Asp Lys Ala Tyr Gln Ser Ile Lys
   1               5                   10                  15
   Trp Pro Met
<210> 128
   <211> 9
   <212> PRT
   <213> clone 33 JUN dimerization inhibitory peptide without phage sequence
<400> 128
   Ala Tyr Gln Ser Ile Lys Trp Pro Met
   1               5
<210> 129
   <211> 120
   <212> DNA
   <213> clone 34 encoding JUN dimerization inhibitory peptide with phage sequence
<400> 129
   aggtcagact acaaggacga cgacgacaag gcttatcaat caataaattc gtcaccagta 60
   ttgccagaaa atagtcaaga attatcactt cacttaaagc aacacgtaac aaaatcatga 120
<210> 130
   <211> 39
   <212> PRT
   <213> clone 34 JUN dimerization inhibitory peptide with phage sequence
<400> 130
   Arg Ser Asp Tyr Lys Asp Asp Asp Asp Lys Ala Tyr Gln Ser Ile Asn
   1               5                   10                  15
   Ser Ser Pro Val Leu Pro Glu Asn Ser Gln Glu Leu Ser Leu His Leu
               20                  25                  30
   Lys Gln His Val Thr Lys Ser
           35
<210> 131
   <211> 29
   <212> PRT
   <213> clone 34 JUN dimerization inhibitory peptide without phage sequence
<400> 131
   Ala Tyr Gln Ser Ile Asn Ser Ser Pro Val Leu Pro Glu Asn Ser Gln
   1               5                   10                  15
   Glu Leu Ser Leu His Leu Lys Gln His Val Thr Lys Ser
               20                  25
<210> 132
   <211> 213
   <212> DNA
   <213> clone 35 encoding JUN dimerization inhibitory peptide with phage sequence
<400> 132
   aggtcagact acaaggacga cgacgacaag gcttatcaat caatcaggtc tggagggata     60
   gagtcgagtt cgaaaaggga gaaggtaagg gtgggaatga ccctaaggac ttacaatcca    120
   aacgaaacct tcttctctat tcttcacgag tttgtgaagt tccttaagag gaggagacta    180
   cttcaagagg ccatagactt gtcgtcgtcg tcc                                 213
<210> .133
   <211> 72
   <212> PRT
   <213> clone 35 JUN dimerization inhibitory peptide with phage sequence
<400> 133
   Arg Ser Asp Tyr Lys Asp Asp Asp Asp Lys Ala Tyr Gln Ser Ile Arg
   1               5                   10                  15
   Ser Gly Gly Ile Glu Ser Ser Ser Lys Arg Glu Lys Val Arg Val Gly
               20                  25                  30
   Met Thr Leu Arg Thr Tyr Asn Pro Asn Glu Thr Phe Phe Ser Ile Leu
           35                  40                  45
   His Glu Phe Val Lys Phe Leu Lys Arg Arg Arg Leu Leu Gln Glu Ala
       50                  55                  60
   Ile Asp Leu Ser Ser Ser Ser Leu
   65                  70
<210> 134
   <211> 62
   <212> PRT
   <213> clone 35 JUN dimerization inhibitory peptide without phage sequence
<400> 134
   Ala Tyr Gln Ser Ile Arg Ser Gly Gly Ile Glu Ser Ser Ser Lys Arg
   1               5                   10                  15
   Glu Lys Val Arg Val Gly Met Thr Leu Arg Thr Tyr Asn Pro Asn Glu
               20                  25                  30
   Thr Phe Phe Ser Ile Leu His Glu Phe Val Lys Phe Leu Lys Arg Arg
   35 40 45
   Arg Leu Leu Gln Glu Ala Ile Asp Leu Ser Ser Ser Ser Leu
   50 55 60
<210> 135
   <211> 78
   <212> DNA
   <213> clone 36 encoding JUN dimerization inhibitory peptide with phage sequence
<400> 135
   aggtcagact acaaggacga cgacgacaag ggactacaag gacgacgacg acaaggttat 60
   caatcaatca agccatga 78
<210> 136
   <211> 25
   <212> PRT
   <213> clone 36 JUN dimerization inhibitory peptide with phage sequence
<400> 136
   Arg Ser Asp Tyr Lys Asp Asp Asp Asp Lys Gly Leu Gln Gly Arg Arg
   1 5 10 15
   Arg Gln Gly Tyr Gln Ser Ile Lys Pro
   20 25
<210> 137
   <211> 15
   <212> PRT
   <213> clone 36 JUN dimerization inhibitory peptide without phage sequence
<400> 137
   Gly Leu Gln Gly Arg Arg Arg Gln Gly Tyr Gln Ser Ile Lys Pro
   1 5 10 15
<210> 138
   <211> 69
   <212> DNA
   <213> clone 39 encoding JUN dimerization inhibitory peptide with phage sequence
<220>
   <221> misc_feature
   <222> (35).. (35)
   <223> N at position 35 is any nucleotide residue
<220>
   <221> misc_feature
   <222> (42).. (42)
   <223> N at position 42 is any nucleotide residue
<220>
   <221> misc_feature
   <222> (43)..(43)
   <223> N at position 43 is any nucleotide residue
<220>
   <221> misc_feature
   <222> (65)..(65) .
   <223> N at position 65 is any nucleotide residue
<400> 138
   aggtcagact acaaggacga cgacgacaag ggacnacaag gnngccgaaa cctggccaac     60
   agccnctaa                                                             69
<210> 139
   <211> 22
   <212> PRT
   <213> clone 39 JUN dimerization inhibitory peptide with phage sequence
<220>
   <221> MISC_FEATURE
   <222> (12)..(12)
   <223> Xaa at position 12 is any amino acid
<220>
   <221> MISC_FEATURE
   <222> (15)..(15)
   <223> Xaa at position 15 is any amino acid
<220>
   <221> MISC_FEATURE
   <222> (22).. (22)
   <223> Xaa at position 22 is any amino acid
<400> 139
   Arg Ser Asp Tyr Lys Asp Asp Asp Asp Lys Gly Xaa Gln Gly Xaa Arg
   1               5                   10                  15
   Asn Leu Ala Asn Ser Xaa
               20
<210> 140
   <211> 12
   <212> PRT
   <213> clone 39 JUN dimerization inhibitory peptide without phage sequence
<220>
   <221> MISC_FEATURE
   <222> (2)..(2)
   <223> Xaa at position 2 is any amino acid residue
<220>
   <221> MISC_FEATURE
   <222> (5)..(5)
   <223> Xaa at position 5 is any amino acid residue
<220>
   <221> MISC_FEATURE
   <222> (12)..(12)
   <223> Xaa at position 12 is any amino acid residue
<400> 140
   Gly Xaa Gln Gly Xaa Arg Asn Leu Ala Asn Ser Xaa
   1               5                   10
<210> 141
   <211> 66
   <212> DNA
   <213> clone 43 encoding JUN dimerization inhibitory peptide with phage sequence
<400> 141
   aggtcagact acaaggacga cgacgacaag gcttatcaat caattcatcc aaggttgact    60
   aaatga                                                               66
<210> 142
   <211> 20
   <212> PRT
   <213> clone 43 JUN dimerization inhibitory peptide with phage sequence
<400> 142
   Ser Asp Tyr Lys Asp Asp Asp Asp Lys Ala Tyr Gln Ser Ile His Pro
   1               5                   10                  15
   Arg Leu Thr Lys
               20
<210> 143
   <211> 11
   <212> PRT
   <213> clone 43 JUN dimerization inhibitory peptide without phage sequence
<400> 143
   Ala Tyr Gln Ser Ile His Pro Arg Leu Thr Lys
   1 5 10
<210> 144
   <211> 216
   <212> DNA
   <213> clone 54 encoding JUN dimerization inhibitory peptide with phage sequence
<220>
   <221> misc_feature
   <222> (47).. (47).
   <223> N at position 47 is any nucleotide residue
<220>
   <221> misc_feature
   <222> (49)..(49)
   <223> N at position 49 is any nucleotide residue
<220>
   <221> misc_feature
   <222> (50)..(50)
   <223> N at position 50 is any nucleotide residue
<220>
   <221> misc_feature
   <222> (66)..(66)
   <223> N at position 66 is any nucleotide residue
<220>
   <221> misc_feature
   <222> (67)..(67)
   <223> N at position 67 is any nucleotide residue
<400> 144
   aggtcagact acaaggacga cgacgacaag gcttatcaat cattccncnn ggcaggctac 60
   cacggnnaca cttcgagaac atttctagtg ggttcggtat ccgcaactgc ccgaaaatta 120
   gttgaagcga ctcaagaaac gatgattgat tatacttgtc gtcgtcgtcc ttgtagtctg 180
   acctggtacc aattgatgca tcgataccgg tactag 216
<210> 145
   <211> 71
   <212> PRT
   <213> clone 54 JUN dimerization inhibitory peptide with phage sequence
<220>
   <221> MISC_FEATURE
   <222> (16)..(16)
   <223> Xaa at position 16 is any amino acid
<220>
   <221> MISC_FEATURE
   <222> (17)..(17)
   <223> Xaa at position 17 is any amino acid
<220>
   <221> MISC_FEATURE
   <222> (23)..(23)
   <223> Xaa at position 23 is any amino acid
<400> 145
   Arg Ser Asp Tyr Lys Asp Asp Asp Asp Lys Ala Tyr Gln Ser Phe Xaa
   1               5                   10                  15
   Xaa Ala Gly Tyr His Gly Xaa Thr Ser Arg Thr Phe Leu Val Gly Ser
               20                  25                  30
   Val Ser Ala Thr Ala Arg Lys Leu Val Glu Ala Thr Gln Glu Thr Met
           35                  40                  45
   Ile Asp Tyr Thr Cys Arg Arg Arg Pro Cys Ser Leu Thr Trp Tyr Gln
       50                  55                  60
   Leu Met His Arg Tyr Arg Tyr
   65                  70
<210> 146
   <211> 61
   <212> PRT
   <213> clone 54 JUN dimerization inhibitory peptide without phage sequence
<220>
   <221> MISC_FEATURE
   <222> (6)..(6)
   <223> Xaa at position 6 is any amino acid
<220>
   <221> MISC_FEATURE
   <222> (7)..(7)
   <223> Xaa at position 7 is any amino acid
<220>
   <221> MISC_FEATURE
   <222> (13)..(13)
   <223> Xaa at position 13 is any amino acid
<400> 146
   Ala Tyr Gln Ser Phe Xaa Xaa Ala Gly Tyr His Gly Xaa Thr Ser Arg
   1               5                   10                  15
   Thr Phe Leu Val Gly Ser Val Ser Ala Thr Ala Arg Lys Leu Val Glu
               20                  25                  30
   Ala Thr Gln Glu Thr Met Ile Asp Tyr Thr Cys Arg Arg Arg Pro Cys
           35                  40                  45
   Ser Leu Thr Trp Tyr Gln Leu Met His Arg Tyr Arg Tyr
       50                  55                  60
<210> 147
   <211> 222
   <212> DNA
   <213> clone 58 encoding JUN dimerization inhibitory peptide with phage sequence
<400> 147
   aggtcagact acaaggacga cgacgacaag gcttatcaat caatcatggc agtggctgcc 60
   cagcagccgg tcgcgttcct ggtaggccgc cagcgtcgcc gcggtcaggt aggaatcgac 120
   tccggcgatc agcaccttcg aacacccctg ttccatgagc tttgtcgtcg tcgtccttgt 180
   agtctggcct ggtaccaatt gatgcatcga taccggtact ag 222
<210> 148
   <211> 73
   <212> PRT
   <213> clone 58 JUN dimerization inhibitory peptide with phage sequence
<400> 148
   Arg Ser Asp Tyr Lys Asp Asp Asp Asp Lys Ala Tyr Gln Ser Ile Met
   1               5                   10                  15
   Ala Val Ala Ala Gln Gln Pro Val Ala Phe Leu Val Gly Arg Gln Arg
               20                  25                  30
   Arg Arg Gly Gln Val Gly Ile Asp Ser Gly Asp Gln His Leu Arg Thr
           35                  40                  45
   Pro Leu Phe His Glu Leu Cys Arg Arg Arg Pro Cys Ser Leu Ala Trp
       50                  55                  60
   Tyr Gln Leu Met His Arg Tyr Arg Tyr
   65                  70
<210> 149
   <211> 63
   <212> PRT
   <213> clone 58 JUN dimerization inhibitory peptide without phage sequence
<400> 149
   Ala Tyr Gln Ser Ile Met Ala Val Ala Ala Gln Gln Pro Val Ala Phe
   1               5                   10                  15
   Leu Val Gly Arg Gln Arg Arg Arg Gly Gln Val Gly Ile Asp Ser Gly
               20                  25                  30
   Asp Gln His Leu Arg Thr Pro Leu Phe His Glu Leu Cys Arg Arg Arg
           35                  40                  45
   Pro Cys Ser Leu Ala Trp Tyr Gln Leu Met His Arg Tyr Arg Tyr
       50                  55                  60
<210> 150
   <211> 84
   <212> DNA
   <213> clone 60 encoding JUN dimerization inhibitory peptide with phage sequence
<400> 150
   aggtcagact acaaggacga cgacgacaag gctaatcaat tgcccaaaat acttgctgga      60
   cggcttatat ttataaagtg ctaa                                             84
<210> 151
   <211> 27
   <212> PRT
   <213> clone 60 JUN dimerization inhibitory peptide with phage sequence
<400> 151
   Arg Ser Asp Tyr Lys Asp Asp Asp Asp Lys Ala Asn Gln Leu Pro Lys
   1               5                   10                  15
   Ile Leu Ala Gly Arg Leu Ile Phe Ile Lys Cys
               20                  25
<210> 152
   <211> 17
   <212> PRT
   <213> clone 60 JUN dimerization inhibitory peptide without phage sequence
<400> 152
   Ala Asn Gln Leu Pro Lys Ile Leu Ala Gly Arg Leu Ile Phe Ile Lys
   1 5 10 15
   Cys
<210> 153
   <211> 225
   <212> DNA
   <213> clone 66 encoding JUN dimerization inhibitory peptide with phage sequence
<400> 153
   aggtcagact acaaggacga cgacgacaag gcttatcaat caatcatagg ggcgggaaaa 60
   tcaacgctaa tcaaagcatt aactggcgta taccacgccg atcgcggcac catctggctg 120
   gaaggccagg ctatctcacc gaaaaatacc gcccacgcgc aacaatgtcg tcgtcgtcct 180
   tgtagtctga cctggtacca attgatgcat cgataccggt actag 225
<210> 154
   <211> 74
   <212> PRT
   <213> clone 66 JUN dimerization inhibitory peptide with phage sequence
<400> 154
   Arg Ser Asp Tyr Lys Asp Asp Asp Asp Lys Ala Tyr Gln Ser Ile Ile
   1 5 10 15
   Gly Ala Gly Lys Ser Thr Leu Ile Lys Ala Leu Thr Gly Val Tyr His
   20 25 30
   Ala Asp Arg Gly Thr Ile Trp Leu Glu Gly Gln Ala Ile Ser Pro Lys
   35 40 45
   Asn Thr Ala His Ala Gln Gln Cys Arg Arg Arg Pro Cys Ser Leu Thr
   50 55 60
   Trp Tyr Gln Leu Met His Arg Tyr Arg Tyr
   65 70
<210> 155
   <211> 64
   <212> PRT
   <213> clone 66 JUN dimerization inhibitory peptide without phage sequence
<400> 155
   Ala Tyr Gln Ser Ile Ile Gly Ala Gly Lys Ser Thr Leu Ile Lys Ala
   1 5 10 15
   Leu Thr Gly Val Tyr His Ala Asp Arg Gly Thr Ile Trp Leu Glu Gly
   20 25 30
   Gln Ala Ile Ser Pro Lys Asn Thr Ala His Ala Gln Gln Cys Arg Arg
   35 40 45
   Arg Pro Cys Ser Leu Thr Trp Tyr Gln Leu Met His Arg Tyr Arg Tyr
   50 55 60
<210> 156
   <211> 123
   <212> DNA
   <213> clones 72, 73, 76 and 77 encoding JUN dimerization inhibitory peptide with phage sequence
<400> 156
   gagctcagat ctcagctggg cccggtacca attgatgcat cgataccggt actagtcgga 60
   ccgcatatgc ccgggcgtac cgcggccgct cgaggcatgc atctagaggg ccgcatcatg 120
   taa 123
<210> 157
   <211> 40
   -<212> PRT
   <213> clones 72, 73, 76 and 77 JUN dimerization inhibitory peptide with phage sequence
<400> 157
   Glu Leu Arg Ser Gln Leu Gly Pro Val Pro Ile Asp Ala Ser Ile Pro
   1 5 10 15
   Val Leu Val Gly Pro His Met Pro Gly Arg Thr Ala Ala Ala Arg Gly
   20 25 30
   Met His Leu Glu Gly Arg Ile Met
   35 40
<210> 158
   <211> 40
   <212> PRT
   <213> clones 72, 73, 76 and 77 JUN dimerization inhibitory peptide without phage sequence
<400> 158
   Glu Leu Arg Ser Gln Leu Gly Pro Val Pro Ile Asp Ala Ser Ile Pro
   1 5 10 15
   Val Leu Val Gly Pro His Met Pro Gly Arg Thr Ala Ala Ala Arg Gly
   20 25 30
   Met His Leu Glu Gly Arg Ile Met
   35 40
<210> 159
   <211> 60
   <212> DNA
   <213> clone 79 encoding JUN dimerization inhibitory peptide with phage sequence
<400> 159
   aggtcagact acaaggacga cgacgacaag gcttatcaat caatcaaatg gccaatgtaa 60
<210> 160
   <211> 19
   <212> PRT
   <213> clone 79 JUN dimerization inhibitory peptide with phage sequence
<400> 160
   Arg Ser Asp Tyr Lys Asp Asp Asp Asp Lys Ala Tyr Gln Ser Ile Lys
   1 5 10 15
   Trp Pro Met
<210> 161
   <211> 9
   <212> PRT
   <213> clone 79 JUN dimerization inhibitory peptide without phage sequence
<400> 161
   Ala Tyr Gln Ser Ile Lys Trp Pro Met
   1 5
<210> 162
   <211> 111
   <212> DNA
   <213> clone 80 encoding JUN dimerization inhibitory peptide with phage sequence
<400> 162
   aggtcagact acaaggacga cgacgacaag gcttatcaat catccactgg tagtgctgcc 60
   atcctcttca atttccgtcg aatggggatc gtgataataa ttcagatcta a 111
<210> 163
   <211> 36
   <212> PRT
   <213> clone 80 JUN dimerization inhibitory peptide with phage sequence
<400> 163
   Arg Ser Asp Tyr Lys Asp Asp Asp Asp Lys Ala Tyr Gln Ser Ser Thr
   1 5 10 15
   Gly Ser Ala Ala Ile Leu Phe Asn Phe Arg Arg Met Gly Ile Val Ile
   20 25 30
   Ile Ile Gln Ile
   35
<210> 164
   <211> 26
   <212> PRT
   <213> clone 80 JUN dimerization inhibitory peptide without phage sequence
<400> 164
   Ala Tyr Gln Ser Ser Thr Gly Ser Ala Ala Ile Leu Phe Asn Phe Arg
   1 5 10 15
   Arg Met Gly Ile Val Ile Ile Ile Gln Ile
   20 25
<210> 165
   <211> 6482
   <212> DNA
   <213> pMF4-5 vector
<400> 165
   cgtacccatt atcttagcct aaaaaaacct tctctttgga actttcagta atacgcttaa 60
   ctgctcattg ctatattgaa gtacggatta gaagccgccg agcgggtgac agccctccga 120
   aggaagactc tcctccgtgc gtcctcgtct tcaccggtcg cgttcctgaa acgcagatgt 180
   gcctcgcgcc gcactgctcc gaacaataaa gattctacaa tactagcttt tatggttatg 240
   aagaggaaaa attggcagta acctggcccc acaaaccttc aaatgaacga atcaaattaa 300
   caaccatagg atgataatgc gattagtttt ttagccttat ttctggggta attaatcagc    360
   gaagcgatga tttttgatct attaacagat atataaatgc aaaaactgca taaccacttt    420
   aactaatact ttcaacattt tcggtttgta ttacttctta ttcaaatgta ataaaagtat    480
   caacaaaaaa ttgttaatat acctctatac tttaacgtca aggaggaatt aagcttatgg    540
   gtgctcctcc aaaaaagaag agaaaggtag ctggtatcaa taaagatatc gaggagtgca    600
   atgccatcat tgagcagttt atcgactacc tgcgcaccgg acaggagatg ccgatggaaa    660
   tggcggatca ggcgattaac gtggtgccgg gcatgacgcc gaaaaccatt cttcacgccg    720
   ggccgccgat ccagcctgac tggctgaaat cgaatggttt tcatgaaatt gaagcggatg    780
   ttaacgatac cagcctcttg ctgagtggag atgcctccta cccttatgat gtgccagatt    840
   atgcctctcc cgaattccgt tgtgcaggta ccagagtact gagcggccgc aatctcgaga    900
   agctttggac ttcttcgcca gaggtttggt caagtctcca atcaaggttg tcggcttgtc    960
   taccttgcca gaaatttacg aaaagatgga aaagggtcaa atcgttggta gatacgttgt   1020
   tgacacttct aaataagcga atttcttatg atttatgatt tttattatta aataagttat   1080
   aaaaaaaata agtgtataca aattttaaag tgactcttag gttttaaaac gaaaattctt   1140
   gttcttgagt aactctttcc tgtaggtcag gttgctttct caggtatagc atgaggtcgc   1200
   tcttattgac cacacctcta ccggcatgcc gagcaaatgc ctgcaaatcg ctccccattt   1260
   cacccaattg tagatatgct aactccagca atgagttgat gaatctcggt gtgtatttta   1320
   tgtcctcaga ggacaacacc tgttgtaatc gttcttccac acggatcctc tagagtcgac   1380
   tagcggccgc ttcgacctgc agcaattctg aaccagtcct aaaacgagta aataggaccg   1440
   gcaattcttc aagcaataaa caggaatacc aattattaaa agataactta gtcagatcgt   1500
   acaataaagc tttgaagaaa aatgcgcctt attcaatctt tgctataaaa aatggcccaa   1560
   aatctcacat tggaagacat ttgatgacct catttctttc aatgaagggc ctaacggagt   1620
   tgactaatgt tgtgggaaat tggagcgata agcgtgcttc tgccgtggcc aggacaacgt   1680
   atactcatca gataacagca atacctgatc actacttcgc actagtttct cggtactatg   1740
   catatgatcc aatatcaaag gaaatgatag cattgaagga tgagactaat ccaattgagg   1800
   agtggcagca tatagaacag ctaaagggta gtgctgaagg aagcatacga taccccgcat   1860
   ggaatgggat aatatcacag gaggtactag actacctttc atcctacata aatagacgca   1920
   tataagtacg catttaagca taaacacgca ctatgccgtt cttctcatgt atatatatat   1980
   acaggcaaca cgcagatata ggtgcgacgt gaacagtgag ctgtatgtgc gcagctcgcg   2040
   ttgcattttc ggaagcgctc gttttcggaa acgctttgaa gttcctattc cgaagttcct   2100
   attctctaga aagtatagga acttcagagc gcttttgaaa accaaaagcg ctctgaagac   2160
   gcactttcaa aaaaccaaaa acgcaccgga ctgtaacgag ctactaaaat attgcgaata   2220
   ccgcttccac aaacattgct caaaagtatc tctttgctat atatctctgt gctatatccc 2280
   tatataacct acccatccac ctttcgctcc ttgaacttgc atctaaactc gacctctaca 2340
   ttttttatgt ttatctctag tattactctt tagacaaaaa aattgtagta agaactattc 2400
   atagagtgaa tcgaaaacaa tacgaaaatg taaacatttc ctatacgtag tatatagaga 2460
   caaaatagaa gaaaccgttc ataattttct gaccaatgaa gaatcatcaa cgctatcact 2520
   ttctgttcac aaagtatgcg caatccacat cggtatagaa tataatcggg gatgccttta 2580
   tcttgaaaaa atgcacccgc agcttcgcta gtaatcagta aacgcgggaa gtggagtcag 2640
   gcttttttta tggaagagaa aatagacacc aaagtagcct tcttctaacc ttaacggacc 2700
   tacagtgcaa aaagttatca agagactgca ttatagagcg cacaaaggag aaaaaaagta 2760
   atctaagatg ctttgttaga aaaatagcgc tctcgggatg catttttgta gaacaaaaaa 2820
   gaagtataga ttctttgttg gtaaaatagc gctctcgcgt tgcatttctg ttctgtaaaa 2880
   atgcagctca gattctttgt ttgaaaaatt agcgctctcg cgttgcattt ttgttttaca 2940
   aaaatgaagc acagattctt cgttggtaaa atagcgcttt cgcgttgcat ttctgttctg 3000
   taaaaatgca gctcagattc tttgtttgaa aaattagcgc tctcgcgttg catttttgtt 3060
   ctacaaaatg aagcacagat gcttcgttaa caaagatatg ctattgaagt gcaagatgga 3120
   aacgcagaaa atgaaccggg gatgcgacgt gcaagattac ctatgcaata gatgcaatag 3180
   tttctccagg aaccgaaata catacattgt cttccgtaaa gcgctagact atatattatt 3240
   atacaggttc aaatatacta tctgtttcag ggaaaactcc caggttcgga tgttcaaaat 3300
   tcaatgatgg gtaacaagta cgatcgtaaa tctgtaaaac agtttgtcgg atattaggct 3360
   gtatctcctc aaagcgtatt cgaatatcat tgagaagctg caggcaagtg cacaaacaat 3420
   acttaaataa atactactca gtaataacct atttcttagc atttttgacg aaatttgcta 3480
   ttttgttaga gtcttttaca ccatttgtct ccacacctcc gcttacatca acaccaataa 3540
   cgccatttaa tctaagcgca tcaccaacat tttctggcgt cagtccacca gctaacataa 3600
   aatgtaagct ttcggggctc tcttgccttc caacccagtc agaaatcgag ttccaatcca 3660
   aaagttcacc tgtcccacct gcttctgaat caaacaaggg aataaacgaa tgaggtttct 3720
   gtgaagctgc actgagtagt atgttgcagt cttttggaaa tacgagtctt ttaataactg 3780
   gcaaaccgag gaactcttgg tattcttgcc acgactcatc tccatgcagt tggacgatat 3840
   caatgccgta atcattgacc agagccaaaa catcctcctt aggttgatta cgaaacacgc 3900
   caaccaagta tttcggagtg cctgaactat ttttatatgc ttttacaaga cttgaaattt 3960
   tccttgcaat aaccgggtca attgttctct ttctattggg cacacatata atacccagca 4020
   agtcagcatc ggaatctaga gcacattctg cggcctctgt gctctgcaag ccgcaaactt 4080
   tcaccaatgg accagaacta cctgtgaaat taataacaga catactccaa gctgcctttg 4140
   tgtgcttaat cacgtatact cacgtgctca atagtcacca atgccctccc tcttggccct 4200
   ctccttttct tttttcgacc gaatttcttg aagacgaaag ggcctcgtga tacgcctatt 4260
   tttataggtt aatgtcatga taataatggt ttcttagacg tcaggtggca cttttcgggg 4320
   aaatgtgcgc ggaaccccta tttgtttatt tttctaaata cattcaaata tgtatccgct 4380
   catgagacaa taaccctgat aaatgcttca ataatattga aaaaggaaga gtatgagtat 4440
   tcaacatttc cgtgtcgccc ttattccctt ttttgcggca ttttgccttc ctgtttttgc 4500
   tcacccagaa acgctggtga aagtaaaaga tgctgaagat cagttgggtg cacgagtggg 4560
   ttacatcgaa ctggatctca acagcggtaa gatccttgag agttttcgcc ccgaagaacg 4620
   ttttccaatg atgagcactt ttaaagttct gctatgtggc gcggtattat cccgtgttga 4680
   cgccgggcaa gagcaactcg gtcgccgcat acactattct cagaatgact tggttgagta 4740
   ctcaccagtc acagaaaagc atcttacgga tggcatgaca gtaagagaat tatgcagtgc 4800
   tgccataacc atgagtgata acactgcggc caacttactt ctgacaacga tcggaggacc 4860
   gaaggagcta accgcttttt tgcacaacat gggggatcat gtaactcgcc ttgatcgttg 4920
   ggaaccggag ctgaatgaag ccataccaaa cgacgagcgt gacaccacga tgcctgtagc 4980
   aatggcaaca acgttgcgca aactattaac tggcgaacta cttactctag cttcccggca 5040
   acaattaata gactggatgg aggcggataa agttgcagga ccacttctgc gctcggccct 5100
   tccggctggc tggtttattg ctgataaatc tggagccggt gagcgtgggt ctcgcggtat 5160
   cattgcagca ctggggccag atggtaagcc ctcccgtatc gtagttatct acacgacggg 5220
   gagtcaggca actatggatg aacgaaatag acagatcgct gagataggtg cctcactgat 5280
   taagcattgg taactgtcag accaagttta ctcatatata ctttagattg atttaaaact 5340
   tcatttttaa tttaaaagga tctaggtgaa gatccttttt gataatctca tgaccaaaat 5400
   cccttaacgt gagttttcgt tccactgagc gtcagacccc gtagaaaaga tcaaaggatc 5460
   ttcttgagat cctttttttc tgcgcgtaat ctgctgcttg caaacaaaaa aaccaccgct 5520
   accagcggtg gtttgtttgc cggatcaaga gctaccaact ctttttccga aggtaactgg 5580
   cttcagcaga gcgcagatac caaatactgt ccttctagtg tagccgtagt taggccacca 5640
   cttcaagaac tctgtagcac cgcctacata cctcgctctg ctaatcctgt taccagtggc 5700
   tgctgccagt ggcgataagt cgtgtcttac cgggttggac tcaagacgat agttaccgga 5760
   taaggcgcag cggtcgggct gaacgggggg ttcgtgcaca cagcccagct tggagcgaac 5820
   gacctacacc gaactgagat acctacagcg tgagctatga gaaagcgcca cgcttcccga 5880
   agggagaaag gcggacaggt atccggtaag cggcagggtc ggaacaggag agcgcacgag 5940
   ggagcttcca gggggaaacg cctggtatct ttatagtcct gtcgggtttc gccacctctg 6000
   acttgagcgt cgatttttgt gatgctcgtc aggggggcgg agcctatgga aaaacgccag 6060
   caacgcggcc tttttacggt tcctggcctt ttgctggcct tttgctcaca tgttctttcc 6120
   tgcgttatcc cctgattctg tggataaccg tattaccgcc tttgagtgag ctgataccgc 6180
   tcgccgcagc cgaacgaccg agcgcagcga gtcagtgagc gaggaagcgg aagagcgccc 6240
   aatacgcaaa ccgcctctcc ccgcgcgttg gccgattcat taatgcagct ggcacgacag 6300
   gtttcccgac tggaaagcgg gcagtgagcg caacgcaatt aatgtgagtt agctcactca 6360
   ttaggcaccc caggctttac actttatgct tccggctcgt atgttgtgtg gaattgtgag 6420
   cggataacaa tttcacacag gaaacagcta tgacatgatt acgaattaat tcgagctcgg 6480
   ta 6482
<210> 166
   <211> 2648
   <212> DNA
   <213> P. falciparum alpha-1 tubulin
<220>
   <221> exon
   <222> (626)..(687)
   <223>
<220>
   <221> exon
   <222> (1042)..(1162)
   <223>
<220>
   <221> exon
   <222> (1347)..(2525)
   <223>
<400> 166
   atatatatat atatatttgt acagaaaata ttttaataaa aaaaaaaaaa aattttaagc      60
   atttcatatt tatatacact tgttttttta tgtgttgaaa gaaaaaaaaa aattatgcta     120
   tatcatattt atatgaccat tttaatgata ctgtgaaagt atacatatat aattaattat     180
   ataataataa taatatatat atacatatat atataatact ctgtttttct tttttttttt     240
   ctttttcttt ttcctttttt tttttttttg cactctaaag gtttgagtaa gtaaatatta     300
   tatataatat atataaaagt ataatttttt tacaaataat taatagattt attttataag     360
   aattaaaaat aataaaatta aaaaaaaaaa aaagaaaaat ttacatatta ttatatatat     420
   atatatatat ttaaatatat atttaaaaaa aaaatatatt ttaatgataa agaagaaata     480
   ttcatacaat aattaataat atatatttga tttgtatttt tttttttttt tttatataca     540
   tatttatata ttttttttta aatcatcatt taagaaaaga aaaagaaaaa aaaagaatat     600
   ttattttaat atatttaaat aaaaa atg aga gaa gta ata agt atc cat gta       652
                               Met Arg Glu Val Ile Ser Ile His Val
                               1               5
   gga caa gct ggt atc caa gtt gga aat gct tgc tg  gtaaaacgat            697
   Gly Gln Ala Gly Ile Gln Val Gly Asn Ala Cys Trp
   10                  15                  20
   aagcttatat attcttacac attattatat taatatatat atatatatat atatatatat    757
   aagaaaaaaa aattatatca tatttattgt gttttcattc ataaaagtta aaatcatatg    817
   tgtgtgtgta atgttctaaa gaatacataa atatatatat atatatatat atatatatat    877
   atatatatat aatacataat aaattcataa tatatatatg tatatattat cgatgtatgt    937
   atacacacct gttttaaatg tatatatatc tataaaaagt tatgcatact tatgaataca    997
   tcttttcttt attcttttta taaataattt gttgaatttt ttag g gaa ttg ttt     1051
                                                     Glu Leu Phe
   tgc cta gag cat gga ata cag ccc gat ggt caa atg ccc tct gac aag     1099
   Cys Leu Glu His Gly Ile Gln Pro Asp Gly Gln Met Pro Ser Asp Lys
   25                  30                  35                  40
   gct tct aga gct aat gat gat gct ttt aat aca ttc ttt tca gaa acg     1147
   Ala Ser Arg Ala Asn Asp Asp Ala Phe Asn Thr Phe Phe Ser Glu Thr
                   45                  50                  55
   ggg gca gga aaa cat gtaagaaata atatacgctg atataatata atatatatat     1202
   Gly Ala Gly Lys His
               60
   atatatatat atatatttgt aacttataaa aagttgtcct ttatataaca gttaaagaac    1262
   atatataaca atatagcata tttattaaaa taagtatata catatatata tatatatata    1322
   tttttttttt tttttccttt ttag gta cca cgt tgt gtt ttt gtc gat tta      1373
                              Val Pro Arg Cys Val Phe Val Asp Leu
                                          65                  70
   gag cca acc gtt gtt gat gaa gtc aga aca gga act tat cgt caa tta     1421
   Glu Pro Thr Val Val Asp Glu Val Arg Thr Gly Thr Tyr Arg Gln Leu
                   75                  80                  85
   ttt cat cct gaa caa tta ata tca gga aaa gaa gat gct gcc aac aat     1469
   Phe His Pro Glu Gln Leu Ile Ser Gly Lys Glu Asp Ala Ala Asn Asn
               90                  95                  100
   ttt gct aga gga cac tat aca atc ggt aaa gaa gtt ata gat gta tgt     1517
   Phe Ala Arg Gly His Tyr Thr Ile Gly Lys Glu Val Ile Asp Val Cys
           105                 110                 115
   ttg gac aga att aga aaa tta gct gat aac tgt acc ggt tta caa gga     1565
   Leu Asp Arg Ile Arg Lys Leu Ala Asp Asn Cys Thr Gly Leu Gln Gly
       120                 125                 130
   ttt tta atg ttc agc gca gtt gga ggt gga aca ggt agt gga ttt ggt     1613
   Phe Leu Met Phe Ser Ala Val Gly Gly Gly Thr Gly Ser Gly Phe Gly
   135                 140                 145                 150
   tgt tta atg tta gaa aga tta tcc gtt gat tat gga aag aaa tcc aaa     1661
   Cys Leu Met Leu Glu Arg Leu Ser Val Asp Tyr Gly Lys Lys Ser Lys
                   155                 160                 165
   ctg aat ttt tgc tgt tgg cca tca cct caa gtt tca act gct gta gtt     1709
   Leu Asn Phe Cys Cys Trp Pro Ser Pro Gln Val Ser Thr Ala Val Val
               170                 175                 180
   gaa cca tac aat tca gtt ttg tct act cat tca tta tta gaa cat act      1757
   Glu Pro Tyr Asn Ser Val Leu Ser Thr His Ser Leu Leu Glu His Thr
           185                 190                 195
   gat gta gca ata atg ctt gat aac gaa gct ata tat gat ata tgc aga      1805
   Asp Val Ala Ile Met Leu Asp Asn Glu Ala Ile Tyr Asp Ile Cys Arg
       200                 205                 210
   aga aat tta gat att gaa aga cca aca tat act aat tta aat aga ttg      1853
   Arg Asn Leu Asp Ile Glu Arg Pro Thr Tyr Thr Asn Leu Asn Arg Leu
   215                 220                 225                 230
   att gct caa gtt att tct tcc tta aca gca tct tta aga ttt gat ggt      1901
   Ile Ala Gln Val Ile Ser Ser Leu Thr Ala Ser Leu Arg Phe Asp Gly
                   235                 240                 245
   gct tta aat gtt gat gta aca gaa ttc caa acc aac tta gta cca tac      1949
   Ala Leu Asn Val Asp Val Thr Glu Phe Gln Thr Asn Leu Val Pro Tyr
               250                 255                 260
   cct cgt att cat ttt atg tta tct tca tat gct cca gtt gtt agt gct      1997
   Pro Arg Ile His Phe Met Leu Ser Ser Tyr Ala Pro Val Val Ser Ala
           265                 270                 275
   gaa aaa gca tac cat gaa caa ttg tcc gtt tct gaa att acc aac tca      2045
   Glu Lys Ala Tyr His Glu Gln Leu Ser Val Ser Glu Ile Thr Asn Ser
       280                 285                 290
   gca ttc gaa cca gca aat atg atg gca aaa tgt gat ccg aga cat gga      2093
   Ala Phe Glu Pro Ala Asn Met Met Ala Lys Cys Asp Pro Arg His Gly
   295                 300                 305                 310
   aaa tat atg gct tgt tgt tta atg tat aga ggt gat gta gta cca aag      2141
   Lys Tyr Met Ala Cys Cys Leu Met Tyr Arg Gly Asp Val Val Pro Lys
                   315                 320                 325
   gat gtg aac gca gct gtt gct acc ata aaa aca aaa aga acc att caa      2189
   Asp Val Asn Ala Ala Val Ala Thr Ile Lys Thr Lys Arg Thr Ile Gln
               330                 335                 340
   ttt gtt gac tgg tgt cct act ggt ttt aaa tgt ggt ata aat tat caa      2237
   Phe Val Asp Trp Cys Pro Thr Gly Phe Lys Cys Gly Ile Asn Tyr Gln
           345                 350                 355
   cca cca act gtt gta cca gga gga gat tta gcc aaa gtt atg aga gct      2285
   Pro Pro Thr Val Val Pro Gly Gly Asp Leu Ala Lys Val Met Arg Ala
       360                 365                 370
   gtt tgt atg atc agc aac tca aca gca att gca gaa gta ttc tca aga      2333
   Val Cys Met Ile Ser Asn Ser Thr Ala Ile Ala Glu Val Phe Ser Arg
   375                 380                 385                 390
   atg gat caa aaa ttt gat tta atg tat gca aaa aga gct ttc gtt cat      2381
   Met Asp Gln Lys Phe Asp Leu Met Tyr Ala Lys Arg Ala Phe Val His
                   395                 400                 405
   tgg tat gta ggt gaa ggt atg gaa gaa gga gaa ttt agt gaa gct aga      2429
   Trp Tyr Val Gly Glu Gly Met Glu Glu Gly Glu Phe Ser Glu Ala Arg
               410                 415                 420
   gaa gat ttg gcc gcc tta gaa aaa gat tat gaa gag gta gga att gaa      2477
   Glu Asp Leu Ala Ala Leu Glu Lys Asp Tyr Glu Glu Val Gly Ile Glu
           425                 430                 435
   tcc aat gaa gca gaa gga gaa gat gaa gga tat gaa gca gat tat taa     2525
   Ser Asn Glu Ala Glu Gly Glu Asp Glu Gly Tyr Glu Ala Asp Tyr
       440                 445                 450
   atttgttaat atataaaagc attatatata tgtatatatt tatatattta tttatatatg   2585
   tgtgtatgta catacatcta tatatatata tatatatata tatatatata tatatatata   2645
   tat                                                                 2648
<210> 167
   <211> 453
   <212> PRT
   <213> P. falciparum alpha-1 tubulin
<400> 167
   Met Arg Glu Val Ile Ser Ile His Val Gly Gln Ala Gly Ile Gln Val
   1 5 10 15
   Gly Asn Ala Cys Trp Glu Leu Phe Cys Leu Glu His Gly Ile Gln Pro
   20 25 30
   Asp Gly Gln Met Pro Ser Asp Lys Ala Ser Arg Ala Asn Asp Asp Ala
   35 40 45
   Phe Asn Thr Phe Phe Ser Glu Thr Gly Ala Gly Lys His Val Pro Arg
   50 55 60
   Cys Val Phe Val Asp Leu Glu Pro Thr Val Val Asp Glu Val Arg Thr
   65 70 75 80
   Gly Thr Tyr Arg Gln Leu Phe His Pro Glu Gln Leu Ile Ser Gly Lys
   85 90 95
   Glu Asp Ala Ala Asn Asn Phe Ala Arg Gly His Tyr Thr Ile Gly Lys
   100 105 110
   Glu Val Ile Asp Val Cys Leu Asp Arg Ile Arg Lys Leu Ala Asp Asn
   115 120 125
   Cys Thr Gly Leu Gln Gly Phe Leu Met Phe Ser Ala Val Gly Gly Gly
   130 135 140
   Thr Gly Ser Gly Phe Gly Cys Leu Met Leu Glu Arg Leu Ser Val Asp
   145 150 155 160
   Tyr Gly Lys Lys Ser Lys Leu Asn Phe Cys Cys Trp Pro Ser Pro Gln
   165 170 175
   Val Ser Thr Ala Val Val Glu Pro Tyr Asn Ser Val Leu Ser Thr His
   180 185 190
   Ser Leu Leu Glu His Thr Asp Val Ala Ile Met Leu Asp Asn Glu Ala
   195 200 205
   Ile Tyr Asp Ile Cys Arg Arg Asn Leu Asp Ile Glu Arg Pro Thr Tyr
   210 215 220
   Thr Asn Leu Asn Arg Leu Ile Ala Gln Val Ile Ser Ser Leu Thr Ala
   225 230 235 240
   Ser Leu Arg Phe Asp Gly Ala Leu Asn Val Asp Val Thr Glu Phe Gln
   245 250 255
   Thr Asn Leu Val Pro Tyr Pro Arg Ile His Phe Met Leu Ser Ser Tyr
   260 265 270
   Ala Pro Val Val Ser Ala Glu Lys Ala Tyr His Glu Gln Leu Ser Val
   275 280 285
   Ser Glu Ile Thr Asn Ser Ala Phe Glu Pro Ala Asn Met Met Ala Lys
   290 295 300
   Cys Asp Pro Arg His Gly Lys Tyr Met Ala Cys Cys Leu Met Tyr Arg
   305 310 315 320
   Gly Asp Val Val Pro Lys Asp Val Asn Ala Ala Val Ala Thr Ile Lys
   325 330 335
   Thr Lys Arg Thr Ile Gln Phe Val Asp Trp Cys Pro Thr Gly Phe Lys
   340 345 350
   Cys Gly Ile Asn Tyr Gln Pro Pro Thr Val Val Pro Gly Gly Asp Leu
   355 360 365
   Ala Lys Val Met Arg Ala Val Cys Met Ile Ser Asn Ser Thr Ala Ile
   370 375 380
   Ala Glu Val Phe Ser Arg Met Asp Gln Lys Phe Asp Leu Met Tyr Ala
   385 390 395 400
   Lys Arg Ala Phe Val His Trp Tyr Val Gly Glu Gly Met Glu Glu Gly
   405 410 415
   Glu Phe Ser Glu Ala Arg Glu Asp Leu Ala Ala Leu Glu Lys Asp Tyr
   420 425 430
   Glu Glu Val Gly Ile Glu Ser Asn Glu Ala Glu Gly Glu Asp Glu Gly
   435 440 445
   Tyr Glu Ala Asp Tyr
   450
<210> 168
   <211> 47
   <212> DNA
   <213> synthetic oligonucleotide
<400> 168
   gatcctcgag gaattcatga gagaagtaat aagtatccat gtaggac 47
<210> 169
   <211> 43
   <212> DNA
   <213> synthetic oligonucleotide
<400> 169
   gatcctcgag ttaataatct gcttcatatc cttcatcttc tcc 43
<210> 170
   <211> 2833
   <212> DNA
   <213> P.falciparum beta-tubulin
<220>
   <221> exon
   <222> (654)..(749)
   <223>
<220>
   <221> exon
   <222> (1112)..(2064)
   <223>
<220>
   <221> exon
   <222> (2228)..(2516)
   <223>
<400> 170
   aattcctagt ttatttaatt taaaaattaa aagatcgaat gctcaacatt ttaaaaagaa      60
   atctgtgaaa catatcttaa caagaaatgg tgtaacaaaa gaaacaatat taaatgataa     120
   attaccaaag ataaatgatg aaattgacag aacatataat ggacacaaaa tggatgaaaa     180
   tttacaggat aaacaaaaaa ggaatcatgg agtaaatata aaattaataa atgaatatga     240
   aaatatcatg tgaagaataa attctcaaaa tcattgattg tatgacaaga ttcaagaatt     300
   ggttatataa aaatatattt aggaaaagta attttgggtc atatgtatca acatttacag     360
   gtgtatttgg aggtgctgca gctgttagct gtttctgcca taagtggagc ttgtataact     420
   aaatttagtg ttacattggt tccggtattt gcatgttttg ggggtgtctt tgcgattatt     480
   ataatattat taatattagg aacatggatg cttgttacat ggttatggca acacaaagaa     540
   gtagtatttt tttttttttt taatttttac ttaatatatc ctcttacaat ataaaatatt    600
   tatatattta aaaaaaaaag aaaaaatttt ctttgagatt attttattaa aga atg      656
                                                              Met
                                                              1
   aga gaa att gtt cat att caa gct ggc caa tgt gga aat caa ata ggt     704
   Arg Glu Ile Val His Ile Gln Ala Gly Gln Cys Gly Asn Gln Ile Gly
           5                   10                  15
   gca aag ttt tgg gaa gtc att tct gat gag cat gga ata gat cca         749
   Ala Lys Phe Trp Glu Val Ile Ser Asp Glu His Gly Ile Asp Pro
       20                  25                  30
   gtaagtttaa aaaaaaaata tatttattta tatgaatctg taaacatatg tatatttata   809
   tatatatata tatatatatg gaagaataat tttgtgtgta taatttgggg tccttcccct   869
   ttattgtatt ctataaatgc ctcctttata ttgataataa tttatatatg taaaccttta   929
   atgacgaggc ttatatataa aaaccttaga tattataaat aaatgtatat tatgtacata   989
   tgacgatatc gctctctcta tatatatata tatatatata tatatatatt tatttattta   1049
   tatatttatt tatttattta tttatttatt tttttttttt tttttatttt tatttttttt   1109
   ag agt ggt acc tat agt ggg gac agt gac tta cag tta gaa aga gtt      1156
      Ser Gly Thr Tyr Ser Gly Asp Ser Asp Leu Gln Leu Glu Arg Val
          35                  40                  45
   gac gtt ttt tac aac gaa gca aca gga ggt aga tat gtt cca aga gct     1204
   Asp Val Phe Tyr Asn Glu Ala Thr Gly Gly Arg Tyr Val Pro Arg Ala
           50                  55                  60
   ata ttg atg gac ttg gaa cct ggt act atg gat agt gtt cgt gct ggc     1252
   Ile Leu Met Asp Leu Glu Pro Gly Thr Met Asp Ser Val Arg Ala Gly
       65                  70                  75
   ccc ttt ggt caa tta ttt cgt cca gat aat ttt gtg ttt ggt caa aca     1300
   Pro Phe Gly Gln Leu Phe Arg Pro Asp Asn Phe Val Phe Gly Gln Thr
   80                  85                  90                  95
   ggt gca gga aat aat tgg gct aaa gga cat tat act gaa ggt gct gaa     1348
   Gly Ala Gly Asn Asn Trp Ala Lys Gly His Tyr Thr Glu Gly Ala Glu
                   100                 105                 110
   ttg ata gat gca gtt tta gat gtg ctt aga aaa gaa gca gaa ggt tgt     1396
   Leu Ile Asp Ala Val Leu Asp Val Leu Arg Lys Glu Ala Glu Gly Cys
               115                 120                 125
   gat tgt tta caa gga ttt cag att act cat tca tta ggt ggt ggt aca     1444
   Asp Cys Leu Gln Gly Phe Gln Ile Thr His Ser Leu Gly Gly Gly Thr
           130                 135                 140
   ggt agt ggt atg ggt act ttg ttg att agt aaa ata aga gag gag tat     1492
   Gly Ser Gly Met Gly Thr Leu Leu Ile Ser Lys Ile Arg Glu Glu Tyr
       145                 150                 155
   cct gat cgt att atg gaa aca ttt tct gta ttt cca tca cca aaa gtt     1540
   Pro Asp Arg Ile Met Glu Thr Phe Ser Val Phe Pro Ser Pro Lys Val
   160                 165                 170                 175
   tct gat act gtt gtt gaa cca tat aat gct aca tta tca gtc cat cag     1588
   Ser Asp Thr Val Val Glu Pro Tyr Asn Ala Thr Leu Ser Val His Gln
                       180                 185                 190
   ttg gtt gaa aat gct gat gaa gtt caa gtt atc gat aat gaa gct tta      1636
   Leu Val Glu Asn Ala Asp Glu Val Gln Val Ile Asp Asn Glu Ala Leu
                   195                 200                 205
   tat gac ata tgt ttt agg act ctt aaa tta aca aca cca aca tat gga      1684
   Tyr Asp Ile Cys Phe Arg Thr Leu Lys Leu Thr Thr Pro Thr Tyr Gly
               210                 215                 220
   gat tta aat cac ctt gta tca gct gca atg tca ggt gta acc tgt tcg      1732
   Asp Leu Asn His Leu Val Ser Ala Ala Met Ser Gly Val Thr Cys Ser
       225                 230                 235
   tta aga ttt cct ggt caa ctt aac agt gac tta aga aaa tta gct gtt      1780
   Leu Arg Phe Pro Gly Gln Leu Asn Ser Asp Leu Arg Lys Leu Ala Val
   240                 245                 250                 255
   aat ttg atc cca ttc cca cgt tta cat ttc ttt atg tac ggg ttt gct      1828
   Asn Leu Ile Pro Phe Pro Arg Leu His Phe Phe Met Tyr Gly Phe Ala
                   260                 265                 270
   cct tta act agt aga ggc agt caa caa tac aga gcc tta act gtg ccg      1876
   Pro Leu Thr Ser Arg Gly Ser Gln Gln Tyr Arg Ala Leu Thr Val Pro
               275                 280                 285
   gag tta aca caa caa atg ttc gac gca aaa aat atg atg tgc aca agt      1924
   Glu Leu Thr Gln Gln Met Phe Asp Ala Lys Asn Met Met Cys Thr Ser
               290                 295                 300
   gat cca aga cat gga aga tat tta acg gca tgt gct atg ttt aga gga      1972
   Asp Pro Arg His Gly Arg Tyr Leu Thr Ala Cys Ala Met Phe Arg Gly
       305                 310                 315
   aga atg tcc aca aag gaa gtt gac gaa caa atg tta aac gtt caa aat      2020
   Arg Met Ser Thr Lys Glu Val Asp Glu Gln Met Leu Asn Val Gln Asn
   320                 325                 330                 335
   aaa aac tca tct tat ttt gtc gaa tgg att cct cac aac aca aa           2064
   Lys Asn Ser Ser Tyr Phe Val Glu Trp Ile Pro His Asn Thr Lys
                   340                 345
   gtaagaagga acaattgata ctagtatgca tgtttttttg tttatatgta tttatatata   2124
   tatatatata tatgtattca tttatatatt ttgaaatata cattttacat ataaattttt   2184
   tttttttctt tttctttttt tttttttttg tttttttctt tag a tca agt gtt tgt    2240
                                                     Ser Ser Val Cys
   gat att cca cca ttg gga tta aaa atg gct gtt act ttt gta gga aac      2288
   Asp Ile Pro Pro Leu Gly Leu Lys Met Ala Val Thr Phe Val Gly Asn
   355                 360                 365                 370
   tca acc gcc att caa gaa atg ttt aaa aga gtt tct gat caa ttt act      2336
   Ser Thr Ala Ile Gln Glu Met Phe Lys Arg Val Ser Asp Gln Phe Thr
                   375                 380                 385
   gct atg ttt aga aga aaa gcc ttt ttg cac tgg tac acc gga gaa ggt      2384
   Ala Met Phe Arg Arg Lys Ala Phe Leu His Trp Tyr Thr Gly Glu Gly
               390                 395                 400
   atg gac gag atg gaa ttt aca gaa gct gaa tca aat atg aat gat tta      2432
   Met Asp Glu Met Glu Phe Thr Glu Ala Glu Ser Asn Met Asn Asp Leu
           405                 410                 415
   gtt tca gaa tat caa caa tat caa gat gct aca gca gaa gag gaa gga     2480
   Val Ser Glu Tyr Gln Gln Tyr Gln Asp Ala Thr Ala Glu Glu Glu Gly
       420                 425                 430
   gaa ttt gaa gaa gaa gaa gga gac gta gaa gcc taa atctatttat          2526
   Glu Phe Glu Glu Glu Glu Gly Asp Val Glu Ala
   435                 440                 445
   atttatgaaa atatatacat attatatata tatgtatatg taattaacaa gaataaaaaa   2586
   taaaaaataa aaaaaaaata aaataaaaaa ataaaaatac ataataaaaa agtataaaat   2646
   aaatatctaa tcattaatta tatataacaa tataatttaa ctcttttttt ttttattatt   2706
   attgaagtta tgttcgggta tatataacat atatataaat tatatatatg ttgcagtttc   2766
   tttttttttt tttttttttt tttttcttat catttgattt tacactcaca tatatatgac   2826
   atatata                                                             2833
<210> 171
   <211> 445
   <212> PRT
   <213> P.falciparum beta-tubulin
<400> 171
   Met Arg Glu Ile Val His Ile Gln Ala Gly Gln Cys Gly Asn Gln Ile
   1               5                   10                  15
   Gly Ala Lys Phe Trp Glu Val Ile Ser Asp Glu His Gly Ile Asp Pro
               20                  25                  30
   Ser Gly Thr Tyr Ser Gly Asp Ser Asp Leu Gln Leu Glu Arg Val Asp
           35                  40                  45
   Val Phe Tyr Asn Glu Ala Thr Gly Gly Arg Tyr Val Pro Arg Ala Ile
       50                  55                  60
   Leu Met Asp Leu Glu Pro Gly Thr Met Asp Ser Val Arg Ala Gly Pro
   65                  70                  75                  80
   Phe Gly Gln Leu Phe Arg Pro Asp Asn Phe Val Phe Gly Gln Thr Gly
                   85                  90                  95
   Ala Gly Asn Asn Trp Ala Lys Gly His Tyr Thr Glu Gly Ala Glu Leu
               100                 105                 110
   Ile Asp Ala Val Leu Asp Val Leu Arg Lys Glu Ala Glu Gly Cys Asp
           115                 120                 125
   Cys Leu Gln Gly Phe Gln Ile Thr His Ser Leu Gly Gly Gly Thr Gly
       130                 135                 140
   Ser Gly Met Gly Thr Leu Leu Ile Ser Lys Ile Arg Glu Glu Tyr Pro
   145 150 155 160
   Asp Arg Ile Met Glu Thr Phe Ser Val Phe Pro Ser Pro Lys Val Ser
   165 170 175
   Asp Thr Val Val Glu Pro Tyr Asn Ala Thr Leu Ser Val His Gln Leu
   180 185 190
   Val Glu Asn Ala Asp Glu Val Gln Val Ile Asp Asn Glu Ala Leu Tyr
   195 200 205
   Asp Ile Cys Phe Arg Thr Leu Lys Leu Thr Thr Pro Thr Tyr Gly Asp
   210 215 220
   Leu Asn His Leu Val Ser Ala Ala Met Ser Gly Val Thr Cys Ser Leu
   225 230 235 240
   Arg Phe Pro Gly Gln Leu Asn Ser Asp Leu Arg Lys Leu Ala Val Asn
   245 250 255
   Leu Ile Pro Phe Pro Arg Leu His Phe Phe Met Tyr Gly Phe Ala Pro
   260 265 270
   Leu Thr Ser Arg Gly Ser Gln Gln Tyr Arg Ala Leu Thr Val Pro Glu
   275 280 285
   Leu Thr Gln Gln Met Phe Asp Ala Lys Asn Met Met Cys Thr Ser Asp
   290 295 300
   Pro Arg His Gly Arg Tyr Leu Thr Ala Cys Ala Met Phe Arg Gly Arg
   305 310 315 320
   Met Ser Thr Lys Glu Val Asp Glu Gln Met Leu Asn Val Gln Asn Lys
   325 330 335
   Asn Ser Ser Tyr Phe Val Glu Trp Ile Pro His Asn Thr Lys Ser Ser
   340 345 350
   Val Cys Asp Ile Pro Pro Leu Gly Leu Lys Met Ala Val Thr Phe Val
   355 360 365
   Gly Asn Ser Thr Ala Ile Gln Glu Met Phe Lys Arg Val Ser Asp Gln
   370 375 380
   Phe Thr Ala Met Phe Arg Arg Lys Ala Phe Leu His Trp Tyr Thr Gly
   385 390 395 400
   Glu Gly Met Asp Glu Met Glu Phe Thr Glu Ala Glu Ser Asn Met Asn
   405 410 415
   Asp Leu Val Ser Glu Tyr Gln Gln Tyr Gln Asp Ala Thr Ala Glu Glu
   420 425 430
   Glu Gly Glu Phe Glu Glu Glu Glu Gly Asp Val Glu Ala
   435 440 445
<210> 172
   <211> 39
   <212> DNA
   <213> synthetic oligonucleotide
<400> 172
   gatcgaattc atgagagaaa ttgttcatat tcaagctgg 39
<210> 173
   <211> 43
   <212> DNA
   <213> synthetic oligonucleotide
<400> 173
   gatcctcgag ttaataatct gcttcatatc cttcatcttc tcc 43
<210> 174
   <211> 1827
   <212> DNA
   <213> C. parvum alpha tubulin
<220>
   <221> exon
   <222> (1)..(1365)
   <223>
<400> 174
   atg aga gaa gtt att tca att cat gtt ggg cag gca gga ata caa att       48
   Met Arg Glu Val Ile Ser Ile His Val Gly Gln Ala Gly Ile Gln Ile
   1               5                   10                  15
   ggt aat gct tgt tgg gaa tta ttt tgc ctt gaa cat gga ata aat cca       96
   Gly Asn Ala Cys Trp Glu Leu Phe Cys Leu Glu His Gly Ile Asn Pro
               20                  25                  30
   gat gga aca atg ccc atg tcc gag caa aat atg ggt att tca gat gat      144
   Asp Gly Thr Met Pro Met Ser Glu Gln Asn Met Gly Ile Ser Asp Asp
           35                  40                  45
   gca ttc aat acg ttc ttc agc gag act gga gct gga aag cat gtg ccc      192
   Ala Phe Asn Thr Phe Phe Ser Glu Thr Gly Ala Gly Lys His Val Pro
       50                  55                  60
   aga gca gtt ttt gta gat ctg gag cca act gtt gtt gac gaa att cgt      240
   Arg Ala Val Phe Val Asp Leu Glu Pro Thr Val Val Asp Glu Ile Arg
   65                  70                  75                  80
   agt ggg aca tat agg caa ctg ttt cat cca gag cag ctt att aat ggt      288
   Ser Gly Thr Tyr Arg Gln Leu Phe His Pro Glu Gln Leu Ile Asn Gly
                   85                  90                  95
   aag gaa gat gcg gct aac aac ttt gca agg ggc cat tat aca gta ggg      336
   Lys Glu Asp Ala Ala Asn Asn Phe Ala Arg Gly His Tyr Thr Val Gly
               100                 105                 110
   aaa gag ata ttg gaa gta tgt ttg gat cga att cgt aaa ctt gct gac      384
   Lys Glu Ile Leu Glu Val Cys Leu Asp Arg Ile Arg Lys Leu Ala Asp
           115                 120                 125
   aat tgt aca gga tta caa ggt ttt ctt atg ttt aat gca gtt gga ggt      432
   Asn Cys Thr Gly Leu Gln Gly Phe Leu Met Phe Asn Ala Val Gly Gly
       130                 135                 140
   gga aca gga gct ggc ctc ggg aca tta tta cta gaa agg ctt tct gtt      480
   Gly Thr Gly Ala Gly Leu Gly Thr Leu Leu Leu Glu Arg Leu Ser Val
   145                 150                 155                 160
   gac tat ggc aaa aaa tct aaa ttg aat ttc tgt aca tgg cca tct ccg      528
   Asp Tyr Gly Lys Lys Ser Lys Leu Asn Phe Cys Thr Trp Pro Ser Pro
                   165                 170                 175
   cag ctt agc act gca gtg gta gag cca tac aac gcc gtc ctt tcc act      576
   Gln Leu Ser Thr Ala Val Val Glu Pro Tyr Asn Ala Val Leu Ser Thr
               180                 185                 190
   cac tct ctc ctt gaa cat gca gat gtt gct gtt atg cta gat aat gaa      624
   His Ser Leu Leu Glu His Ala Asp Val Ala Val Met Leu Asp Asn Glu
           195                 200                 205
   gca ata tat gat atc tgt aga cga aat ttg aac att gag cag cct gct      672
   Ala Ile Tyr Asp Ile Cys Arg Arg Asn Leu Asn Ile Glu Gln Pro Ala
       210                 215                 220
   tat acg aac cta aat aga tta att gcg caa gta ata tcc tcc ttg act      720
   Tyr Thr Asn Leu Asn Arg Leu Ile Ala Gln Val Ile Ser Ser Leu Thr
   225                 230                 235                 240
   gca tct ttg cgt ttc gat ggt gca ttg aat gtc gat atc acc gag ttt      768
   Ala Ser Leu Arg Phe Asp Gly Ala Leu Asn Val Asp Ile Thr Glu Phe
                   245                 250                 255
   caa acc aat ttg gtt cct tac cca agg atc cac ttt atg ctt tca tca      816
   Gln Thr Asn Leu Val Pro Tyr Pro Arg Ile His Phe Met Leu Ser Ser
               260                 265                 270
   tat gct cca att att tcc gct gaa aaa gct ttt cac gaa caa tta agt      864
   Tyr Ala Pro Ile Ile Ser Ala Glu Lys Ala Phe His Glu Gln Leu Ser
           275                 280                 285
   gtt gcc gag att act aat gca gtt ttt gaa cct caa aat caa atg gct      912
   Val Ala Glu Ile Thr Asn Ala Val Phe Glu Pro Gln Asn Gln Met Ala
       290                 295                 300
   aag tgt gac cct aga cac ggt aaa tat atg gca tgc tgc ttg atg tac      960
   Lys Cys Asp Pro Arg His Gly Lys Tyr Met Ala Cys Cys Leu Met Tyr
   305                 310                 315                 320
   aga ggt gat gtt gtt cca aaa gat aca aat gca gca gtt gca aca att     1008
   Arg Gly Asp Val Val Pro Lys Asp Thr Asn Ala Ala Val Ala Thr Ile
                   325                 330                 335
   aaa act aaa aga act att cag ttt gtt gat tgg tgc cca aca ggc ttc     1056
   Lys Thr Lys Arg Thr Ile Gln Phe Val Asp Trp Cys Pro Thr Gly Phe
               340                 345                 350
   aaa tgt gga att aat tat caa ccg ccg act gtt gtg cct ggc ggg gat      1104
   Lys Cys Gly Ile Asn Tyr Gln Pro Pro Thr Val Val Pro Gly Gly Asp
           355                 360                 365
   ctc gct aag gtt atg cgt gca tgt tgt atg att tca aac tca act gca      1152
   Leu Ala Lys Val Met Arg Ala Cys Cys Met Ile Ser Asn Ser Thr Ala
       370                 375                 380
   ata gct gag gtc ttc aat agg atg gat cat aaa ttt gat tta atg tat      1200
   Ile Ala Glu Val Phe Asn Arg Met Asp His Lys Phe Asp Leu Met Tyr
   385                 390                 395                 400
   tca aaa agg gca ttc gtg cat tgg tat gtc ggc gag ggc atg gaa gaa      1248
   Ser Lys Arg Ala Phe Val His Trp Tyr Val Gly Glu Gly Met Glu Glu
                   405                 410                 415
   ggt gaa ttt agc gag gca aga gaa gac tta gct gca tta gag aaa gac      1296
   Gly Glu Phe Ser Glu Ala Arg Glu Asp Leu Ala Ala Leu Glu Lys Asp
               420                 425                 430
   tat gaa gag gtt gga att gaa att gca gat gga gaa gat gaa gaa gtt      1344
   Tyr Glu Glu Val Gly Ile Glu Ile Ala Asp Gly Glu Asp Glu Glu Val
           435                 440                 445
   cat tac gag ggc gat ttc tag ttatccttgt tcattgaatt ctctagattt         1395
   His Tyr Glu Gly Asp Phe
       450
   aggaggtttg gtttaccgcc cggcgggctc gtgtagatta gtatcttaat ctatggaatt    1455
   ttccctattt agttatagtc aaaagcggat ccttgctgca ggtgtgttgt ctttattatc    1515
   cggatatgtg cttggtgtga ctaacatacc gagggctgtt ttgctcaatt ttttgtcctt    1575
   ttcttggatg aaatggtcta tgcatgtccg tggctatcaa attaccaata gttgtataaa    1635
   ttttgagagt tttatcatgg ttctcatcaa ttcctcactt ttacttggtg cttccatagg    1695
   ctctattgct tctgtgtttg tggaaagata ttttggtaag gttaaaggca ttctctctgc    1755
   atattttttg tttggtattg gttctgtatt ttgtttgaac aaggatttag taattttctt   1815
   aattggaagg gt                                                        1827
<210> 175
   <211> 454
   <212> PRT
   <213> C. parvum alpha tubulin
<400> 175
   Met Arg Glu Val Ile Ser Ile His Val Gly Gln Ala Gly Ile Gln Ile
   1 5 10 15
   Gly Asn Ala Cys Trp Glu Leu Phe Cys Leu Glu His Gly Ile Asn Pro
   20 25 30
   Asp Gly Thr Met Pro Met Ser Glu Gln Asn Met Gly Ile Ser Asp Asp
   35 40 45
   Ala Phe Asn Thr Phe Phe Ser Glu Thr Gly Ala Gly Lys His Val Pro
   50 55 60
   Arg Ala Val Phe Val Asp Leu Glu Pro Thr Val Val Asp Glu Ile Arg
   65 70 75 80
   Ser Gly Thr Tyr Arg Gln Leu Phe His Pro Glu Gln Leu Ile Asn Gly
   85 90 95
   Lys Glu Asp Ala Ala Asn Asn Phe Ala Arg Gly His Tyr Thr Val Gly
   100 105 110
   Lys Glu Ile Leu Glu Val Cys Leu Asp Arg Ile Arg Lys Leu Ala Asp
   115 120 125
   Asn Cys Thr Gly Leu Gln Gly Phe Leu Met Phe Asn Ala Val Gly Gly
   130 135 140
   Gly Thr Gly Ala Gly Leu Gly Thr Leu Leu Leu Glu Arg Leu Ser Val
   145 150 155 160
   Asp Tyr Gly Lys Lys Ser Lys Leu Asn Phe Cys Thr Trp Pro Ser Pro
   165 170 175
   Gln Leu Ser Thr Ala Val Val Glu Pro Tyr Asn Ala Val Leu Ser Thr
   180 185 190
   His Ser Leu Leu Glu His Ala Asp Val Ala Val Met Leu Asp Asn Glu
   195 200 205
   Ala Ile Tyr Asp Ile Cys Arg Arg Asn Leu Asn Ile Glu Gln Pro Ala
   210 215 220
   Tyr Thr Asn Leu Asn Arg Leu Ile Ala Gln Val Ile Ser Ser Leu Thr
   225 230 235 240
   Ala Ser Leu Arg Phe Asp Gly Ala Leu Asn Val Asp Ile Thr Glu Phe
   245 250 255
   Gln Thr Asn Leu Val Pro Tyr Pro Arg Ile His Phe Met Leu Ser Ser
   260 265 270
   Tyr Ala Pro Ile Ile Ser Ala Glu Lys Ala Phe His Glu Gln Leu Ser
   275 280 285
   Val Ala Glu Ile Thr Asn Ala Val Phe Glu Pro Gln Asn Gln Met Ala
   290                 295                 300
   Lys Cys Asp Pro Arg His Gly Lys Tyr Met Ala Cys Cys Leu Met Tyr
   305                 310                 315                 320
   Arg Gly Asp Val Val Pro Lys Asp Thr Asn Ala Ala Val Ala Thr Ile
                   325                 330                 335
   Lys Thr Lys Arg Thr Ile Gln Phe Val Asp Trp Cys Pro Thr Gly Phe
               340                 345                 350
   Lys Cys Gly Ile Asn Tyr Gln Pro Pro Thr Val Val Pro Gly Gly Asp
           355                 360                 365
   Leu Ala Lys Val Met Arg Ala Cys Cys Met Ile Ser Asn Ser Thr Ala
       370                 375                 380
   Ile Ala Glu Val Phe Asn Arg Met Asp His Lys Phe Asp Leu Met Tyr
   385                 390                 395                 400
   Ser Lys Arg Ala Phe Val His Trp Tyr Val Gly Glu Gly Met Glu Glu
                   405                 410                 415
   Gly Glu Phe Ser Glu Ala Arg Glu Asp Leu Ala Ala Leu Glu Lys Asp
               420                 425                 430
   Tyr Glu Glu Val Gly Ile Glu Ile Ala Asp Gly Glu Asp Glu Glu Val
           435                 440                 445
   His Tyr Glu Gly Asp Phe
       450
<210> 176
   <211> 47
   <212> DNA
   <213> synthetic oligonucleotide
<400> 176
   gatcctcgag gaattcatga gagaagttat ttcaattcat gttgggc 47
<210> 177
   <211> 34
   <212> DNA
   <213> synthetic oligonucleotide
<400> 177
   gatcctcgag ctagaaatcg ccctcgtaat gaac 34
<210> 178
   <211> 1618
   <212> DNA
   <213> C.parvum beta tubulin
<220>
   <221> exon
   <222> (121)..(126)
   <223>
<220>
   <221> exon
   <222> (217)..(301)
   <223>
<220>
   <221> exon
   <222> (302)..(1546)
   <223>
<400> 178
   tgggtaatct tctggctatt ggggaacttg aattatagat tataataagt tgttgattac      60
   tataatttgt aggatattca tattaattga ttcctaatta ccattaataa gaaaaagaga     120
   atg aga gaaattgttc atgttcaagg aggacaatgt gggaaccaga ttggtgctaa        176
   Met Arg
   1
   attctgggaa gtcatttctg atgagcacgg gatcgaccct gta agt ttt gaa aat       231
                                               Val Ser Phe Glu Asn
                                                           5
   aat tga aat aat ttg aac tga ata att gac ttt ttt ttt ttc ttt ttt       279
   Asn     Asn Asn Leu Asn     Ile Ile Asp Phe Phe Phe Phe Phe Phe
               10                      15                  20
   ttt ctt tga aat atg aat tta g ac  tgg tac tgg tac tta tca tgg aga     327
   Phe Leu     Asn Met Asn Leu   Asp Trp Tyr Trp Tyr Leu Ser Trp Arg
                   25                    30                  35
   atc aga ttt aca gat gga acg tat taa tgt ttt cta caa tga agc ttc       375
   Ile Arg Phe Thr Asp Gly Thr Tyr     Cys Phe Leu Gln     Ser Phe
               40                      45                      50
   ggg tgg aag ata cgt tcc aag agc gat ttt ggt aga tct tga gcc agg       423
   Gly Trp Lys Ile Arg Ser Lys Ser Asp Phe Gly Arg Ser     Ala Arg
                   55                  60                      65
   aac aat gga ttc agt tag agc tgg tcc ttt tgg atc att att tag acc       471
   Asn Asn Gly Phe Ser     Ser Trp Ser Phe Trp Ile Ile Ile     Thr
                   70                      75
   tga taa ctt cat ttt tgg tca aag tgg agc tgg taa tat ttg ggc caa       519
           Leu His Phe Trp Ser Lys Trp Ser Trp     Tyr Leu Gly Gln
           80                  85                      90
   agg tca tta tac aga ggg agc tga act ctt aga tgc tgt aat gga tgt       567
   Arg Ser Leu Tyr Arg Gly Ser     Thr Leu Arg Cys Cys Asn Gly Cys
           95                      100                 105
   agt tag aca tga ggc aga atc ttg tga ttg ctt aca ggg ttt cca aat       615
   Ser     Thr     Gly Arg Ile Leu     Leu Leu Thr Gly Phe Pro Asn
                   110                     115                 120
   cac tca ctc ttt ggg agg tgg tac agg atc agg tat ggg tac ttt gtt       663
   His Ser Leu Phe Gly Arg Trp Tyr Arg Ile Arg Tyr Gly Tyr Phe Val
                   125                 130                 135
   ggt tgg aaa gat tca tga aga att ccc aga tcg tat ttt gca gac ctt      711
   Gly Trp Lys Asp Ser     Arg Ile Pro Arg Ser Tyr Phe Ala Asp Leu
               140                 145                 150
   ttc agt att ccc atc tcc aaa ggt atc aga tac tgt agt aga gcc cta      759
   Phe Ser Ile Pro Ile Ser Lys Gly Ile Arg Tyr Cys Ser Arg Ala Leu
               155                 160                 165
   caa tgc aac ctt atc aat tca cca gtt ggt tga aaa ttc gga tgc tgt      807
   Gln Cys Asn Leu Ile Asn Ser Pro Val Gly     Lys Phe Gly Cys Cys
           170                 175                 180
   tca agt aat cga taa tga agc ctt gta tga tat ttg ctt tag aac att      855
   Ser Ser Asn Arg         Ser Leu Val     Tyr Leu Leu     Asn Ile
           185                                 190
   gaa act aac aaa tcc atc tta tgg aga ttt gaa tca ctt ggt ttc agt      903
   Glu Thr Asn Lys Ser Ile Leu Trp Arg Phe Glu Ser Leu Gly Phe Ser
   195                 200                 205                 210
   tgc tat gtc tgg cgt aac ttg ttg cct acg ttt ccc agg tca act aaa      951
   Cys Tyr Val Trp Arg Asn Leu Leu Pro Thr Phe Pro Arg Ser Thr Lys
                   215                 220                 225
   cag tga ctt aag aaa gat gtc agt taa ctt ggt tcc ttt ccc aag att      999
   Gln     Leu Lys Lys Asp Val Ser     Leu Gly Ser Phe Pro Lys Ile
                   230                 235                 240
   aca ctt ctt cat gat tgg gtt tgc acc tct aac atc aag agg ttc tca     1047
   Thr Leu Leu His Asp Trp Val Cys Thr Ser Asn Ile Lys Arg Phe Ser
                   245                 250                 255
   aca ata cag atc ttt atc agt tgc aga gct tac cca aca aat gtt tga     1095
   Thr Ile Gln Ile Phe Ile Ser Cys Arg Ala Tyr Pro Thr Asn Val
               260                 265                 270
   tgc aaa gaa tat gat gtg tgc ttc aga ccc tcg aca tgg aag ata tct     1143
   Cys Lys Glu Tyr Asp Val Cys Phe Arg Pro Ser Thr Trp Lys Ile Ser
               275                 280                 285
   cac agc tag tgt aat gtt cag agg aag aat gtc aac aaa gga agt tga     1191
   His Ser     Cys Asn Val Gln Arg Lys Asn Val Asn Lys Gly Ser
               290                 295                 300
   tga aca gat gct ttt ggt tca aaa cag aaa ctc atc tta ttt tgt tga     1239
       Thr Asp Ala Phe Gly Ser Lys Gln Lys Leu Ile Leu Phe Cys
                   305                 310                 315
   atg gat tcc aaa taa tat caa gtc atc agt ttg tga tat tcc tcc aaa     1287
   Met Asp Ser Lys     Tyr Gln Val Ile Ser Leu     Tyr Ser Ser Lys
                       320                 325
   agg att gaa gat ggc ttc aac ctt tgt tgg aaa ctc tac tgc cat cca     1335
   Arg Ile Glu Asp Gly Phe Asn Leu Cys Trp Lys Leu Tyr Cys His Pro
   330                 335                 340                 345
   gga aat gtt cag acg tgt tgc tga gca att cac ttc cat gtt cag gag     1383
   Gly Asn Val Gln Thr Cys Cys     Ala Ile His Phe His Val Gln Glu
                   350                 355                 360
   aaa ggc ctt ctt gca ctg gat atc tgg aga ggg gat gga tga gat gga     1431
   Lys Gly Leu Leu Ala Leu Asp Ile Trp Arg Gly Asp Gly     Asp Gly
                       365                 370                     375
   att cac tga agc tga gac gaa tat gaa cga ttt agt ttc tga ata tca      1479
   Ile His     Ser     Asp Glu Tyr Glu Arg Phe Ser Phe     Ile Ser
                           380                 385
   aca ata tca aga tgt tcc agt tga aga aga tga ata ccc aga tga cga      1527
   Thr Ile Ser Arg Cys Ser Ser     Arg Arg     Ile Pro Arg     Arg
       390                 395                         400
   aca tca tat tga ggc tta a acttcttgga cataaatcaa accattggga           1576
   Thr Ser Tyr     Gly Leu
                   405
   agctatcctc atggggtggc tatagtcgat ttcatcattt tt                       1618
<210> 179
   <211> 446
   <212> PRT
   <213> C.parvum beta tubulin
<400> 179
   Met Arg Glu Ile Val His Val Gln Gly Gly Gln Cys Gly Asn Gln Ile
   1               5                   10                  15
   Gly Ala Lys Phe Trp Glu Val Ile Ser Asp Glu His Gly Ile Asp Pro
               20                  25                  30
   Thr Gly Thr Gly Thr Tyr His Gly Glu Ser Asp Leu Gln Met Glu Arg
           35                  40                  45
   Ile Asn Val Phe Tyr Asn Glu Ala Ser Gly Gly Arg Tyr Val Pro Arg
       50                  55                  60
   Ala Ile Leu Val Asp Leu Glu Pro Gly Thr Met Asp Ser Val Arg Ala
   65                  70                  75                  80
   Gly Pro Phe Gly Ser Leu Phe Arg Pro Asp Asn Phe Ile Phe Gly Gln
                   85                  90                  95
   Ser Gly Ala Gly Asn Ile Trp Ala Lys Gly His Tyr Thr Glu Gly Ala
               100                 105                 110
   Glu Leu Leu Asp Ala Val Met Asp Val Val Arg His Glu Ala Glu Ser
           115                 120                 125
   Cys Asp Cys Leu Gln Gly Phe Gln Ile Thr His Ser Leu Gly Gly Gly
       130                 135                 140
   Thr Gly Ser Gly Met Gly Thr Leu Leu Val Gly Lys Ile His Glu Glu
   145                 150                 155                 160
   Phe Pro Asp Arg Ile Leu Gln Thr Phe Ser Val Phe Pro Ser Pro Lys
                   165                 170                 175
   Val Ser Asp Thr Val Val Glu Pro Tyr Asn Ala Thr Leu Ser Ile His
               180                 185                 190
   Gln Leu Val Glu Asn Ser Asp Ala Val Gln Val Ile Asp Asn Glu Ala
           195                 200                 205
   Leu Tyr Asp Ile Cys Phe Arg Thr Leu Lys Leu Thr Asn Pro Ser Tyr
       210                 215                 220
   Gly Asp Leu Asn His Leu Val Ser Val Ala Met Ser Gly Val Thr Cys
   225                 230                 235                 240
   Cys Leu Arg Phe Pro Gly Gln Leu Asn Ser Asp Leu Arg Lys Met Ser
                   245                 250                 255
   Val Asn Leu Val Pro Phe Pro Arg Leu His Phe Phe Met Ile Gly Phe
               260                 265                 270
   Ala Pro Leu Thr Ser Arg Gly Ser Gln Gln Tyr Arg Ser Leu Ser Val
           275                 280                 285
   Ala Glu Leu Thr Gln Gln Met Phe Asp Ala Lys Asn Met Met Cys Ala
       290                 295                 300
   Ser Asp Pro Arg His Gly Arg Tyr Leu Thr Ala Ser Val Met Phe Arg
   305                 310                 315                 320
   Gly Arg Met Ser Thr Lys Glu Val Asp Glu Gln Met Leu Leu Val Gln
                   325                 330                 335
   Asn Arg Asn Ser Ser Tyr Phe Val Glu Trp Ile Pro Asn Asn Ile Lys
               340                 345                 350
   Ser Ser Val Cys Asp Ile Pro Pro Lys Gly Leu Lys Met Ala Ser Thr
           355                 360                 365
   Phe Val Gly Asn Ser Thr Ala Ile Gln Glu Met Phe Arg Arg Val Ala
       370                 375                 380
   Glu Gln Phe Thr Ser Met Phe Arg Arg Lys Ala Phe Leu His Trp Ile
   385                 390                 395                 400
   Ser Gly Glu Gly Met Asp Glu Met Glu Phe Thr Glu Ala Glu Thr Asn
                   405                 410                 415
   Met Asn Asp Leu Val Ser Glu Tyr Gln Gln Tyr Gln Asp Val Pro Val
   420 425 430
   Glu Glu Asp Glu Tyr Pro Asp Asp Glu His His Ile Glu Ala
   435 440 445
<210> 180
   <211> 41
   <212> DNA
   <213> synthetic oligonucleotide
<400> 180
   gatccaattg atgagagaaa ttgttcatgt tcaaggagga c 41
<210> 181
   <211> 41
   <212> DNA
   <213> synthetic oligonucleotide
<400> 181
   gatcctcgag ttaagcctca atatgatgtt cgtcatctgg g 41
<210> 182
   <211> 3646
   <212> DNA
   <213> T. brucei alpha tubulin
<220>
   <221> exon
   <222> (1961)..(3316)
   <223>
<400> 182
   atgcgcgaaa tcgtctgcgt tcaggctggc caatgcggta accagatcgg ctcaaagttc 60
   tgggaggtga tcagtgacga gcacggtgtg gaccccacag gtacctacca gggtgactct 120
   gacctgcagc tggagcgcat caatgtgtac tttgatgagg caacgggagg tcgctatgtg 180
   ccccgctccg tgctgattga tctggagcca ggtacaatgg actccgtacg tgctggcccc 240
   tatggtcaga tcttccgccc cgacaacttc atctttggac agtctggcgc cggcaacaac 300
   tgggcaaagg gccactacac ggagggtgcg gaactgatcg actctgtgct cgatgtgtgc 360
   tgcaaggagg cggagagctg tgactgcctc caaggcttcc agatctgcca ctcccttggt 420
   ggtggtactg gctccggcat gggtacgctg ctcatctcga agcttcgcga gcagtaccct 480
   gaccgtatca tgatgacttt ctccatcatc ccatccccca aggtgtccga cactgtcgtc 540
   gagccgtaca atacgactct ctccgtgcac caacttgtgg aaaactccga tgagtcgatg 600
   tgcattgaca acgaggcact gtacgatatt tgcttccgca ccctgaaact gacaacacca 660
   acgttcggtg acctgaacca cttggtgtct gctgttgtgt ccggcgtcac ctgctgcctg 720
   cgcttccctg gtcagttgaa ctctgacctc cgtaagttgg ctgtgaacct tgtcccattc 780
   ccgcgtctgc acttcttcat gatgggcttc gccccgctga ccagccgcgg ctcgcagcag 840
   taccgcggtc tctccgtgcc cgagctaacg cagcagatgt tcgatgcgaa aaacatgatg     900
   caagctgcag atcctcgtca cggccgctac ctgacagcgt ctgcactctt ccgcggccgc     960
   atgtcgacga aggaggttga tgagcagatg ctgaacgtgc agaacaagaa ctcgtcctac    1020
   ttcattgagt ggatcccgaa caacatcaag tcctctgttt gcgatatccc acccaaggga    1080
   ctcaagatgg ctgtcacctt cattggcaac aacacctgca tccaggagat gttccgccgt    1140
   gtgggagagc agttcaccct catgttccgt cgcaaggcgt tcttgcactg gtacactggc    1200
   gagggtatgg acgagatgga attcacggag gcagagtcca acatgaacga tctcgtgtct    1260
   gagtaccagc agtaccagga tgccacgatt gaggaggagg gcgagttcga cgaggaggag    1320
   caatactaga cgcggacggg gcatttcccg ttcgtcatta gcagtaggta atgaagatgt    1380
   ttgtttctcg tcccctttct ccttcgtcct tctgtcattt tgttcttttg tgtttatgtt    1440
   ttgttgttgt tttctttaat tttttttttc ttccacgttt gtgtacatcc gcgcgccact    1500
   ctattcagag agccacggat agtagaggag gtgggaaggg tatatgaggg acacgcgtac    1560
   catgatgtgg gatgtattgg ggtccctgtc tgtccttacg tgactatgta tgaaccgtca    1620
   cgtgtaagat gagctagtga gatcaacagt acaactcatc aacacgcctt cttctcgtta    1680
   aatgtacaca atcttgatcc tccaccttta tgggtcccat tgtttgcctc ttccgctgtg    1740
   tggagtgcgc ctacacgcac ttctcacttc gtaagtggtg gtggcgtaag tattgcctaa    1800
   tgttgactct atattctcct ctcctcaccc cctcgcggtg ctgatttctg acagatcttc    1860
   aaacactagt ttaagcaaag gactattcat ccgtttatat tagcaacagt aggtactagc    1920
   accactaaca acaacaacaa agcacttcta tttatttatc atg cgt gag gct atc      1975
                                               Met Arg Glu Ala Ile
                                               1               5
   tgc atc cac att ggt cag gct ggt tgc cag gtt ggt aac gcc tgc tgg      2023
   Cys Ile His Ile Gly Gln Ala Gly Cys Gln Val Gly Asn Ala Cys Trp
                   10                  15                  20
   gaa ttg ttc tgc ctg gaa cac ggc att caa ccc gat ggt gcg atg ccc      2071
   Glu Leu Phe Cys Leu Glu His Gly Ile Gln Pro Asp Gly Ala Met Pro
               25                  30                  35
   tct gac aag acg att ggc gtt gag gat gat gcg ttc aac acc ttc ttc      2119
   Ser Asp Lys Thr Ile Gly Val Glu Asp Asp Ala Phe Asn Thr Phe Phe
           40                  45                  50
   tct gag act ggt gct ggc aag cac gtt ccc cgc gcg gtg ttc ttg gac      2167
   Ser Glu Thr Gly Ala Gly Lys His Val Pro Arg Ala Val Phe Leu Asp
       55                  60                  65
   ctg gag cca aca gtg gtg gat gaa gtg cgc act ggc acg tac cgc cag      2215
   Leu Glu Pro Thr Val Val Asp Glu Val Arg Thr Gly Thr Tyr Arg Gln
   70                  75                  80                  85
   ctg ttc cac ccc gag cag ctg atc tcc ggc aag gag gat gcg gcc aac      2263
   Leu Phe His Pro Glu Gln Leu Ile Ser Gly Lys Glu Asp Ala Ala Asn
                   90                  95                  100
   aac tac gct cgt ggc cac tac acc att ggt aag gag atc gtc gac ctc        2311
   Asn Tyr Ala Arg Gly His Tyr Thr Ile Gly Lys Glu Ile Val Asp Leu
               105                 110                 115
   tgc ctg gac cgc atc cgc aag ctc gct gac aac tgc act ggt ctt cag        2359
   Cys Leu Asp Arg Ile Arg Lys Leu Ala Asp Asn Cys Thr Gly Leu Gln
           120                 125                 130
   ggc ttc ctc gtg tat cac gcc gtc ggc ggt ggc act ggt tct ggc ctg        2407
   Gly Phe Leu Val Tyr His Ala Val Gly Gly Gly Thr Gly Ser Gly Leu
       135                 140                 145
   ggt gcg ctg ctc ttg gag cgc ctc tcc gtg gac tat ggc aag aag tcc        2455
   Gly Ala Leu Leu Leu Glu Arg Leu Ser Val Asp Tyr Gly Lys Lys Ser
   150                 155                 160                 165
   aag ctc ggc tac acg gtg tat cca tca ccg cag gtg tcg acg gct gtc        2503
   Lys Leu Gly Tyr Thr Val Tyr Pro Ser Pro Gln Val Ser Thr Ala Val
                   170                 175                 180
   gtg gag ccc tac aac tct gtg ctc tcg aca cac tca ctt ctg gag cac        2551
   Val Glu Pro Tyr Asn Ser Val Leu Ser Thr His Ser Leu Leu Glu His
               185                 190                 195
   acc gat gtt gct gcg atg ctt gac aat gaa gca att tat gat ttg act        2599
   Thr Asp Val Ala Ala Met Leu Asp Asn Glu Ala Ile Tyr Asp Leu Thr
           200                 205                 210
   cgc cgc aac ctc gat att gag cgc ccc acg tac acc aac ctg aac cgc        2647
   Arg Arg Asn Leu Asp Ile Glu Arg Pro Thr Tyr Thr Asn Leu Asn Arg
       215                 220                 225
   ctc atc ggt cag gtg gtt tcc tcg ctg aca gcg tcc ctc cgc ttc gac        2695
   Leu Ile Gly Gln Val Val Ser Ser Leu Thr Ala Ser Leu Arg Phe Asp
   230                 235                 240                 245
   ggt gca ttg aac gtg gat ctg aca gag ttc cag aca aac ctt gtg ccg        2743
   Gly Ala Leu Asn Val Asp Leu Thr Glu Phe Gln Thr Asn Leu Val Pro
                   250                 255                 260
   tac cca cgt atc cac ttc gtg ctg aca agc tat gca cca gtc atc tcc        2791
   Tyr Pro Arg Ile His Phe Val Leu Thr Ser Tyr Ala Pro Val Ile Ser
               265                 270                 275
   gca gag aag gcc tac cac gag caa ctc tct gtc tct gag atc tcg aac        2839
   Ala Glu Lys Ala Tyr His Glu Gln Leu Ser Val Ser Glu Ile Ser Asn
           280                 285                 290
   gct gtg ttt gag ccc gcc tcc atg atg aca aag tgc gac ccc cgc cac        2887
   Ala Val Phe Glu Pro Ala Ser Met Met Thr Lys Cys Asp Pro Arg His
       295                 300                 305
   ggc aag tac atg gcg tgc tgc ctc atg tac cgt ggt gac gtt gtg cca        2935
   Gly Lys Tyr Met Ala Cys Cys Leu Met Tyr Arg Gly Asp Val Val Pro
   310                 315                 320                 325
   aag gat gtg aat gct gcc gtc gcg acc atc aag acg aag cgc acg att        2983
   Lys Asp Val Asn Ala Ala Val Ala Thr Ile Lys Thr Lys Arg Thr Ile
                   330                 335                 340
   cag ttc gtg gac tgg tct ccc aca ggc ttc aag tgc ggt atc aac tac        3031
   Gln Phe Val Asp Trp Ser Pro Thr Gly Phe Lys Cys Gly Ile Asn Tyr
               345                 350                 355
   cag cca ccc acg gtg gtg cca ggt ggt gac ctt gcc aag gtg cag cgc      3079
   Gln Pro Pro Thr Val Val Pro Gly Gly Asp Leu Ala Lys Val Gln Arg
           360                 365                 370
   gcg gta tgc atg atc gcc aac tcc acg gcc atc gca gag gtg ttc gcc      3127
   Ala Val Cys Met Ile Ala Asn Ser Thr Ala Ile Ala Glu Val Phe Ala
       375                 380                 385
   cgt att gac cac aaa ttc gat ctc atg tac agc aag cgc gcc ttc gtg      3175
   Arg Ile Asp His Lys Phe Asp Leu Met Tyr Ser Lys Arg Ala Phe Val
   390                 395                 400                     405
   cac tgg tac gtc ggt gag ggt atg gaa gag ggt gag ttc tcc gag gcc      3223
   His Trp Tyr Val Gly Glu Gly Met Glu Glu Gly Glu Phe Ser Glu Ala
                   410                 415                 420
   cgt gaa gac ctt gca gca ctt gag aag gac tac gaa gag gtt ggt gcc      3271
   Arg Glu Asp Leu Ala Ala Leu Glu Lys Asp Tyr Glu Glu Val Gly Ala
               425                 430                 435
   gag tcc gcg gat atg gac ggt gag gag gat gtg gag gag tac tag          3316
   Glu Ser Ala Asp Met Asp Gly Glu Glu Asp Val Glu Glu Tyr
           440                 445                 450
   aaagtgtgac aacgtcgcac catgtgtagg ttttcattta tgttctttct ttcttttttt    3376
   tgtgaatttg ttttctgtct caaatgtttt taattcgctt gggacctatg tttttcttgt    3436
   ttttttgctc accctttgtg taggaggcac cctgtcacgt ctgtggttgc gtgtatgcct    3496
   tccttcccct tattcgcttc ttcctgtcgt gtcacacctc tttctctctc tccctttccg    3556
   ccttttcttt caatcttgtt ttctcgacca gccctactag aggagaaaga atagtaaccc    3616
   tttcatcaaa gaaaatagtt caaacgaatt                                      3646
<210> 183
   <211> 451
   <212> PRT
   <213> T. brucei alpha tubulin
<400> 183
   Met Arg Glu Ala Ile Cys Ile His Ile Gly Gln Ala Gly Cys Gln Val
   1               5                   10                  15
   Gly Asn Ala Cys Trp Glu Leu Phe Cys Leu Glu His Gly Ile Gln Pro
               20                  25                  30
   Asp Gly Ala Met Pro Ser Asp Lys Thr Ile Gly Val Glu Asp Asp Ala
           35                  40                  45
   Phe Asn Thr Phe Phe Ser Glu Thr Gly Ala Gly Lys His Val Pro Arg
       50                  55                  60
   Ala Val Phe Leu Asp Leu Glu Pro Thr Val Val Asp Glu Val Arg Thr
   65                  70                  75                  80
   Gly Thr Tyr Arg Gln Leu Phe His Pro Glu Gln Leu Ile Ser Gly Lys
   85 90 95
   Glu Asp Ala Ala Asn Asn Tyr Ala Arg Gly His Tyr Thr Ile Gly Lys
   100 105 110
   Glu Ile Val Asp Leu Cys Leu Asp Arg Ile Arg Lys Leu Ala Asp Asn
   115 120 125
   Cys Thr Gly Leu Gln Gly Phe Leu Val Tyr His Ala Val Gly Gly Gly
   130 135 140
   Thr Gly Ser Gly Leu Gly Ala Leu Leu Leu Glu Arg Leu Ser Val Asp
   145 150 155 160
   Tyr Gly Lys Lys Ser Lys Leu Gly Tyr Thr Val Tyr Pro Ser Pro Gln
   165 170 175
   Val Ser Thr Ala Val Val Glu Pro Tyr Asn Ser Val Leu Ser Thr His
   180 185 190
   Ser Leu Leu Glu His Thr Asp Val Ala Ala Met Leu Asp Asn Glu Ala
   195 200 205
   Ile Tyr Asp Leu Thr Arg Arg Asn Leu Asp Ile Glu Arg Pro Thr Tyr
   210 215 220
   Thr Asn Leu Asn Arg Leu Ile Gly Gln Val Val Ser Ser Leu Thr Ala
   225 230 235 240
   Ser Leu Arg Phe Asp Gly Ala Leu Asn Val Asp Leu Thr Glu Phe Gln
   245 250 255
   Thr Asn Leu Val Pro Tyr Pro Arg Ile His Phe Val Leu Thr Ser Tyr
   260 265 270
   Ala Pro Val Ile Ser Ala Glu Lys Ala Tyr His Glu Gln Leu Ser Val
   275 280 285
   Ser Glu Ile Ser Asn Ala Val Phe Glu Pro Ala Ser Met Met Thr Lys
   290 295 300
   Cys Asp Pro Arg His Gly Lys Tyr Met Ala Cys Cys Leu Met Tyr Arg
   305 310 315 320
   Gly Asp Val Val Pro Lys Asp Val Asn Ala Ala Val Ala Thr Ile Lys
   325 330 335
   Thr Lys Arg Thr Ile Gln Phe Val Asp Trp Ser Pro Thr Gly Phe Lys
               340                 345                 350
   Cys Gly Ile Asn Tyr Gln Pro Pro Thr Val Val Pro Gly Gly Asp Leu
           355                 360                 365
   Ala Lys Val Gln Arg Ala Val Cys Met Ile Ala Asn Ser Thr Ala Ile
       370                 375                 380
   Ala Glu Val Phe Ala Arg Ile Asp His Lys Phe Asp Leu Met Tyr Ser
   385                 390                 395                 400
   Lys Arg Ala Phe Val His Trp Tyr Val Gly Glu Gly Met Glu Glu Gly
                   405                 410                 415
   Glu Phe Ser Glu Ala Arg Glu Asp Leu Ala Ala Leu Glu Lys Asp Tyr
               420                 425                 430
   Glu Glu Val Gly Ala Glu Ser Ala Asp Met Asp Gly Glu Glu Asp Val
           435                 440                 445
   Glu Glu Tyr
       450
<210> 184
   <211> 32
   <212> DNA
   <213> synthetic oligonucleotide
<400> 184
   gatcgaattc atgcgtgagg ctatctgcat cc 32
<210> 185
   <211> 37
   <212> DNA
   <213> synthetic oligonucleotide
<400> 185
   gatcctcgag ctagtactcc tccacatcct cctcacc 37
<210> 186
   <211> 3646
   <212> DNA
   <213> T. brucei beta-tubulin
<220>
   <221> exon
   <222> (1)..(1329)
   <223>
<400> 186
   atg cgc gaa atc gtc tgc gtt cag gct ggc caa tgc ggt aac cag atc 48
   Met Arg Glu Ile Val Cys Val Gln Ala Gly Gln Cys Gly Asn Gln Ile
   1               5                   10                  15
   ggc tca aag ttc tgg gag gtg atc agt gac gag cac ggt gtg gac ccc       96
   Gly Ser Lys Phe Trp Glu Val Ile Ser Asp Glu His Gly Val Asp Pro
               20                  25                  30
   aca ggt acc tac cag ggt gac tct gac ctg cag ctg gag cgc atc aat      144
   Thr Gly Thr Tyr Gln Gly Asp Ser Asp Leu Gln Leu Glu Arg Ile Asn
           35                  40                  45
   gtg tac ttt gat gag gca acg gga ggt cgc tat gtg ccc cgc tcc gtg      192
   Val Tyr Phe Asp Glu Ala Thr Gly Gly Arg Tyr Val Pro Arg Ser Val
       50                  55                  60
   ctg att gat ctg gag cca ggt aca atg gac tcc gta cgt gct ggc ccc      240
   Leu Ile Asp Leu Glu Pro Gly Thr Met Asp Ser Val Arg Ala Gly Pro
   65                  70                  75                  80
   tat ggt cag atc ttc cgc ccc gac aac ttc atc ttt gga cag tct ggc      288
   Tyr Gly Gln Ile Phe Arg Pro Asp Asn Phe Ile Phe Gly Gln Ser Gly
                   85                  90                  95
   gcc ggc aac aac tgg gca aag ggc cac tac acg gag ggt gcg gaa ctg      336
   Ala Gly Asn Asn Trp Ala Lys Gly His Tyr Thr Glu Gly Ala Glu Leu
               100                 105                 110
   atc gac tct gtg ctc gat gtg tgc tgc aag gag gcg gag agc tgt gac      384
   Ile Asp Ser Val Leu Asp Val Cys Cys Lys Glu Ala Glu Ser Cys Asp
           115                 120                 125
   tgc ctc caa ggc ttc cag atc tgc cac tcc ctt ggt ggt ggt act ggc      432
   Cys Leu Gln Gly Phe Gln Ile Cys His Ser Leu Gly Gly Gly Thr Gly
       130                 135                 140
   tcc ggc atg ggt acg ctg ctc atc tcg aag ctt cgc gag cag tac cct      480
   Ser Gly Met Gly Thr Leu Leu Ile Ser Lys Leu Arg Glu Gln Tyr Pro
   145                 150                 155                 160
   gac cgt atc atg atg act ttc tcc atc atc cca tcc ccc aag gtg tcc      528
   Asp Arg Ile Met Met Thr Phe Ser Ile Ile Pro Ser Pro Lys Val Ser
                   165                 170                 175
   gac act gtc gtc gag ccg tac aat acg act ctc tcc gtg cac caa ctt      576
   Asp Thr Val Val Glu Pro Tyr Asn Thr Thr Leu Ser Val His Gln Leu
               180                 185                 190
   gtg gaa aac tcc gat gag tcg atg tgc att gac aac gag gca ctg tac      624
   Val Glu Asn Ser Asp Glu Ser Met Cys Ile Asp Asn Glu Ala Leu Tyr
           195                 200                 205
   gat att tgc ttc cgc acc ctg aaa ctg aca aca cca acg ttc ggt gac      672
   Asp Ile Cys Phe Arg Thr Leu Lys Leu Thr Thr Pro Thr Phe Gly Asp
       210                 215                 220
   ctg aac cac ttg gtg tct gct gtt gtg tcc ggc gtc acc tgc tgc ctg      720
   Leu Asn His Leu Val Ser Ala Val Val Ser Gly Val Thr Cys Cys Leu
   225                 230                 235                 240
   cgc ttc cct ggt cag ttg aac tct gac ctc cgt aag ttg gct gtg aac      768
   Arg Phe Pro Gly Gln Leu Asn Ser Asp Leu Arg Lys Leu Ala Val Asn
                   245                 250                 255
   ctt gtc cca ttc ccg cgt ctg cac ttc ttc atg atg ggc ttc gcc ccg      816
   Leu Val Pro Phe Pro Arg Leu His Phe Phe Met Met Gly Phe Ala Pro
               260                 265                 270
   ctg acc agc cgc ggc tcg cag cag tac cgc ggt ctc tcc gtg ccc gag       864
   Leu Thr Ser Arg Gly Ser Gln Gln Tyr Arg Gly Leu Ser Val Pro Glu
           275                 280                 285
   cta acg cag cag atg ttc gat gcg aaa aac atg atg caa gct gca gat       912
   Leu Thr Gln Gln Met Phe Asp Ala Lys Asn Met Met Gln Ala Ala Asp
       290                 295                 300
   cct cgt cac ggc cgc tac ctg aca gcg tct gca ctc ttc cgc ggc cgc       960
   Pro Arg His Gly Arg Tyr Leu Thr Ala Ser Ala Leu Phe Arg Gly Arg
   305                 310                 315                 320
   atg tcg acg aag gag gtt gat gag cag atg ctg aac gtg cag aac aag      1008
   Met Ser Thr Lys Glu Val Asp Glu Gln Met Leu Asn Val Gln Asn Lys
                   325                 330                 335
   aac tcg tcc tac ttc att gag tgg atc ccg aac aac atc aag tcc tct      1056
   Asn Ser Ser Tyr Phe Ile Glu Trp Ile Pro Asn Asn Ile Lys Ser Ser
               340                 345                 350
   gtt tgc gat atc cca ccc aag gga ctc aag atg gct gtc acc ttc att      1104
   Val Cys Asp Ile Pro Pro Lys Gly Leu Lys Met Ala Val Thr Phe Ile
           355                 360                 365
   ggc aac aac acc tgc atc cag gag atg ttc cgc cgt gtg gga gag cag      1152
   Gly Asn Asn Thr Cys Ile Gln Glu Met Phe Arg Arg Val Gly Glu Gln
       370                 375                 380
   ttc acc ctc atg ttc cgt cgc aag gcg ttc ttg cac tgg tac act ggc      1200
   Phe Thr Leu Met Phe Arg Arg Lys Ala Phe Leu His Trp Tyr Thr Gly
   385                 390                 395                 400
   gag ggt atg gac gag atg gaa ttc acg gag gca gag tcc aac atg aac      1248
   Glu Gly Met Asp Glu Met Glu Phe Thr Glu Ala Glu Ser Asn Met Asn
                   405                 410                 415
   gat ctc gtg tct gag tac cag cag tac cag gat gcc acg att gag gag      1296
   Asp Leu Val Ser Glu Tyr Gln Gln Tyr Gln Asp Ala Thr Ile Glu Glu
               420                 425                 430
   gag ggc gag ttc gac gag gag gag caa tac tag acgcggacgg ggcatttccc    1349
   Glu Gly Glu Phe Asp Glu Glu Glu Gln Tyr
           435                 440
   gttcgtcatt agcagtaggt aatgaagatg tttgtttctc gtcccctttc tccttcgtcc    1409
   ttctgtcatt ttgttctttt gtgtttatgt tttgttgttg ttttctttaa tttttttttt    1469
   cttccacgtt tgtgtacatc cgcgcgccac tctattcaga gagccacgga tagtagagga    1529
   ggtgggaagg gtatatgagg gacacgcgta ccatgatgtg ggatgtattg gggtccctgt    1589
   ctgtccttac gtgactatgt atgaaccgtc acgtgtaaga tgagctagtg agatcaacag    1649
   tacaactcat caacacgcct tcttctcgtt aaatgtacac aatcttgatc ctccaccttt    1709
   atgggtccca ttgtttgcct cttccgctgt gtggagtgcg cctacacgca cttctcactt    1769
   cgtaagtggt ggtggcgtaa gtattgccta atgttgactc tatattctcc tctcctcacc    1829
   ccctcgcggt gctgatttct gacagatctt caaacactag tttaagcaaa ggactattca    1889
   tccgtttata ttagcaacag taggtactag caccactaac aacaacaaca aagcacttct   1949
   atttatttat catgcgtgag gctatctgca tccacattgg tcaggctggt tgccaggttg   2009
   gtaacgcctg ctgggaattg ttctgcctgg aacacggcat tcaacccgat ggtgcgatgc   2069
   cctctgacaa gacgattggc gttgaggatg atgcgttcaa caccttcttc tctgagactg   2129
   gtgctggcaa gcacgttccc cgcgcggtgt tcttggacct ggagccaaca gtggtggatg   2189
   aagtgcgcac tggcacgtac cgccagctgt tccaccccga gcagctgatc tccggcaagg   2249
   aggatgcggc caacaactac gctcgtggcc actacaccat tggtaaggag atcgtcgacc   2309
   tctgcctgga ccgcatccgc aagctcgctg acaactgcac tggtcttcag ggcttcctcg   2369
   tgtatcacgc cgtcggcggt ggcactggtt ctggcctggg tgcgctgctc ttggagcgcc   2429
   tctccgtgga ctatggcaag aagtccaagc tcggctacac ggtgtatcca tcaccgcagg   2489
   tgtcgacggc tgtcgtggag ccctacaact ctgtgctctc gacacactca cttctggagc   2549
   acaccgatgt tgctgcgatg cttgacaatg aagcaattta tgatttgact cgccgcaacc   2609
   tcgatattga gcgccccacg tacaccaacc tgaaccgcct catcggtcag gtggtttcct   2669
   cgctgacagc gtccctccgc ttcgacggtg cattgaacgt ggatctgaca gagttccaga   2729
   caaaccttgt gccgtaccca cgtatccact tcgtgctgac aagctatgca ccagtcatct   2789
   ccgcagagaa ggcctaccac gagcaactct ctgtctctga gatctcgaac gctgtgtttg   2849
   agcccgcctc catgatgaca aagtgcgacc cccgccacgg caagtacatg gcgtgctgcc   2909
   tcatgtaccg tggtgacgtt gtgccaaagg atgtgaatgc tgccgtcgcg accatcaaga   2969
   cgaagcgcac gattcagttc gtggactggt ctcccacagg cttcaagtgc ggtatcaact   3029
   accagccacc cacggtggtg ccaggtggtg accttgccaa ggtgcagcgc gcggtatgca   3089
   tgatcgccaa ctccacggcc atcgcagagg tgttcgcccg tattgaccac aaattcgatc   3149
   tcatgtacag caagcgcgcc ttcgtgcact ggtacgtcgg tgagggtatg gaagagggtg   3209
   agttctccga ggcccgtgaa gaccttgcag cacttgagaa ggactacgaa gaggttggtg   3269
   ccgagtccgc ggatatggac ggtgaggagg atgtggagga gtactagaaa gtgtgacaac   3329
   gtcgcaccat gtgtaggttt tcatttatgt tctttctttc ttttttttgt gaatttgttt   3389
   tctgtctcaa atgtttttaa ttcgcttggg acctatgttt ttcttgtttt tttgctcacc   3449
   ctttgtgtag gaggcaccct gtcacgtctg tggttgcgtg tatgccttcc ttccccttat   3509
   tcgcttcttc ctgtcgtgtc acacctcttt ctctctctcc ctttccgcct tttctttcaa   3569
   tcttgttttc tcgaccagcc ctactagagg agaaagaata gtaacccttt catcaaagaa   3629
   aatagttcaa acgaatt                                                  3646
<210> 187
   <211> 442
   <212> PRT
   <213> T. brucei beta-tubulin
<400> 187
   Met Arg Glu Ile Val Cys Val Gln Ala Gly Gln Cys Gly Asn Gln Ile
   1               5                   10                  15
   Gly Ser Lys Phe Trp Glu Val Ile Ser Asp Glu His Gly Val Asp Pro
               20                  25                  30
   Thr Gly Thr Tyr Gln Gly Asp Ser Asp Leu Gln Leu Glu Arg Ile Asn
           35                  40                  45
   Val Tyr Phe Asp Glu Ala Thr Gly Gly Arg Tyr Val Pro Arg Ser Val
       50                  55                  60
   Leu Ile Asp Leu Glu Pro Gly Thr Met Asp Ser Val Arg Ala Gly Pro
   65                  70                  75                  80
   Tyr Gly Gln Ile Phe Arg Pro Asp Asn Phe Ile Phe Gly Gln Ser Gly
                   85                  90                  95
   Ala Gly Asn Asn Trp Ala Lys Gly His Tyr Thr Glu Gly Ala Glu Leu
               100                 105                 110
   Ile Asp Ser Val Leu Asp Val Cys Cys Lys Glu Ala Glu Ser Cys Asp
           115                 120                 125
   Cys Leu Gln Gly Phe Gln Ile Cys His Ser Leu Gly Gly Gly Thr Gly
       130                 135                 140
   Ser Gly Met Gly Thr Leu Leu Ile Ser Lys Leu Arg Glu Gln Tyr Pro
   145                 150                 155                 160
   Asp Arg Ile Met Met Thr Phe Ser Ile Ile Pro Ser Pro Lys Val Ser
                   165                 170                 175
   Asp Thr Val Val Glu Pro Tyr Asn Thr Thr Leu Ser Val His Gln Leu
               180                 185                 190
   Val Glu Asn Ser Asp Glu Ser Met Cys Ile Asp Asn Glu Ala Leu Tyr
           195                 200                 205
   Asp Ile Cys Phe Arg Thr Leu Lys Leu Thr Thr Pro Thr Phe Gly Asp
       210                 215                 220
   Leu Asn His Leu Val Ser Ala Val Val Ser Gly Val Thr Cys Cys Leu
   225                 230                 235                 240
   Arg Phe Pro Gly Gln Leu Asn Ser Asp Leu Arg Lys Leu Ala Val Asn
                   245                 250                 255
   Leu Val Pro Phe Pro Arg Leu His Phe Phe Met Met Gly Phe Ala Pro
               260                 265                 270
   Leu Thr Ser Arg Gly Ser Gln Gln Tyr Arg Gly Leu Ser Val Pro Glu
           275                 280                 285
   Leu Thr Gln Gln Met Phe Asp Ala Lys Asn Met Met Gln Ala Ala Asp
       290                 295                 300
   Pro Arg His Gly Arg Tyr Leu Thr Ala Ser Ala Leu Phe Arg Gly Arg
   305                 310                 315                 320
   Met Ser Thr Lys Glu Val Asp Glu Gln Met Leu Asn Val Gln Asn Lys
                   325                 330                 335
   Asn Ser Ser Tyr Phe Ile Glu Trp Ile Pro Asn Asn Ile Lys Ser Ser
               340                 345                 350
   Val Cys Asp Ile Pro Pro Lys Gly Leu Lys Met Ala Val Thr Phe Ile
           355                 360                 365
   Gly Asn Asn Thr Cys Ile Gln Glu Met Phe Arg Arg Val Gly Glu Gln
       370                 375                 380
   Phe Thr Leu Met Phe Arg Arg Lys Ala Phe Leu His Trp Tyr Thr Gly
   385                 390                 395                 400
   Glu Gly Met Asp Glu Met Glu Phe Thr Glu Ala Glu Ser Asn Met Asn
                   405                 410                 415
   Asp Leu Val Ser Glu Tyr Gln Gln Tyr Gln Asp Ala Thr Ile Glu Glu
               420                 425                 430
   Glu Gly Glu Phe Asp Glu Glu Glu Gln Tyr
           435                 440
<210> 188
   <211> 37
   <212> DNA
   <213> synthetic oligonucleotide
-<400> 188
   gatcctcgag ctagtactcc tccacatcct cctcacc 37
<210> 189
   <211> 32
   <212> DNA
   <213> synthetic oligonucleotide
<400> 189
   gatcctcgag ctagtattgc tcctcctcgt cg 32
<210> 190
   <211> 5
   <212> PRT
   <213> sortase A cleavage site
<220>
   <221> MISC_FEATURE
   <222> (3)..(3)
   <223> Xaa at position 3 is any amino acid
<400> 190
   Leu Pro Xaa Thr Gly
   1 5
<210> 191
   <211> 5
   <212> PRT
   <213> sortase B cleavage site
<400> 191
   Asn Pro Gln Thr Asn
   1 5
<210> 192
   <211> 765
   <212> DNA
   <213> FemX gene
<220>
   <221> CDS
   <222> (1)..(765)
   <223>
<400> 192
   atg ata ttg aaa ttt gat cac atc att cat tat ata gat cag tta gat        48
   Met Ile Leu Lys Phe Asp His Ile Ile His Tyr Ile Asp Gln Leu Asp
   1               5                   10                  15
   cgg ttt agt ttt cca gga gat gtt ata aaa tta cat tca ggt ggg tat        96
   Arg Phe Ser Phe Pro Gly Asp Val Ile Lys Leu His Ser Gly Gly Tyr
               20                  25                  30
   cat cat aaa tat gga aca ttc aat aaa tta ggt tat atc aat gaa aat       144
   His His Lys Tyr Gly Thr Phe Asn Lys Leu Gly Tyr Ile Asn Glu Asn
           35                  40                  45
   tat att gag cta cta gat gta gaa aat aat gaa aag ttg aaa aag atg       192
   Tyr Ile Glu Leu Leu Asp Val Glu Asn Asn Glu Lys Leu Lys Lys Met
       50                  55                  60
   gca aaa acg ata gaa ggc gga gtc gct ttt gct act caa att gtt caa       240
   Ala Lys Thr Ile Glu Gly Gly Val Ala Phe Ala Thr Gln Ile Val Gln
   65                  70                  75                  80
   gag aag tat gag caa ggc ttt aaa aat att tgt ttg cat aca aat gat        288
   Glu Lys Tyr Glu Gln Gly Phe Lys Asn Ile Cys Leu His Thr Asn Asp
                   85                  90                  95
   ata gag gca gtt aaa aat aaa cta caa agt gag cag gtt gaa gta gta        336
   Ile Glu Ala Val Lys Asn Lys Leu Gln Ser Glu Gln Val Glu Val Val
               100                 105                 110
   ggg ccg att caa atg gaa aga gat aca cat aaa gat ggt aag gta aag        384
   Gly Pro Ile Gln Met Glu Arg Asp Thr His Lys Asp Gly Lys Val Lys
           115                 120                 125
   tgg caa ttg ctt tat ata atg aat cag gat gat gat gaa att aag cca        432
   Trp Gln Leu Leu Tyr Ile Met Asn Gln Asp Asp Asp Glu Ile Lys Pro
       130                 135                 140
   cca ttt ttt att caa tgg gaa gaa agt gat tcc atg cgt act aaa aaa        480
   Pro Phe Phe Ile Gln Trp Glu Glu Ser Asp Ser Met Arg Thr Lys Lys
   145                 150                 155                 160
   ttg caa aaa tat ttt caa aaa caa ttt tca att gaa act gtt att gtg        528
   Leu Gln Lys Tyr Phe Gln Lys Gln Phe Ser Ile Glu Thr Val Ile Val
                   165                 170                 175
   aaa agt aaa aac cga tca caa aca gta tcg aat tgg ttg aaa tgg ttt        576
   Lys Ser Lys Asn Arg Ser Gln Thr Val Ser Asn Trp Leu Lys Trp Phe
               180                 185                 190
   gat atg gac att gta gaa gag aat gac cat tac aca gat ttg att tta        624
   Asp Met Asp Ile Val Glu Glu Asn Asp His Tyr Thr Asp Leu Ile Leu
           195                 200                 205
   aaa aat gat gat att tat ttt aga att gaa gat ggt aaa gtt tca aaa        672
   Lys Asn Asp Asp Ile Tyr Phe Arg Ile Glu Asp Gly Lys Val Ser Lys
       210                 215                 220
   tat cat tcg gtt atc ata aaa gac gca caa gca act tca cca tat tca        720
   Tyr His Ser Val Ile Ile Lys Asp Ala Gln Ala Thr Ser Pro Tyr Ser
   225                 230                 235                 240
   att ttt atc aga ggt gct att tat cgc ttt gaa cca tta gta taa            765
   Ile Phe Ile Arg Gly Ala Ile Tyr Arg Phe Glu Pro Leu Val
                   245                 250
<210> 193
   <211> 254
   <212> PRT
   <213> FemX gene
<400> 193
   Met Ile Leu Lys Phe Asp His Ile Ile His Tyr Ile Asp Gln Leu Asp
   1               5                   10                  15
   Arg Phe Ser Phe Pro Gly Asp Val Ile Lys Leu His Ser Gly Gly Tyr
               20                  25                  30
   His His Lys Tyr Gly Thr Phe Asn Lys Leu Gly Tyr Ile Asn Glu Asn
           35                  40                  45
   Tyr Ile Glu Leu Leu Asp Val Glu Asn Asn Glu Lys Leu Lys Lys Met
       50                  55                  60
   Ala Lys Thr Ile Glu Gly Gly Val Ala Phe Ala Thr Gln Ile Val Gln
   65                  70                  75                  80
   Glu Lys Tyr Glu Gln Gly Phe Lys Asn Ile Cys Leu His Thr Asn Asp
                   85                  90                  95
   Ile Glu Ala Val Lys Asn Lys Leu Gln Ser Glu Gln Val Glu Val Val
               100                 105                 110
   Gly Pro Ile Gln Met Glu Arg Asp Thr His Lys Asp Gly Lys Val Lys
           115                 120                 125
   Trp Gln Leu Leu Tyr Ile Met Asn Gln Asp Asp Asp Glu Ile Lys Pro
       130                 135                 140
   Pro Phe Phe Ile Gln Trp Glu Glu Ser Asp Ser Met Arg Thr Lys Lys
   145                 150                 155                 160
   Leu Gln Lys Tyr Phe Gln Lys Gln Phe Ser Ile Glu Thr Val Ile Val
                   165                 170                 175
   Lys Ser Lys Asn Arg Ser Gln Thr Val Ser Asn Trp Leu Lys Trp Phe
               180                 185                 190
   Asp Met Asp Ile Val Glu Glu Asn Asp His Tyr Thr Asp Leu Ile Leu
           195                 200                 205
   Lys Asn Asp Asp Ile Tyr Phe Arg Ile Glu Asp Gly Lys Val Ser Lys
       210                 215                 220
   Tyr His Ser Val Ile Ile Lys Asp Ala Gln Ala Thr Ser Pro Tyr Ser
   225                 230                 235                 240
   Ile Phe Ile Arg Gly Ala Ile Tyr Arg Phe Glu Pro Leu Val
                   245                 250
<210> 194
   <211> 621
   <212> DNA
   <213> sortase A gene
<220>
   <221> CDS
   <222> (1)..(207)
   <223>
<400> 194
   atg aaa aaa tgg aca aat cga tta atg aca atc gct ggt gtg gta ctt      48
   Met Lys Lys Trp Thr Asn Arg Leu Met Thr Ile Ala Gly Val Val Leu
   1               5                   10                  15
   atc cta gtg gca gca tat ttg ttt gct aaa cca cat atc gat aat tat      96
   Ile Leu Val Ala Ala Tyr Leu Phe Ala Lys Pro His Ile Asp Asn Tyr
               20                  25                  30
   ctt cac gat aaa gat aaa gat gaa aag att gaa caa tat gat aaa aat     144
   Leu His Asp Lys Asp Lys Asp Glu Lys Ile Glu Gln Tyr Asp Lys Asn
           35                  40                  45
   gta aaa gaa cag gcg agt aaa gat aaa aag cag caa gct aaa cct caa     192
   Val Lys Glu Gln Ala Ser Lys Asp Lys Lys Gln Gln Ala Lys Pro Gln
       50                  55                  60
   att ccg aaa gat aaa tcgaaagtgg caggctatat tgaaattcca gatgctgata     247
   Ile Pro Lys Asp Lys
   65
   ttaaagaacc agtatatcca ggaccagcaa cacctgaaca attaaataga ggtgtaagct   307
   ttgcagaaga aaacgaatca ctagatgatc aaaatatttc aattgcagga cacactttca   367
   ttgaccgtcc gaactatcaa tttacaaatc ttaaagcagc caaaaaaggt agtatggtgt   427
   actttaaagt tggtaatgaa acacgtaagt ataaaatgac aagtataaga gatgttaagc   487
   ctacagatgt agaagttcta gatgaacaaa aaggtaaaga taaacaatta acattaatta   547
   cttgtgatga ttacaatgaa aagacaggcg tttgggaaaa acgtaaaatc tttgtagcta   607
   cagaagtcaa ataa                                                     621
<210> 195
   <211> 69
   <212> PRT
   <213> sortase A gene
<400> 195
   Met Lys Lys Trp Thr Asn Arg Leu Met Thr Ile Ala Gly Val Val Leu
   1               5                   10                  15
   Ile Leu Val Ala Ala Tyr Leu Phe Ala Lys Pro His Ile Asp Asn Tyr
               20                  25                  30
   Leu His Asp Lys Asp Lys Asp Glu Lys Ile Glu Gln Tyr Asp Lys Asn
           35                  40                  45
   Val Lys Glu Gln Ala Ser Lys Asp Lys Lys Gln Gln Ala Lys Pro Gln
       50                  55                  60
   Ile Pro Lys Asp Lys
   65
<210> 196
   <211> 735
   <212> DNA
   <213> sortase B gene
<220>
   <221> CDS
   <222> (1)..(293)
   <223>
<400> 196
   atg aga atg aag cga ttt tta act att gta caa att tta ttg gtt gta      48
   Met Arg Met Lys Arg Phe Leu Thr Ile Val Gln Ile Leu Leu Val Val
   1               5                   10                  15
   att att atc att ttt ggt tac aaa att gtt caa aca tat att gaa gac      96
   Ile Ile Ile Ile Phe Gly Tyr Lys Ile Val Gln Thr Tyr Ile Glu Asp
               20                  25                  30
   aag caa gaa cgc gca aat tat gag aaa tta caa caa aaa ttt caa atg     144
   Lys Gln Glu Arg Ala Asn Tyr Glu Lys Leu Gln Gln Lys Phe Gln Met
           35                  40                  45
   ctg atg agc aaa cat caa gca cat gtg aga cca caa ttt gaa tca ctt     192
   Leu Met Ser Lys His Gln Ala His Val Arg Pro Gln Phe Glu Ser Leu
       50                  55                  60
   gaa aaa ata aat aaa gac att gtt gga tgg ata aaa tta tca gga aca     240
   Glu Lys Ile Asn Lys Asp Ile Val Gly Trp Ile Lys Leu Ser Gly Thr
   65                  70                  75                  80
   tca ttaaattatc cagtactaca aggtaagaca aatcacgatt atttaaattt          293
   Ser
   agattttgag cgagaacatc gacgtaaagg tagtattttt atggatttta gaaatgaatt   353
   gaagaattta aatcataata ctattttata cgggcaccat gtcggtgata atacgatgtt   413
   tgatgtgtta gaagattatt taaagcaatc gttttatgaa aaacacaaga taattgaatt   473
   tgacaataaa tatggtaaat atcaattgca agtatttagt gcatataaaa ctactactaa   533
   agataattac atacgtacag attttgaaaa tgatcaagat tatcaacaat ttttagatga   593
   aacaaaacgt aaatctgtaa ttaattcaga tgttaatgta acggtaaaag atagaataat   653
   gactttatca acgtgcgaag atgcatatag tgaaacaacg aaaagaattg ttgttgtcgc   713
   aaaaataatt aaggtaagtt aa                                             735
<210> 197
   <211> 81
   <212> PRT
   <213> sortase B gene
<400> 197
   Met Arg Met Lys Arg Phe Leu Thr Ile Val Gln Ile Leu Leu Val Val
   1               5                   10                  15
   Ile Ile Ile Ile Phe Gly Tyr Lys Ile Val Gln Thr Tyr Ile Glu Asp
               20                  25                  30
   Lys Gln Glu Arg Ala Asn Tyr Glu Lys Leu Gln Gln Lys Phe Gln Met
           35                  40                  45
   Leu Met Ser Lys His Gln Ala His Val Arg Pro Gln Phe Glu Ser Leu
       50                  55                  60
   Glu Lys Ile Asn Lys Asp Ile Val Gly Trp Ile Lys Leu Ser Gly Thr
   65                  70                  75                  80
   Ser